# EUROPEAN PATENT APPLICATION

(11) **EP 4 279 089 A1**
(43) Date of publication of application: **22.11.2023**
(21) Application number: 22755607.3
(22) Date of filing: 22.02.2022
(51) Int. Cl.: A61K 47/54, A61K 47/68, A61K 31/5365, A61K 38/07, A61K 31/437, A61K 31/404, A61K 31/704, A61K 31/5517, A61P 35/00, A61P 29/00, A61P 31/12, A61P 31/00, A61P 37/02, C07D 498/18

(54) **DISACCHARIDE LINKER, DISACCHARIDE-SMALL MOLECULE DRUG CONJUGATE AND SUGAR CHAIN FIXED-POINT ANTIBODY-DRUG CONJUGATE, AND PREPARATION METHOD THEREFOR AND USE THEREOF**

(30) Priority: 22.02.2021 CN 202110198313
(71) Applicant: Shanghai Institute of Materia Medica, Chinese Academy of Sciences, Pudong, Shanghai 201203 (CN)
(72) Inventor: HUANG, Wei, Shanghai 201203 (CN); TANG, Feng, Shanghai 201203 (CN); SHI, Wei, Shanghai 201203 (CN); JIAO, Shang, Shanghai 201203 (CN); WANG, Siqi, Shanghai 201203 (CN)
(74) Representative: Bandpay & Greuter
(86) International application number: PCT/CN2022/077167
(87) International publication number: WO 2022/174834

(57) **Abstract**

The present application relates to a disaccharide linker, a disaccharide-small molecule drug conjugate and a sugar chain fixed-point antibody-drug conjugate, a preparation method and the use thereof. The structure of the disaccharide linker is as shown in the following formula I. The present invention provides a new-type fixed- point and quantitative antibody-drug conjugate form, and the stability and cytotoxicity of the antibody-drug conjugate are improved.

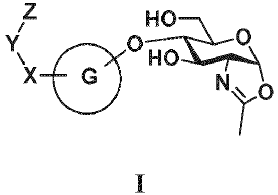

## Description

### TECHNICAL FIELD

The present invention belongs to the fields of medicinal chemistry and biotechnological medicine, in particular, the present application relates to a class of disaccharide linkers, non-natural glycoengineered antibodies and glycosite-specific and quantitative antibody-drug conjugates prepared therefrom, and preparation method and use thereof.

### BACKGROUND

Antibody-Drug Conjugates (ADCs), which are composed of an antibody, a cytotoxin and a linker, deliver cytotoxins to tumor tissues through antibodies to achieve targeted delivery of toxins, thereby exerting anti-tumor activity. Compared with traditional chemotherapy drugs, ADCs have lower body toxicity and better therapeutic index. Random coupling was mainly used for ADC drugs in the early stage to couple cytotoxins to lysine (Lys) or cysteine (Cys) with high abundance in antibodies. The ADCs formed in this way have defects of heterogeneous coupling site and number, poor *in vivo* stability, efficacy and pharmacokinetic properties, and narrow therapeutic window. Site-specific ADCs can solve the above problems. Currently, the mainstream site-specific coupling technologies used in the preparation of site-specific ADCs include THIOMAB technology, unnatural amino acid insertion technology, enzyme-catalyzed technology, and glycosite-specific coupling technology, etc., and each technology has its own characteristics.

The glycosite-specific ADC compound is prepared by glycosite-specific coupling technology, and the cytotoxin is site-specifically modified at the N297 glycosylation site of the Fc domain of antibody. Currently, *in vitro* antibody glycosite-specific modification methods mainly include glycosyltransferase technology and endoglycosidase technology.

In the glycosyltransferase technology, galactose or sialic acid with reactive functional groups is transferred to the glycosylation site of the antibody by using galactosyltransferase or sialyltransferase, which is further coupled to cytotoxins to prepare glycosite-specific ADC compounds. For example, Zhu et al. firstly used β1,4-galactosidase to hydrolyze the galactose at the end of the N-glycan at the glycosylation site of antibody and then used galactosyltransferase to transfer the ketocarbonyl-containing GalNAc to the end of the N-glycan and further coupled the toxin containing hydroxylamine functional group to obtain the glycosite-specific ADC compound. Qun Zhou et al. used galactosyltransferase and sialyltransferase to transfer galactose and sialic acid to the antibody glycosylation site sequentially, and then used sodium periodate to oxidize the terminal sialic acid so as to introduce an aldehyde group at the glycosylation site, providing a reactive site for toxin coupling. Floris L. van Delft et al firstly used the endoglycosidase Endo-S to hydrolyze the heterogeneous N-glycan of the antibody, then used galactosyltransferase to transfer the azide-containing GalNAz to the glycosylation site of the antibody, and finally used the click chemical reactions to achieve the modification of toxins at the glycosylation site.

Endoglycosidase technology utilizes endoglycosidases and bioorthogonal reactions to realize the preparation of glycosite-specific ADCs. Our team and the Davis team used semi-synthetic modification methods to obtain azido-modified oligosaccharide oxazoline substrates, and finally transferred oligosaccharides modified by the bioorthogonal groups to the glycosylation site of the antibody by using two endoglycosidases, Endo-S and its mutant enzyme Endo-S D233Q in sequence, and further obtained the glycosite-specific ADC compounds by using bioorthogonal reactions.

Compared with random coupling, both the existing glycosyltransferase technology and endoglycosidase technology can be used to obtain more homogeneous ADC compounds, but they have defects respectively. In glycosyltransferase technology, it is necessary to synthesize sugar substrates with CMP or UDP in activated forms, and glycosyltransferases generally have weak catalytic activity, resulting the longer reaction time, and the production efficiency and cost are difficult to control. It is also difficult to obtain the oligosaccharide substrate used in the endoglycosidase technology, as for the semi-synthetic modification means, the oligosaccharide substrate needs to be extracted from egg yolk, the purification steps are complicated, and it is even more difficult for the total synthesis means. Both glycosyltransferase and endoglycosidase methods involve multiple enzymes and multi-step reactions, and thus they have certain limitations in drug efficacy and production, and the stability of antibodies is poor. The current technologies related to the two means are highly dependent on bioorthogonal reactions to achieve the modification of toxins at the glycosylation site of antibodies, which limits the development of sugar-chain site-specific ADC drugs.

The present patent application proposes a class of disaccharide linkers, which can be efficiently transferred to the glycosylation site of the antibody under the action of the wild-type endoglycosidase Endo-S2. When the disaccharide structure has a bioorthogonal group, a glycoengineered antibody with a bioorthogonal group can be obtained under the catalysis of enzymes, and a glycosite-specific ADC compound based on the disaccharide structure can be prepared by using the bioorthogonal reaction in "two steps". When the disaccharide structure directly bears functional groups such as drugs, novel glycosite-specific ADC compounds can be prepared in one step under the catalysis of enzymes. The preparation method of the glycosite-specific ADC compounds provided by the invention is simple and efficient, and the obtained ADC has a novel molecular structure and good *in vivo* and *in vitro* activities.

### SUMMARY OF THE INVENTION

One of the technical goals of the present invention is to provide a disaccharide linker, which can realize the site-specific and quantitative introduction of small molecule drugs into antibodies.

Another technical goal of the present invention is to provide the use of the disaccharide linker in the preparation of antibody-drug conjugates.

Further technical goal of the present invention is to provide a disaccharide linker-small molecule drug conjugate.

Further technical goal of the present invention is to provide a small molecule drug-antibody conjugate linked by the disaccharide linker.

Further technical goal of the present invention is to provide the use of the disaccharide linker-small molecule drug conjugate or the small molecule drug-antibody conjugate linked by the disaccharide linker in the preparation of drugs or diagnostic reagents.

In one aspect, the present invention provides a disaccharide linker represented by the following general formula I:

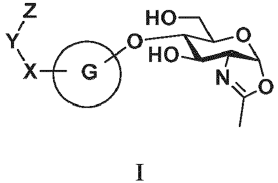

In general formula I,
G ring represents a structure derived from a monosaccharide molecule, which is connected to the 4-position of N-acetyl-D-glucosamine ring closed in 1,2 positions through a glycosidic bond, wherein the monosaccharide molecule is selected from the group consisting of galactose, N-acetyl-galactose, glucose, mannose, fucose, sialic acid sugar; the glycosidic bond is 1,4-glycosidic bond, 2,4-glycosidic bond or 3,4-glycosidic bond;
Z-Y-X- represents a substituent on the G ring, and the substitution position of Z-Y-X- is any position other than position 1 of the G ring derived from the monosaccharide molecule,
Wherein, in the structure Z-Y-X-, Z-Y- may or may not exist,
When Z-Y- does not exist, X is an aldehyde group, a phosphoric acid group, -NH₂, -CH₂-NH₂, -COOH, -CH₂SRₚ, -CH₂SeRₚ, -N₃, -CH₂-N₃, wherein Rₚ is a protecting group;
When Z-Y- exists, X is selected from the group consisting of -CH₂-, -CH₂-O-, -CH₂-S-, -CH₂-Se-, -CO-NH-, -ON=CH-, -CONH-N=CH-, -NHCH₂-, -CH=CH-, and the following structures: 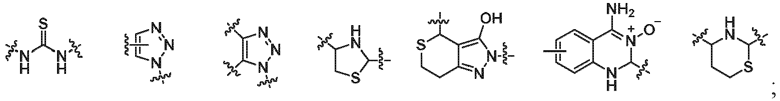
Y is a divalent linker or a multivalent linker connecting X and Z,
preferably, Y is selected from the following groups: -(CH₂)ₘ-(CH-w)ₙ-, -(CH₂-CH₂-O)ₘ-(CH-w)ₙ-, -(PO₄)ₙ-, wherein m and n are independently selected from an integer between 0-30, w is a hydrogen atom or a polyethylene glycol structure with different lengths; or a combination of cleavable fragments and the above-mentioned linking fragments;
Z is selected from the following cases i)-iv):
   i) reactive groups with bioorthogonal reactivity or fragments of functional molecules,
      Preferably, Z is selected from the following reactive groups: azide residues, aldehyde residues, thiol residues, alkyne residues, alkene residues, halogen residues, tetrazine residues, nitrone residues, hydroxylamine residues, nitrile residues, hydrazine residues, ketone residues, boronic acid residues, cyanobenzothiazole residues, allyl residues, phosphine residues, maleimide residues, disulfide residues, thioester residues, α-halogenated carbonyl residues, isonitrile residues, sydnones residues, selenium residues, conjugated diene residues, phosphoric acid residues, cycloalkyne residues and cycloalkene residues,
      Alternatively, Z is selected from the following groups: 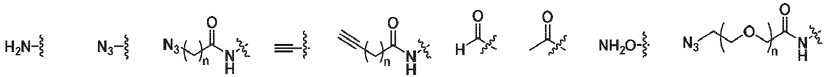 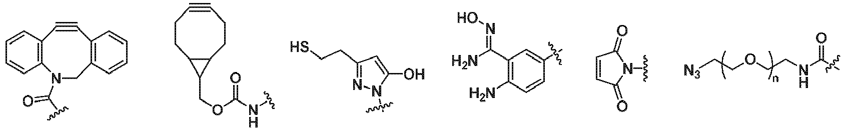 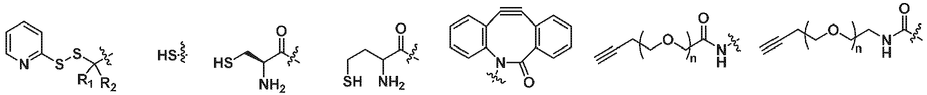 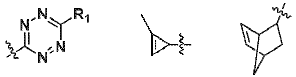
      Wherein, n is an integer of 1-30, R₁ and R₂ are independently selected from H, -CH₃, -CH₂CH₃, cyclopropyl or cyclobutyl;
      Preferably, the functional molecules are selected from: toxins, drugs, fluorescent probes, polyethylene glycol, lipids, polypeptides, nanobodies, DNA and related drugs, RNA and related drugs, cholesterol, antibiotics or radioisotope labels, contrast agents and MRI agents;
   ii) 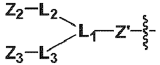
      Wherein, L₁ is a trivalent linker with three reactive groups,
      Preferably, L₁ is a branched-chain amino acid with reactive functional groups which is derived from lysine, aspartic acid, glutamic acid, propargylglycine, cysteine, and the following structures: 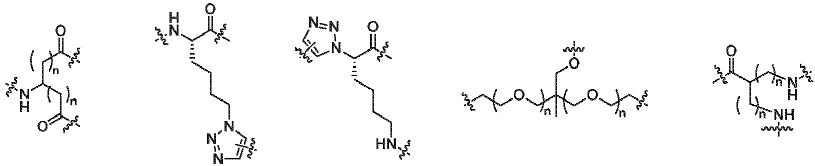 Wherein, n is an integer of 1-30,
      L₂ and L₃ are divalent or multivalent linkers connecting L₁ with Z₂ and Z₃,
      Preferably, L₂ and L₃ are independently selected from the following structures: -(CH₂)ₘ-(CH-w)ₙ-, -(CH₂-CH₂-O)ₘ-(CH-w)ₙ-, -(PO₄)ₙ-, wherein m and n are independently selected from integers between 0-30, w is a hydrogen atom or other side chain structures, such as polyethylene glycol with different lengths; or a combination of cleavable fragments and the above-mentioned linking fragments,
      Z' is a linking fragment coupling L₁ to the sugar linker, independently is absent or is -(CH₂)ₚ-, where p is an integer from 1 to 5, or is a group that can react with the Z group in case i),
      For example, Z' is selected from the following groups: 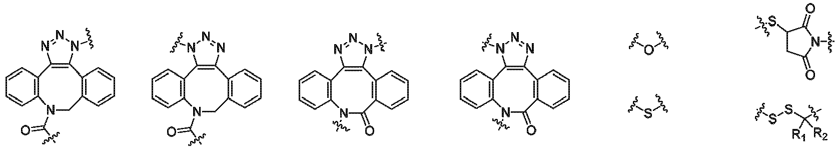 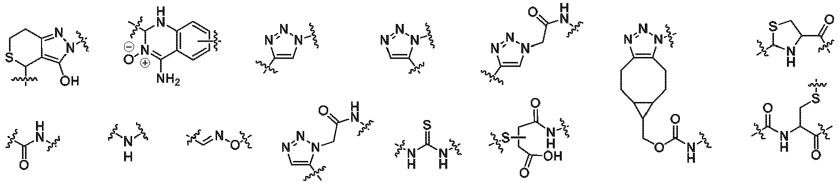 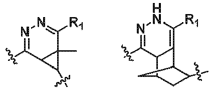
      Wherein, R₁ and R₂ are each independently selected from H, -CH₃, -CH₂CH₃, cyclopropyl or cyclobutyl;
      The definitions of Z₂ and Z₃ are the same as the definitions of Z in case i);
   iii) 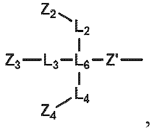 Wherein, L₆ is a tetravalent linker with four reactive groups,
      Preferably, L₆ is selected from dimerized lysine, dimerized glutamic acid, dimerized aspartic acid, aspartic acid-glutamic acid dipeptide structure, aspartic acid-lysine dipeptide structure, glutamate-lysine structure, or is a structure selected from the following: 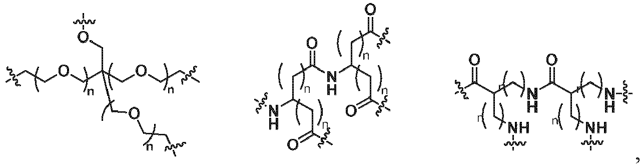
      Wherein, n is an integer of 1-30, the definitions of L₂, L₃, L₄ are the same as the definitions of L₂, L₃ in ii), the definition of Z' is the same as the definition of Z' in ii), and the definitions of Z₂, Z₃, Z₄ are the same as the definitions of Z₂ and Z₃ in ii);
   iv) 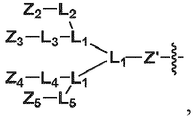
      wherein, the definition of L₁ is the same as the definition of L₁ in ii), the definitions of L₂, L₃, L₄, L₅ are the same as the definitions of L₂, L₃ in ii), the definitions of Z' is the same as the definition of Z' in ii), and the definitions of Z₂, Z₃, Z₄, Z₅ are the same as the definitions of Z₂ and Z₃ in ii);
      Or, when Y, Z are absent, X is selected from: 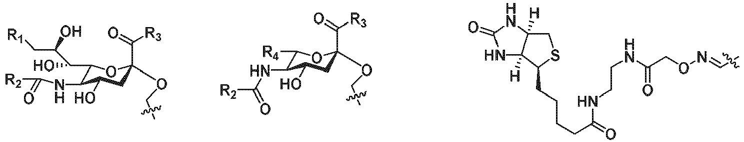
      Wherein, R₁ is hydroxyl -OH or azido -N₃, R₂ is any group, R₃ is hydroxyl -OH or any group containing -NH-, R₄ is any group, 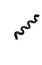 represents the connection position.

In a specific embodiment, the disaccharide linker of general formula I is represented by the following general formula II:

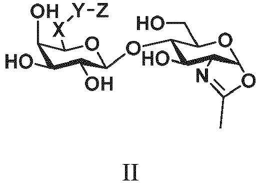

In general formula II, X, Y and Z are each as defined above.

In a specific embodiment, the disaccharide linker is selected from the following specific compounds:

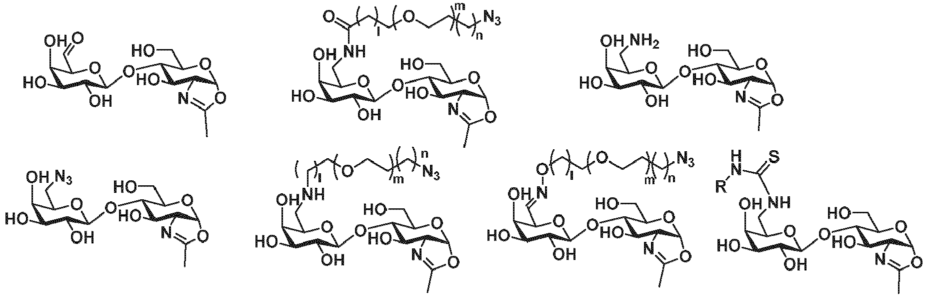

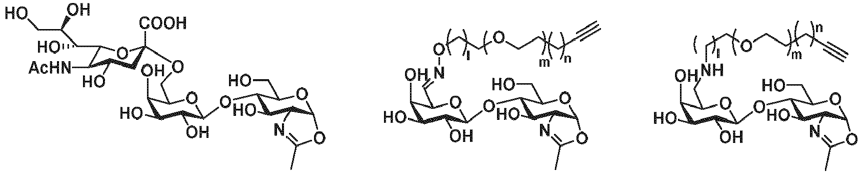

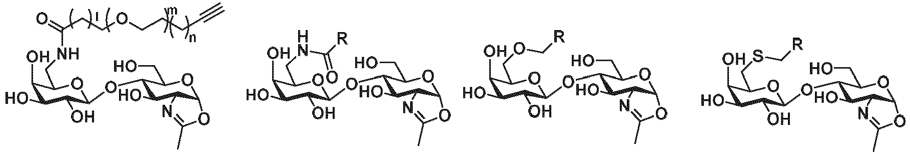

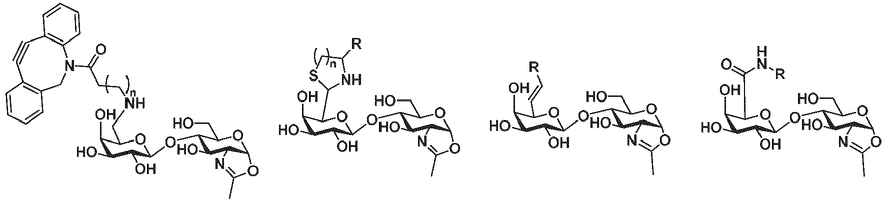

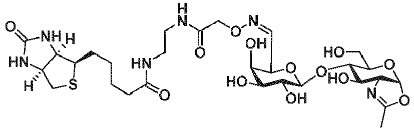

Wherein, R is a fragment related in the above Y and Z or a combination thereof, 1, m and n are each independently integers of 0-30.

In another aspect, the present invention provides a method for preparing the above-mentioned disaccharide linker, as shown in the following reaction scheme:

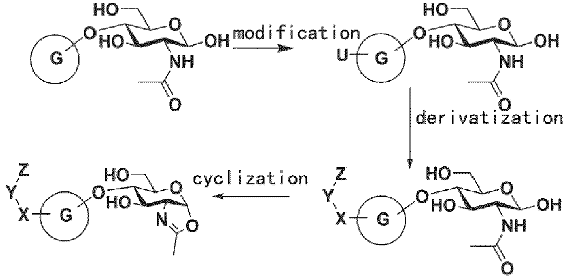

In the above reaction scheme, the G ring is as defined above, and the modification position of the monosaccharide is any modifiable position other than position 1; U is an introduced active group, and is selected from aldehyde group, amino group, azido group, alkyne group; the definitions of X, Y, and Z are the same as those defined above,

The method comprises the steps of:
1) The disaccharide structure with an acetylglucosamine structure at the end is modified under the action of enzymes or other small molecular compounds to obtain a disaccharide structure with an active group U, and a Z-Y-X- having orthogonal reactivity or containing a functional molecular fragment is introduced to the disaccharide structure with an active group U via derivatization; and
2) The disaccharide structure introduced with Z-Y-X- having orthogonal reactivity or containing a functional molecular fragment is converted into the disaccharide linker of general formula I by a cyclization reaction.

In a specific embodiment, the modification reaction in step 1) is an oxidation reaction, the enzyme is galactose oxidase, and U is an aldehyde group.

In a specific embodiment, the derivatization reaction in step 1) is an oxime-forming reaction, reductive amination, a reaction involving an amino group, or a reaction involving an azido group.

In a specific embodiment, in step 2), the cyclization reaction is carried out by using 2-chloro-1,3-dimethylimidazoline chloride or 2-chloro-1,3-dimethyl-1H- benzimidazole-3-chloro.

In another aspect, the present invention provides a disaccharide-small molecule drug conjugate represented by the following general formulas III, IV or V:

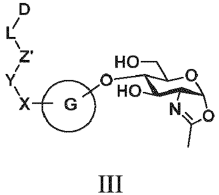

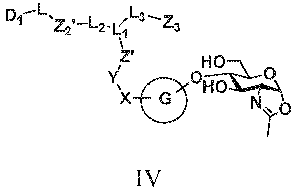

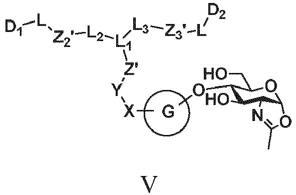

In the above general formulas III, IV and V, ring G, X, Y, Z₃, Z', L₁, L₂, L₃ are as defined above, respectively, and in the structures of general formula IV or V, each L may be the same or different from each other , Z₂', Z₃' are linker structures formed by bioorthogonal groups and Z₂, Z₃, and each Z' can be the same or different from each other, and can also coexist or not independently;
L is a divalent linker connecting D, D₁ or D₂ with the remaining part of general formulas III-V; Preferably, L is selected from -(CH₂)ₐ-(OCH₂CH₂)_{b}-(NHCO)ₙ-(CH₂)_{c}-,
or selected from the following groups:

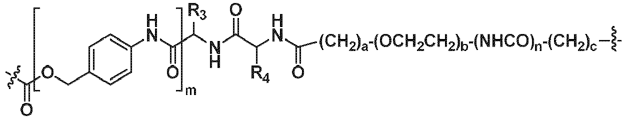

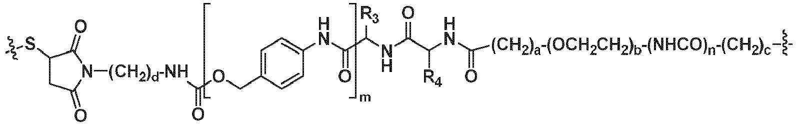

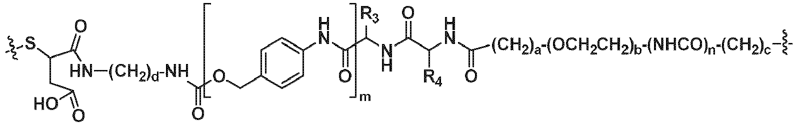

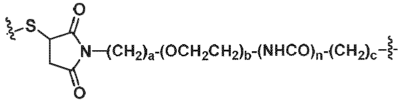

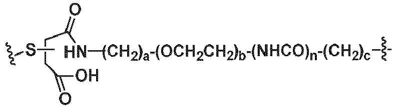

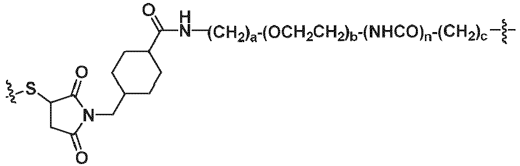

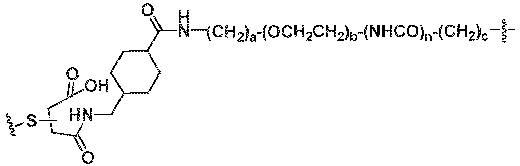

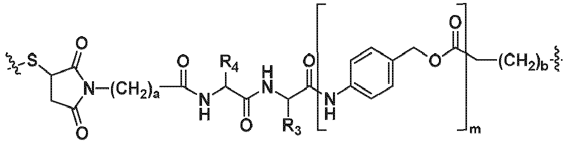

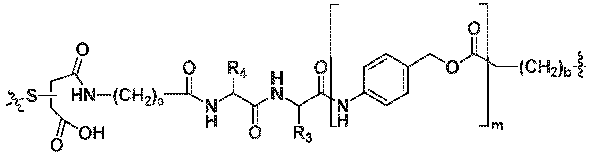

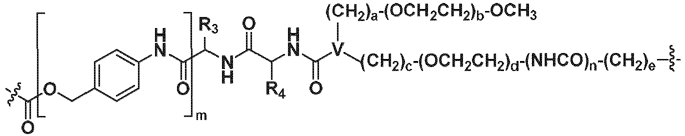

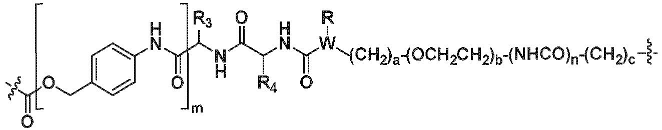

Wherein V and W are bifunctional linkers, including a structure with lysine and propargylglycine as bifunctional linkers, for example, L is selected from:

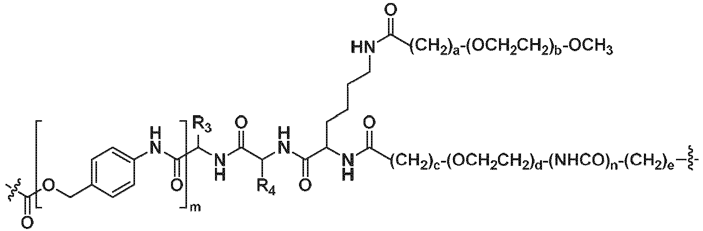

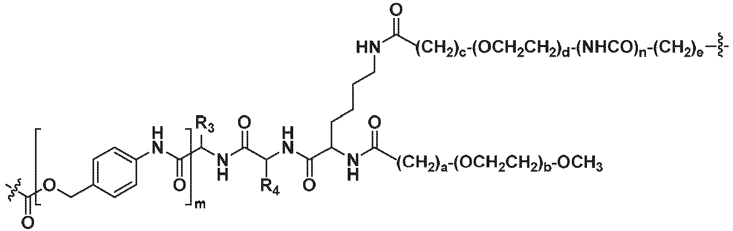

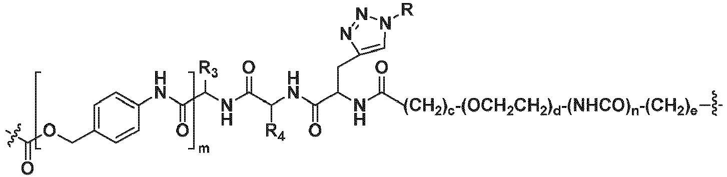

Wherein, a, b, c, d and e are each independently selected from integers between 0-30, m and n are 0 or 1, R₃ and R₄ are each independently selected from CH₃-, (CH₃)₂CH-, PhCH₂, NH₂(CH₂)₄-, NH₂CONH(CH₂)₃-, R is selected from azidizable monosaccharides, disaccharides, oligosaccharides or PEG structures with different lengths with azido groups, or combinations of PEG and chain or cyclic monosaccharides, disaccharides, and oligosaccharides, wherein the oligosaccharides include branched oligosaccharide chains; 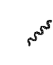 represents connection position;
D, D₁ and D₂ each independently represent a group derived from a cytotoxic compound, a small-molecule drug, or a fluorescent probe, and the small-molecule drug is preferably selected from maytansine, DM-1, DM-4, MMAE, MMAF, SN-38, Dxd, duocamycin, amanitin, PBDs, vincristine, vinblastine, vinorelbine, VP-16, camptothecin, paclitaxel, docetaxel, epothilone A. epothilone B, nocodazole, colchicine, estramustine, cemadotin, eleutherobin, fluorescent reagents, monosaccharides, disaccharides, oligosaccharides, and derivatives thereof, or The small molecule drug is a radiotherapeutic agent;
Preferably, D, D₁ and D₂ are each independently selected from the following groups:

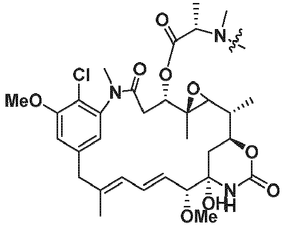

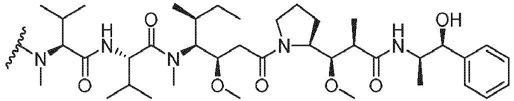

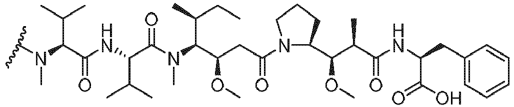

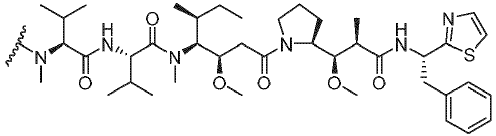

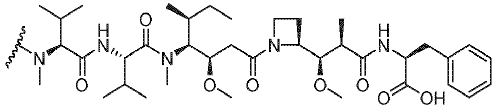

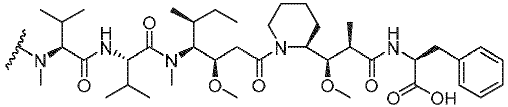

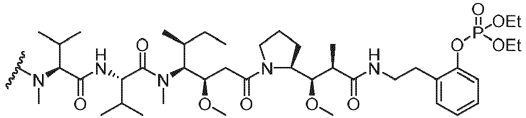

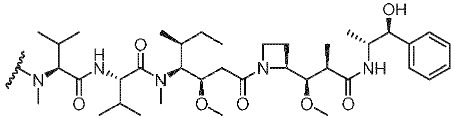

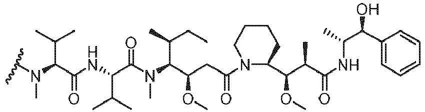

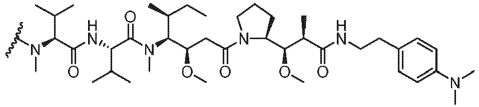

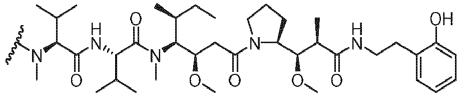

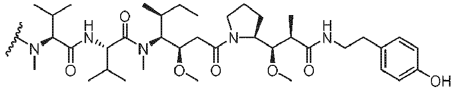

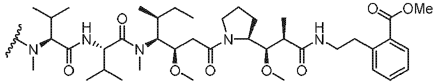

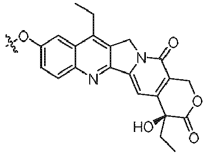

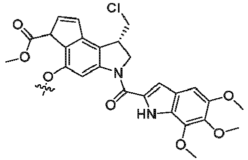

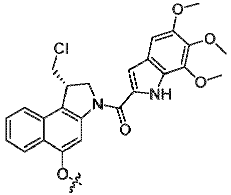

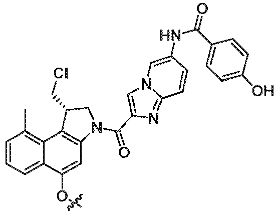

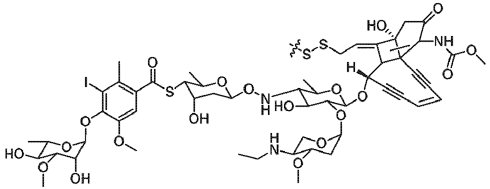

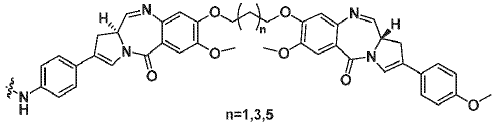

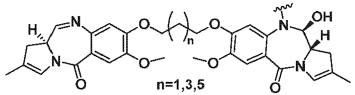

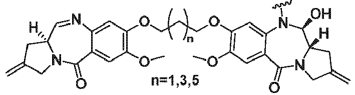

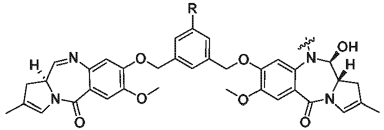

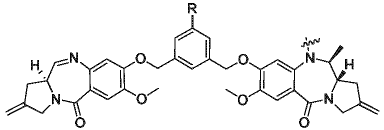

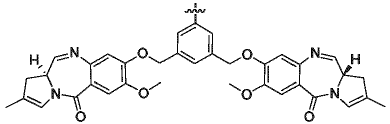

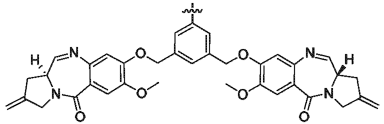

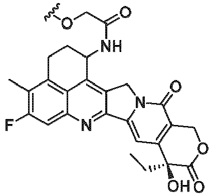

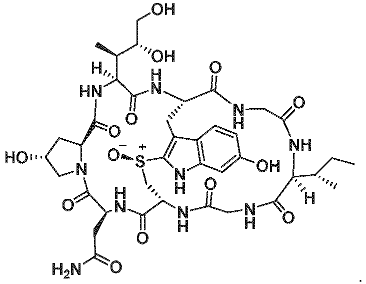

In a specific embodiment, the disaccharide-small molecule drug conjugate is represented by the following general formulas VI, VII, VIII:

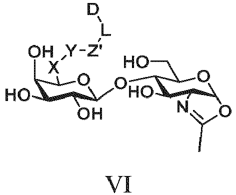

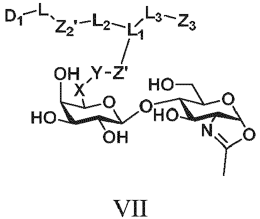

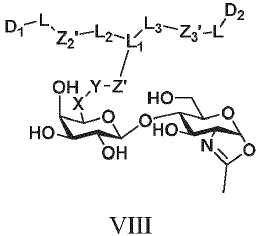

The respective substituents in the general formulas VI, VII and VIII are as defined above, respectively.

In a specific embodiment, the disaccharide-small molecule drug conjugate is selected from any of the following compounds:

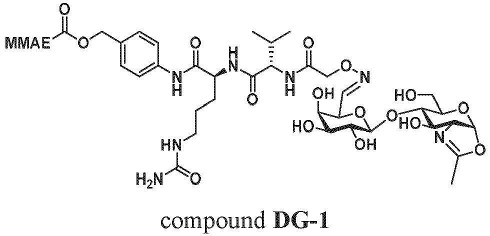

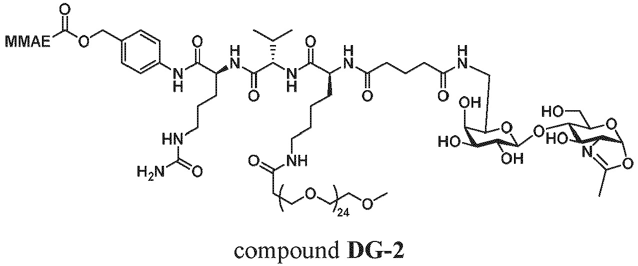

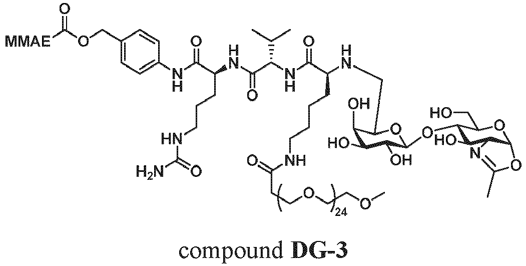

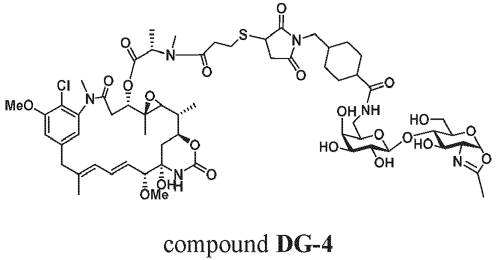

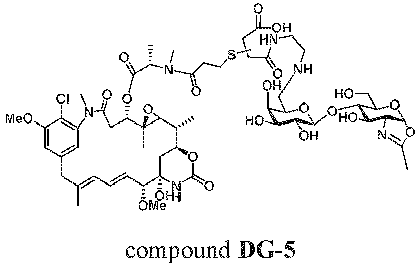

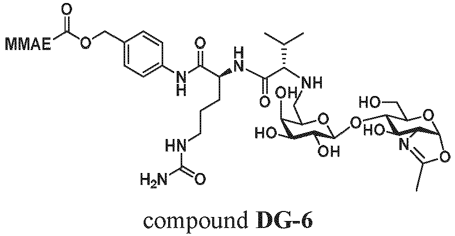

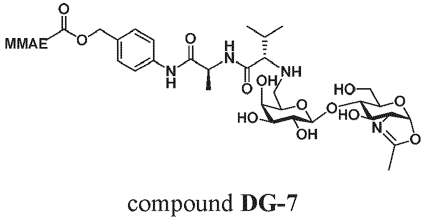

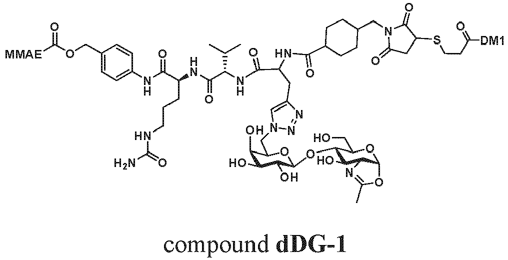

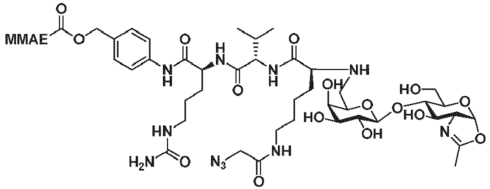

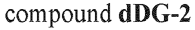

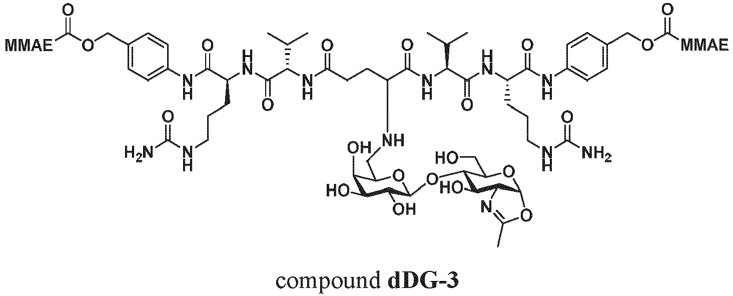

In each of the above structures, the structure of the MMAE moiety is: 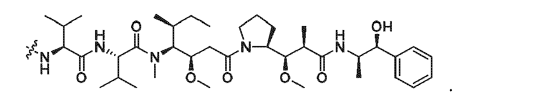 .

In yet another aspect, the present invention provides a glycoengineered antibody with the site-specific linkage at the N-glycosylation site of the Fc region of the antibody represented by the following general formula IX:

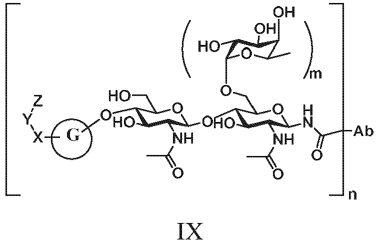

Wherein, in the above general formula IX, ring G and X, Y and Z are as defined above, respectively, m is selected from 0 or 1, n is selected from 1 or 2; Ab is a monoclonal antibody, a bifunctional antibody or a polyclonal antibody, which is a therapeutic antibody or a functional antibody originated from different species,
Preferably, Ab is selected from the group consisting of: trastuzumab, pertuzumab, rituximab, cetuximab, muromonab, gemtuzumab ozogamicin, abciximab, daclizumab, adalimumab, palivizumab, basiliximab, bevacizumab, panitumumab, nimotuzumab, denosumab, dixituzumab, Ramucirumab, necituzumab, ipilimumab, daratumumab, Brentuximab, alemtuzumab, elotuzumab, blinatumomab, nivolumab, pembrolizumab, atezolizumab, avelumab, durvalumab, toripalimab, catumaxomab, belintumumab, emicizumab, amivantamab (Rybrevant).

In a specific embodiment, the glycoengineered antibody is represented by the following general formula X:

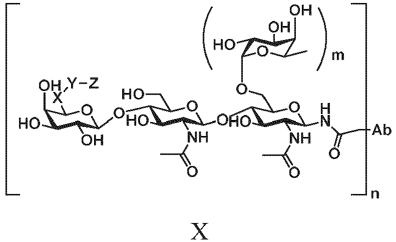

In the above general formula X, X, Y and Z are as defined above, respectively, m is selected from 0 or 1, n is selected from 1 or 2; Ab is an antibody.

In another aspect, the present invention provides a method for preparing the above-mentioned glycoengineered antibody, wherein the method is carried out by the following method I or the following method II:

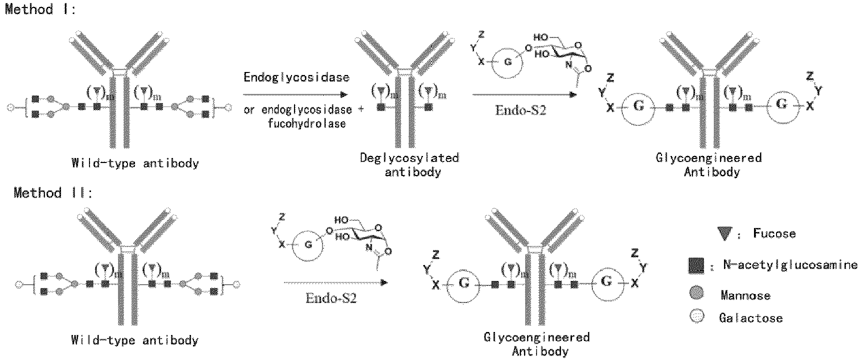

Wherein, in the above reaction scheme, m is selected from 0 or 1, and G ring, X, Y, and Z are as defined above, respectively,
Method I:
   The wild-type antibody is hydrolyzed by endoglycosidase or endoglycosidase combined with fucosidase to remove the heterogeneous sugar chain at the conservative glycosylation site of the natural antibody to obtain a deglycosylated antibody. The said disaccharide linker is then co-incubated with the wild-type antibody, and the disaccharide linker is connected to the conserved glycosylation site of the Fc domain of the antibody under the catalysis of the wild-type endoglycosidase, and the antibody of general formula X modified by a α1,6-acetylglucosamine disaccharide containing or not containing fucose is prepared, and the antibody is modified by the disaccharide linker of general formula I containing the orthogonal reactive group;
Method II:
   The said disaccharide linker is co-incubated with the wild-type antibody, the N-oligosaccharide structure of the Fc domain of the wild-type antibody is hydrolyzed under the catalysis of the wild-type endoglycosidase, and at the same time the disaccharide linker is connected to the conservative glycosylation site of the Fc domain of the antibody, thus the antibody of general formula X modified by a α1,6-acetylglucosamine disaccharide containing or not containing fucose is prepared, and the antibody is modified by the disaccharide linker of general formula I containing the orthogonal reactive group,
   Preferably, the wild-type endoglycosidase is N-acetylglucosaminidase, more preferably, the N-acetylglucosaminidase is Endo-S2 (Endoglycosidase-S2), for example, Endoglycosidase Endo-S2 derived from Streptococcus pyogenes; when preparing core-free fucosylated compounds, an endoglycosidase should be used with fucohydrolase together.

In a specific embodiment, the glycoengineered antibody is prepared by the following method I or II: 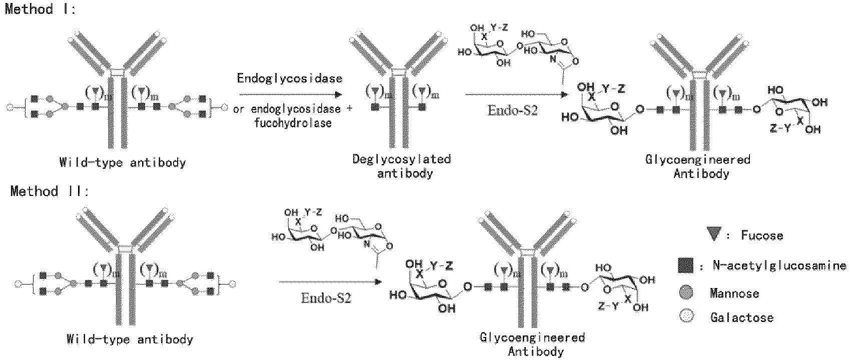 Wherein, in the above reaction scheme, m is selected from 0 or 1, and X, Y, and Z are as defined above, respectively,
Method I:
   The wild-type antibody is hydrolyzed by endoglycosidase or endoglycosidase combined with fucosidase to remove the heterogeneous sugar chain at the conservative glycosylation site of the natural antibody to obtain a deglycosylated antibody, and the said disaccharide linker and the wild-type antibody are then co-incubated, and the disaccharide linker is connected to the conserved glycosylation site of the Fc domain of the antibody under the catalysis of the wild-type endoglycosidase, and the antibody of general formula X modified by a α1,6-acetylglucosamine disaccharide containing or not containing fucose is prepared, and the antibody is modified by the disaccharide linker of general formula I containing an orthogonal reactive group;
Method II:
   The said disaccharide linker is co-incubated with the wild-type antibody, the N-oligosaccharide structure of the Fc domain of the wild-type antibody is hydrolyzed under the catalytic action of the wild-type endoglycosidase, and at the same time the disaccharide linker is connected to the conservative glycosylation site of the Fc domain of the antibody, thus the antibody of general formula X modified by a α1,6-acetylglucosamine disaccharide containing or not containing fucose is prepared, and the antibody is modified by the disaccharide linker of general formula I containing an orthogonal reactive group,
   Preferably, the wild-type endoglycosidase is N-acetylglucosaminidase, more preferably, the N-acetylglucosaminidase is Endo-S2 (Endoglycosidase-S2), for example, Endoglycosidase Endo-S2 derived from Streptococcus pyogenes; when preparing core-free fucosylated compounds, endoglycosidase should be used with fucohydrolase together.

In yet another aspect, the present invention provides an antibody-drug conjugate represented by the following general formula XI:

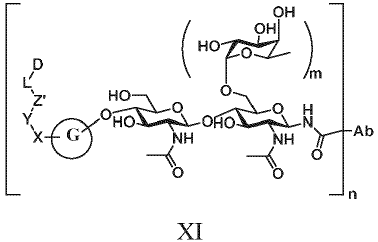

In general formula XI, ring G and X, Y, Z', L and D are as defined above, respectively, m is selected from 0 or 1, n is selected from 1 or 2; Ab is an antibody, and the connection site of the sugar structure is the conserved N-glycosylation site on antibody Fc.

In a specific embodiment, the antibody-drug conjugate is represented by the following general formula XII:

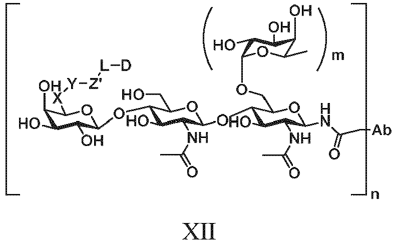

In formula XII, X, Y, Z', L and D are as defined above, respectively, m is selected from 0 or 1, n is selected from 1 or 2; Ab is an antibody, and the connection site of the sugar structure is the conserved N-glycosylation site on antibody Fc.

In addition, the present invention also provides an antibody-drug conjugate, which has the following structure: in the structures of general formula XI and general formula XII, -Z'-L-D is replaced by: 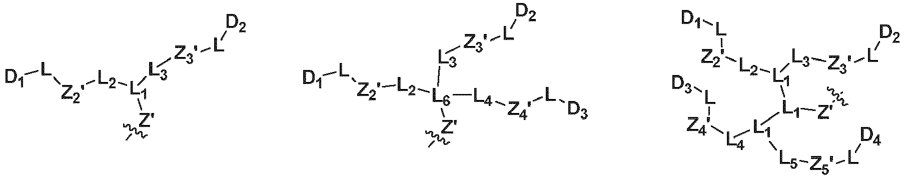 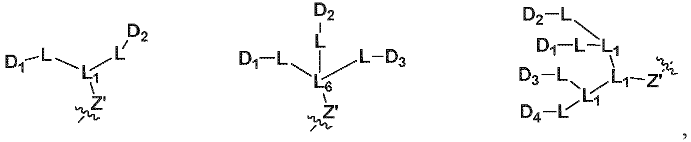 wherein, Z', L, L₁-L₆ and D₁ and D₂ are as defined above, respectively, and Z₂', Z₃', Z₄', Z₅' are linking fragments generated by the reaction between the bioorthogonal groups of functional molecules and Z₂, Z₃, Z₄, Z₅ respectively, they can be absent simultaneously or independently. The definitions of D₃ and D₄ are the same as those of D₁ and D₂. When the structures of D₁-D₄ are the same, the antibody-drug conjugate of general formula XI or XII represents a antibody-drug conjugate loading the same drug structure with high drug loading (drug-antibody ratio, drug to antibody ratio, DAR value), when D₁-D₄ are different, the antibody-drug conjugate of general formula XI or XII represents an antibody-drug conjugate loading different drug structures in a multidrug-form.

In another aspect, the present invention provides a method for preparing the above-mentioned antibody-drug conjugate, and the method includes the following two methods I and II:
Method I:
   a) The above-mentioned disaccharide linker is co-incubated with a wild-type antibody, the Asn297 N-oligosaccharide structure of the Fc domain of the wild-type antibody is hydrolyzed under the catalytic action of wild-type endoglycosidase, meanwhile the disaccharide linker is linked to the Asn297 site of the Fc domain of the antibody, or the above-mentioned disaccharide linker is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus preparing the antibody of general formula X modified by a α1,6-acetylglucosamine disaccharide containing or not containing fucose, which is modified by the disaccharide linker of general formula I containing the orthogonal reactive group,
   b) The antibody of general formula IX modified by a α1,6-acetylglucosamine disaccharide containing or not containing fucose, which is modified by the disaccharide linker of general formula I containing the orthogonal reactive group, obtained in step a), is coupled with a small molecule drug modified with a corresponding group capable of performing a specific coupling reaction with the orthogonal reactive group to prepare the antibody-drug conjugate of general formula XI or XII;
Method II:
   The above disaccharide-small molecule drug conjugate is co-incubated with a wild-type antibody, the Asn297 N-oligosaccharide structure of the Fc domain of the wild-type antibody is hydrolyzed under the catalytic action of wild-type endoglycosidase, meanwhile the disaccharide-small molecule drug conjugate is linked to the Asn297 site of the Fc domain of the antibody, or the above disaccharide-small molecule drug conjugate is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus the antibody-drug conjugate of general formula XI or XII is prepared.

In a specific embodiment, the wild-type endoglycosidase is N-acetylglucosaminidase, more preferably, the N-acetylglucosaminidase is Endo-S2 (Endoglycosidase-S2, derived from Streptococcus pyogenes endoglycosidase Endo-S2); when preparing non-core fucosylated compounds, endoglycosidase should be used with fucohydrolase together,
In a specific embodiment, in Method I, the orthogonal reactive group and the corresponding group capable of performing a specific coupling reaction with the orthogonal reactive group are selected from any combination of the following: azido group and alkynyl, mercapto and maleimide group, mercapto and mercapto or activated forms of mercapto, aldehyde group and amino, aldehyde group and aminooxy group or hydrazine group.

In a specific embodiment, in the step b) of Method I, the drug linker has the following groups, so as to be coupled with the small molecule drug modified by the corresponding group:

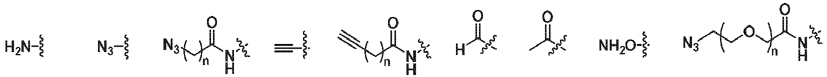

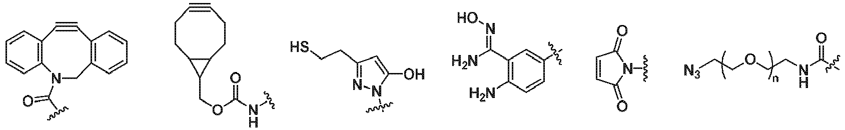

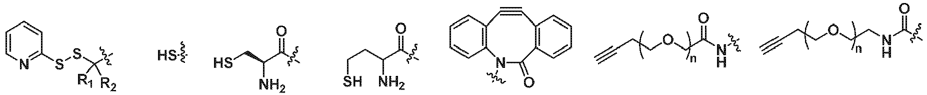

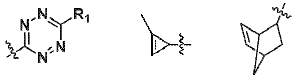

In a specific embodiment, the small molecule drug modified by the corresponding group is selected from the following compounds:

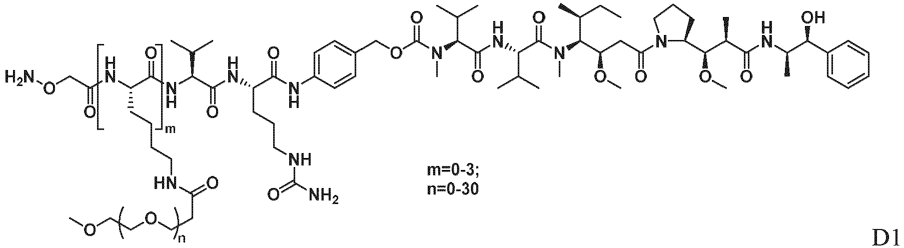

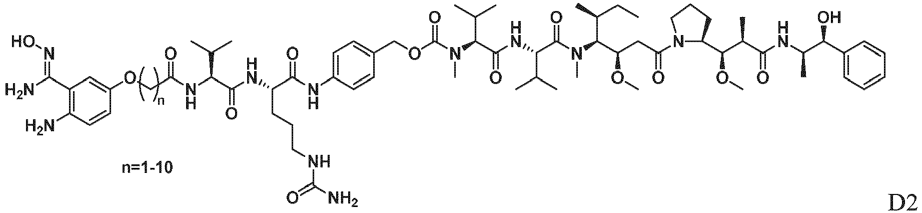

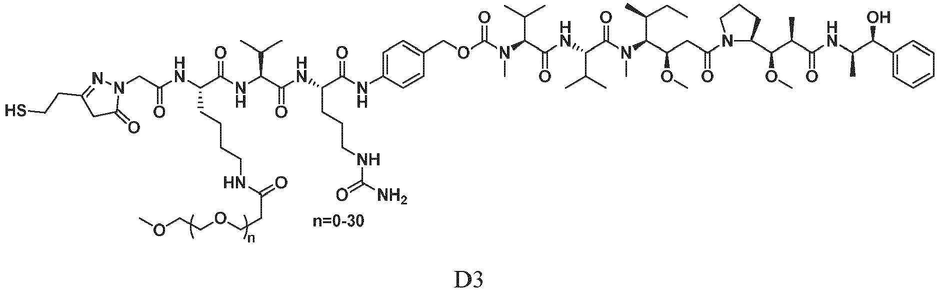

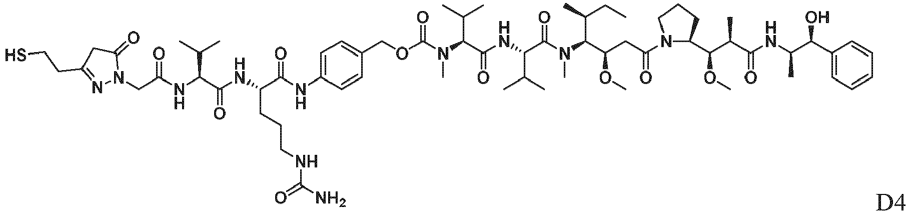

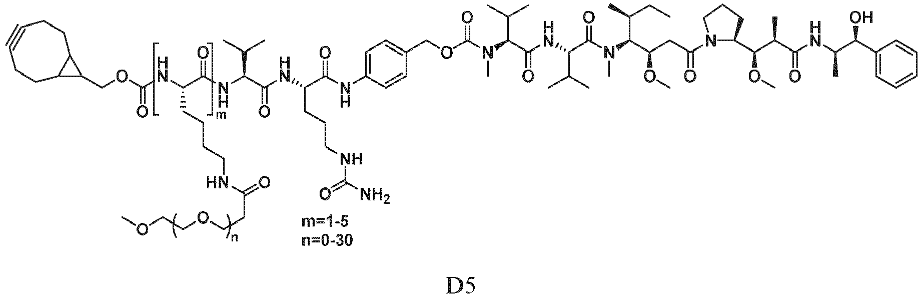

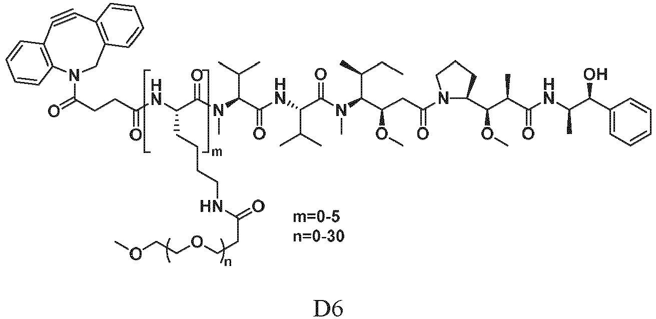

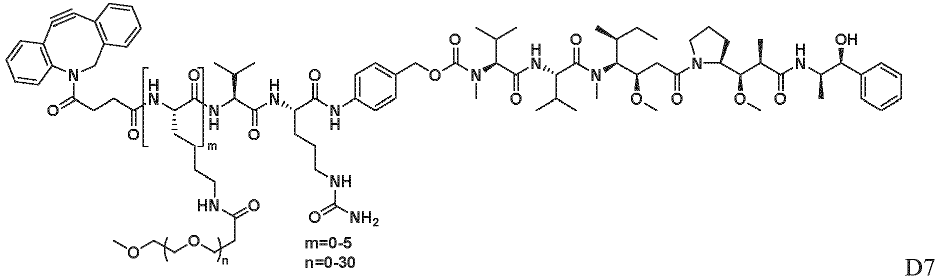

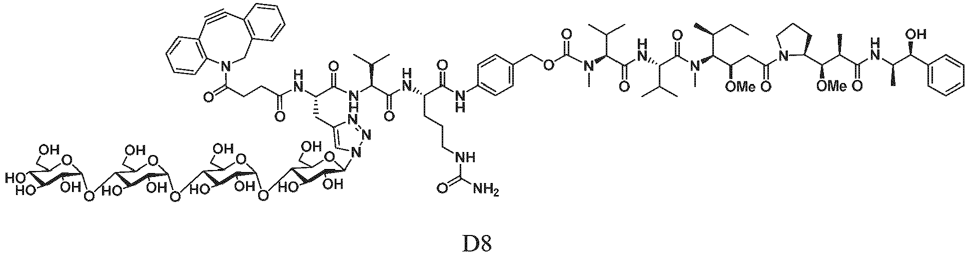

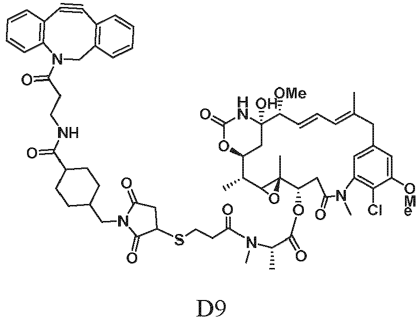

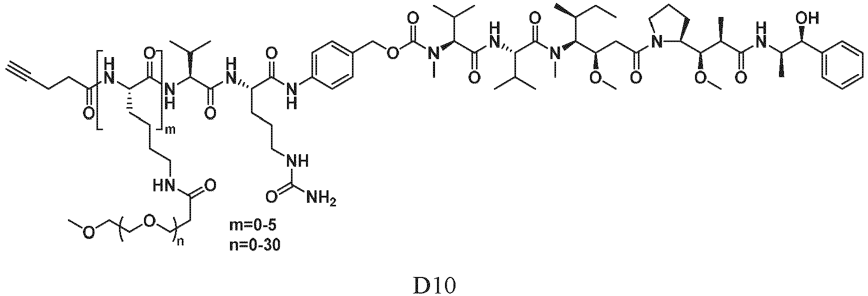

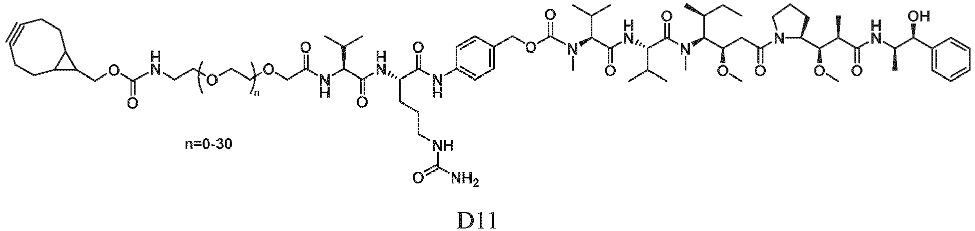

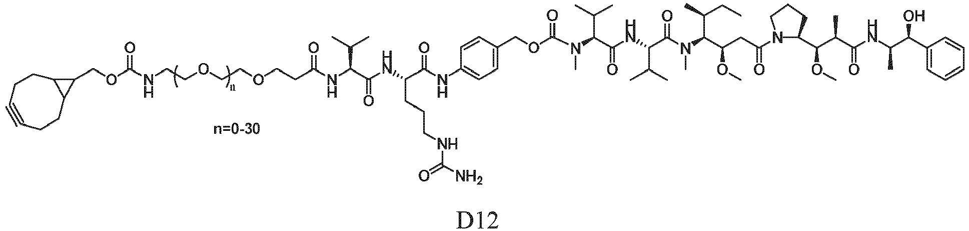

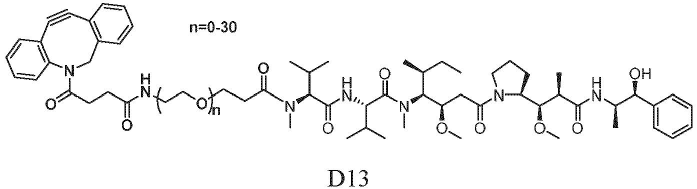

In a specific embodiment, Method I is performed as shown in the following reaction scheme:

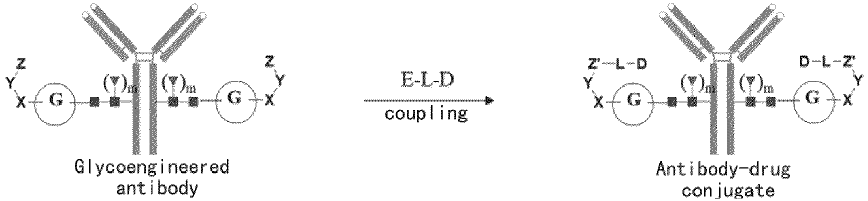

Wherein, in the above reaction scheme, m is selected from 0 or 1, X, Y, Z, Z', L, and D are as defined above, respectively; E is an orthogonal reactive group that can react with Z, wherein, the glycoengineered antibody in the reaction scheme can be obtained according to the method described above.

In a specific embodiment, Method I is performed as shown in the following reaction scheme:

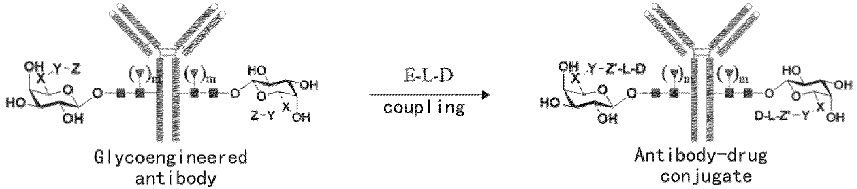

Wherein, in the above reaction scheme, m is selected from 0 or 1, X, Y, Z, Z', L, and D are as defined above, respectively; E is an orthogonal reactive group that can react with Z, wherein, the glycoengineered antibody in the reaction scheme is obtained according to the method described above.

In a specific embodiment, the preparation method is as shown in the following reaction scheme:

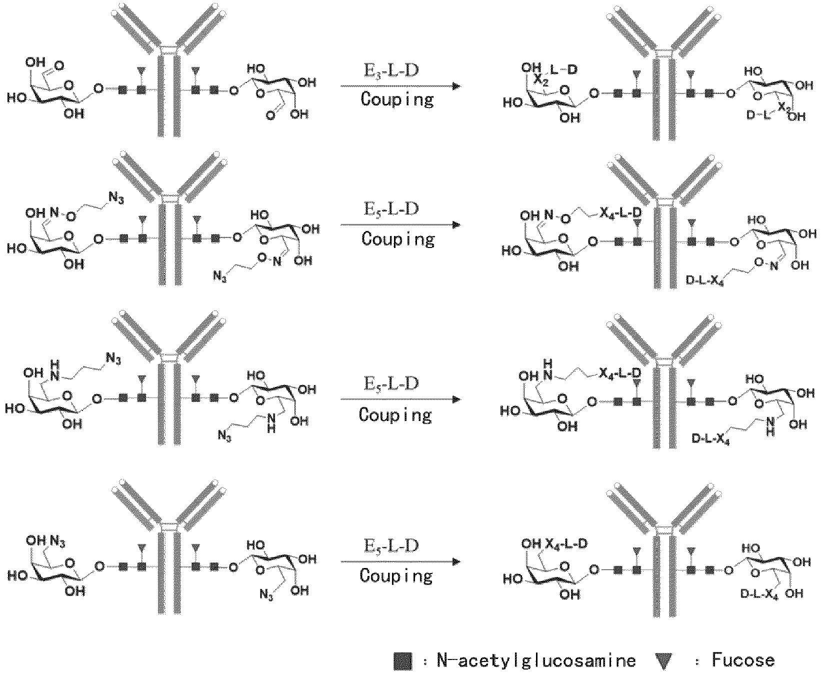

Wherein, L and D are as defined above, respectively, and E₃ is a corresponding group that reacts orthogonally with an aldehyde group, and is selected from thiopyrazolone, o-aminobenzamidoxime, and hydroxylamine, such as: 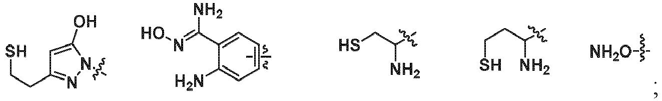
X₂ is the structure formed by the reaction between an aldehyde group and E₃;
E₅ is a corresponding group that undergoes an orthogonal reaction with an azido group, which is selected from a straight-chain alkynyl group, a DBCO structure, and a BCN structure, and X₄ is a structure formed by the reaction between an azido group and E₅.

In a specific embodiment, Method II includes:
As shown in the following two reaction schemes, the endoglycosidase is co-incubated with an antibody and the above-mentioned disaccharide-small molecule drug conjugate, when the N-oligosaccharide at the coSnserved glycosylation site Asn297 of the Fc domain of the antibody is hydrolyzed, the disaccharide-small molecule drug conjugate is transferred to Asn297 site (Method 1), or the above disaccharide-small molecule drug conjugate is co-incubated with a deglycosylated antibody and endoglycosidase (method II), in which the said deglycosylated antibody is obtained by treating the wild-type antibody with endoglycosidase in advance, and it can also be obtained by removing fucose using fucohydrolase at the same time, to realize the site-specific and quantitative introduction of small molecule drugs into the sugar chain, and obtain the corresponding antibody- drug conjugates.
Method I: 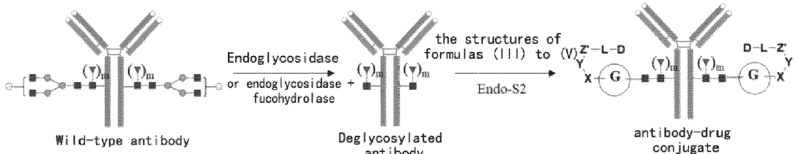
Method II: 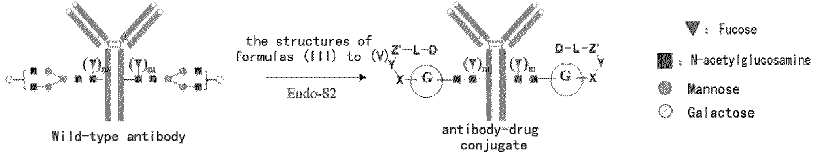
Method I: 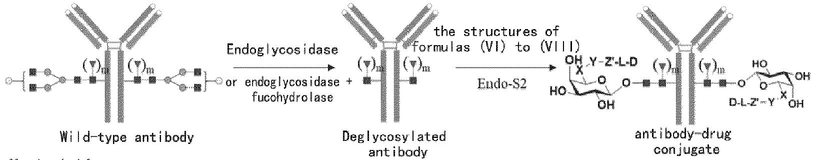
Method II:

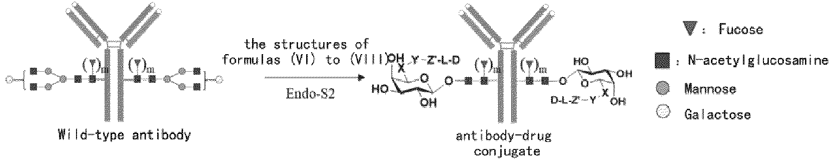

In another aspect, the present invention provides the use of the aforementioned disaccharide linker or disaccharide-small molecule drug conjugate in antibody glycoengineered modification or in the preparation of an antibody-drug conjugate.

In another aspect, the invention provides the use of the above antibody-drug conjugate in the preparation of drugs, pharmaceutical compositions or diagnostic reagents, wherein the drugs in the conjugate can be selected from anti-tumor drugs, anti-inflammatory drugs, antiviral drugs, anti-infectious diseases drugs or other immunotherapeutic drugs.

### Advantageous effect

The present invention enriches and develops new connection methods and structures based on the glycosite-specific modification, introduces reactions other than orthogonal reactions, such as amide reactions, for the disaccharide structure and drug linkers to form a novel site-specific and quantitative antibody-drug conjugate form, such that the antibody-drug conjugates have improved stability and cytotoxicity, and good druggability.

Based on the principle that the wild type endoglycosidase has hydrolytic activity to the N-oligosaccharide at the conservative glycosylation site of the antibody and also has transglycosylation activity to the specific novel disaccharide linker, the present invention designs and prepares disaccharide linkers with orthogonal reactive groups or disaccharide linkers with drug linkers or two drug linkers, and realizes the site-specific insertion of orthogonal reactive groups on the antibody through enzyme catalytic reaction, further achieves site-specific and quantitative coupling with small molecule drugs or site-specific and quantitative coupling with small molecule drugs through enzyme catalyzed reactions by a direct one-step method. This process has the advantage that there is no need to hydrolyze heterogeneous N-oligosaccharides at conserved glycosylation sites on the Fc domain of wild-type antibodies in advance, thus reducing purification steps, and thus the operation is simple and can be easily industrialized.

The site-specific antibody-drug conjugates of general formulas IX and X prepared from the disaccharide linkers of general formulas I and II have a uniform chemical structure with fixed point quantitative modification, and have advantages e.g., the structure is definite and uniform, and quality is controllable when compared to the marketed antibody-drug conjugates having non homogeneous structures. At the same time, this preparation method has advantages in simple operation, fewer purification steps and the like, over other site-specific coupling methods, and have good drug resistance and can be easily industrialized. At the same time, the site-specific antibody-drug conjugates exhibit good anti-tumor activity.

### BRIEF DESCRIPTION OF THE DRAWINGS

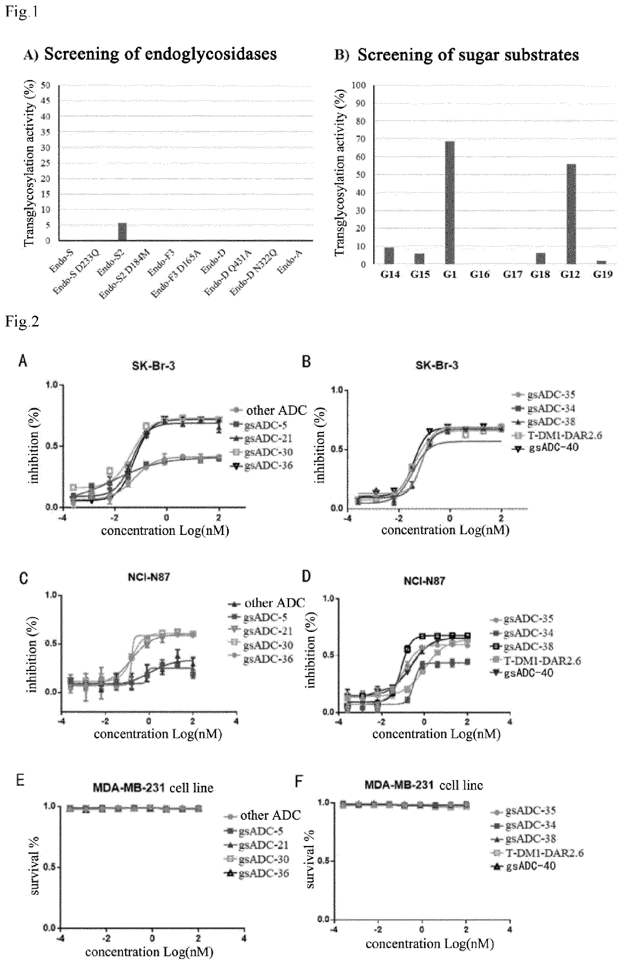

Figure 1 shows the screening results of the sugar substrates and endoglycosidase in the early stage of this application. A: Screening different glycoside endonucleases for antibody transglycosylation activity against G14; B: Screening the capability of endoglycosidase Endo-S2 of recognizing and transferring different sugar substrates to antibody glycosylation sites.
Figure 2 shows the *in vitro* activity experimental results of some ADCs connected by the disaccharide linker of the present application. **A:** The inhibitory rate of some gsADCs (gsADC-5, gsADC-21, gsADC-30, gsADC-36) on SK-Br-3 cells; **B**: The inhibitory rate of some gsADC (gsADC-35, gsADC-34, gsADC-38) on SK-Br-3 cells; **C:** The inhibitory rate of some gsADCs (gsADC-5, gsADC-21, gsADC-30, gsADC-36) on NCI-N87 cells; **D:** The inhibitory rate of some gsADC (gsADC-35, gsADC-34, gsADC-38) on NCI-N87 cells; **E:** The effect of some gsADC (gsADC-5, gsADC-21, gsADC-30, gsADC-36) on the survival rate of MDA-MB-231 cells; **F:** The effect of some gsADC (gsADC-35, gsADC-34, gsADC-38) on the survival rate of MDA-MB-231 cells.

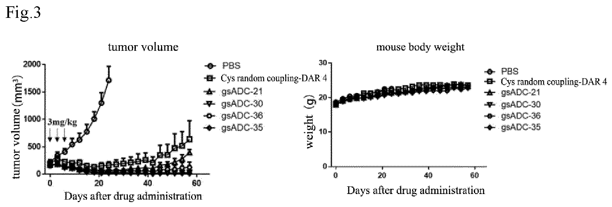

Figure 3 shows the *in vivo* activity experimental results of some ADCs connected by the disaccharide linkers of the present application, showing the inhibitory effects on tumor volume *in vivo* (A) and the effect on body weight of nude mice (B) of gsADC-21, gsADC-30, gsADC-35, and gsADC-36, respectively.

### DETAILED DESCRIPTION

Term: "multivalent linker" herein refers to a linker which has a valence greater than divalent.

The glycosidase used in the invention is expressed in the Escherichia coli system in the laboratory. The small molecule cytotoxic drug DM1 and MMAE used in the invention were purchased from RESUPERPHARMTECH (Shanghai); DBCO and BCN compounds were purchased from Chengdu Bioconebio Co., Ltd; 3-azidopropylamine was purchased from J&K Scientific (shanghai); N-acetyl-D-lactosamine and acetonitrile were purchased from Shanghai Acmec Biochemical Co., Ltd; BTTAA was purchased from Taizhou Greenchem Company; Galactose oxidase, catalase and horseradish peroxidase were purchased from Sangon Biotech (Shanghai); Amino acid compounds were purchased from JL Biochem (Shanghai) Ltd; 4-pentyne acid was purchased from Shanghai Bide Pharmaceutical Technology Co., Ltd. Other compounds and reagents of which the manufacturers are not specified were purchased from SINOPHARM Chemical Reagent Co., Ltd.

The instruments and chromatographic columns used in the present invention include: Waters Xevo G2-XS QTOF, analytical high-performance liquid chromatography (Thermo ultimate 3000), analytical high-performance liquid chromatography (Beijing Innovation Tongheng LC3000), and preparative high-performance liquid chromatography (Beijing Innovation Tongheng LC3000); Thermo C18 (AcclaimTM 120,5 µm. 4.6 x 250 mm), Agilent SB-C18 (5 µm. 4.6 x 150 mm), Waters C18 column (ACQUITY UPLC BEH C18, 1.7 µm. 2.1 × 50 mm). Instrument for measuring antibody molecular weight: Liquid chromatography-mass spectrometry (LC-MS), Waters Xevo G2-XS QTOF, equipped with Waters C4 (ACQUITY UPLC Protein BEH C4, 1.7 µm. 2.1 mm x 50 mm).

The preparation route of the disaccharide linker, glycoengineered antibody, and glycosite-specific antibody-drug conjugate (ADC) in the present application is illustrated and summarized in the following reaction scheme.

### General preparation example

### The synthesis route of the disaccharide linkers is as follows:

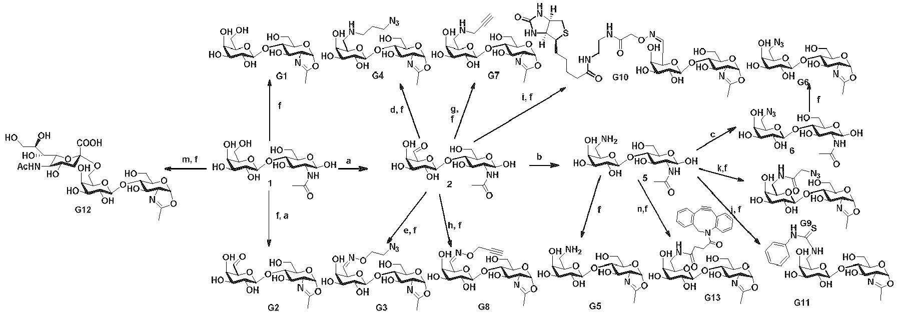

**Note:** a. Galactose oxidase GOase, catalase, horseradish peroxidase HRP, O₂, pH 7.0, 30°C; b. Hydroxylamine hydrochloride, sodium carbonate, rt; Sodium borohydride, nickel chloride hexahydrate, 4°C; c. 1H imidazol-1-sulfonyl azide hydrochloride, potassium carbonate, copper sulfate, 37°C; d. 3-azidopropylamine, NaCNBH₃, pH 6.0, 0°C; e. O-(2-azido ethyl) hydroxylamine hydrochloride, pH 7.4, 37°C; f. DMC, triethylamine, 0°C or CDMBI, potassium phosphate, 0°C; g. propargyl amine, NaCNBH₃, pH 6.0, 0°C; h. O-(2-propargyl) hydroxylamine hydrochloride, pH 7.4, 37°C; i. Biotin-ONH₂, pH 7.4, 37°C; j. FITC-NCS, pH 7.4, 37°C; k. Azidoacetic acid active ester, pH 7.4; m. CMP sialic acid (as shown in compound 70 below), α-2,6-sialyltransferase, 100 mM Tris buffer, pH 8.0. n. DBCO-CONHS **80**, pH 7.4/DMF.

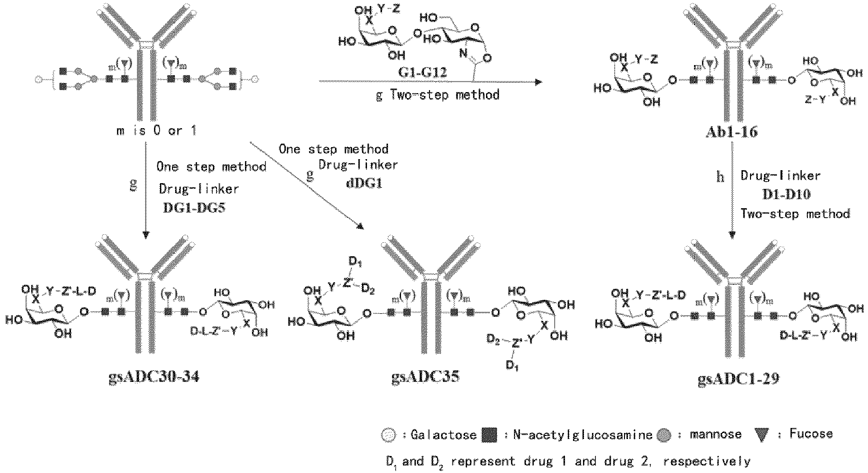

**Note:** g. Endo-S2, containing (not containing) a certain amount of DMSO, DMA, or DMF as co solvents, buffer solution of pH 7.0, 30°C; h. buffer of pH 7.0 containing (not containing) a certain amount of DMSO, DMA, or DMF as co solvents, general operation 3 to general operation 10.

### General operation 1:

### Method 1 for preparing non natural glycoengineered antibodies:

The derivative disaccharide oxazoline (i.e. compounds **G1-G11, G13-G14**) and sialic acid-derived disaccharide oxazoline (i.e. compound **G12**) as prepared, wild-type antibody, and wild-type endoglycosidase Endo-S2 are incubated each at concentrations of 0.5 mM, 5 mg/mL, and 0.4 mg/mL, respectively, in a reaction system of pH 7.0 at 30°C for 0.5 to 12 hours. After purification by protein A, the desired non natural glycoengineered antibodies **Ab-1** to **Ab-14** are obtained, as shown in **examples 37-50** below.

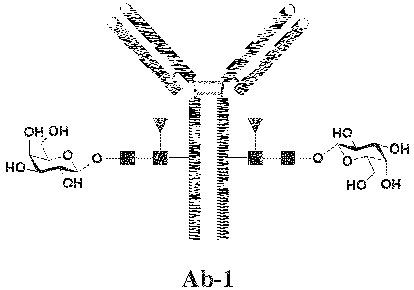

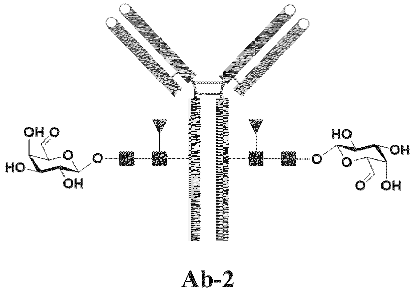

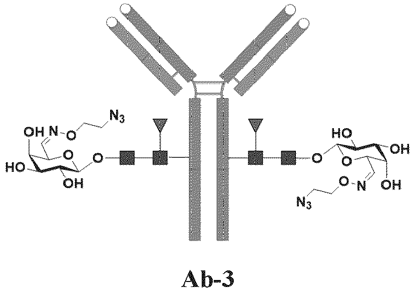

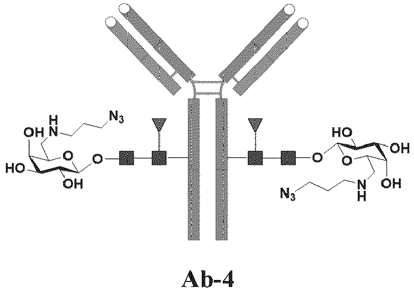

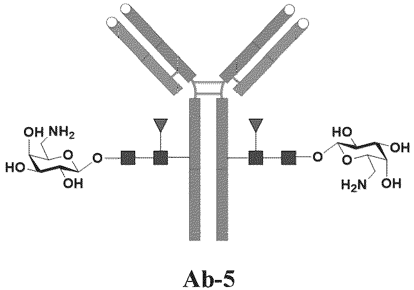

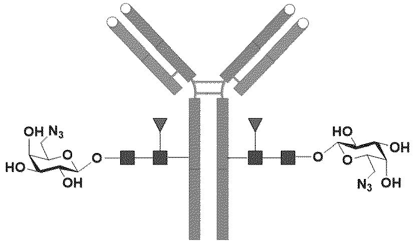

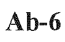

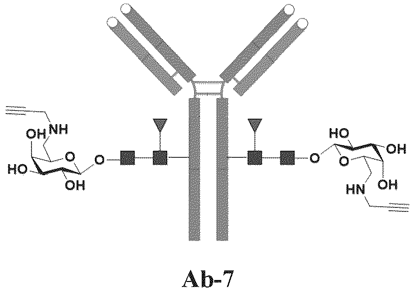

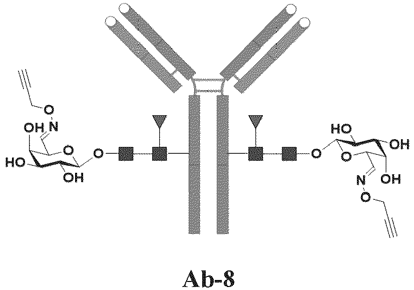

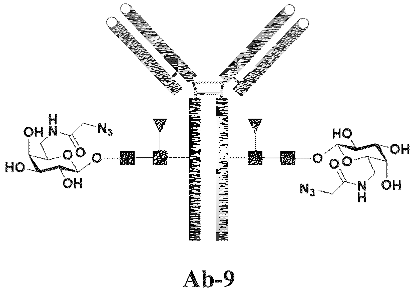

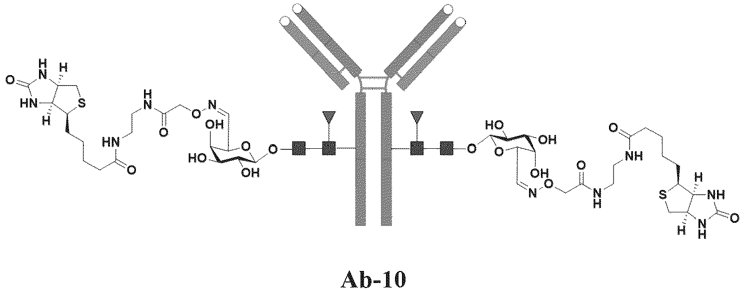

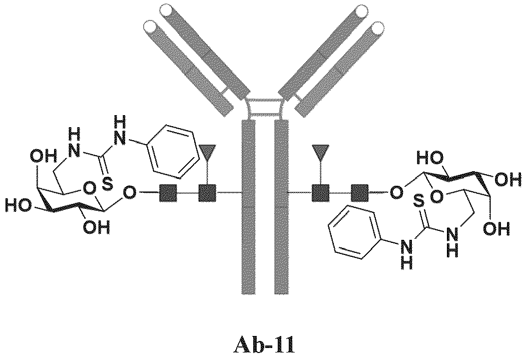

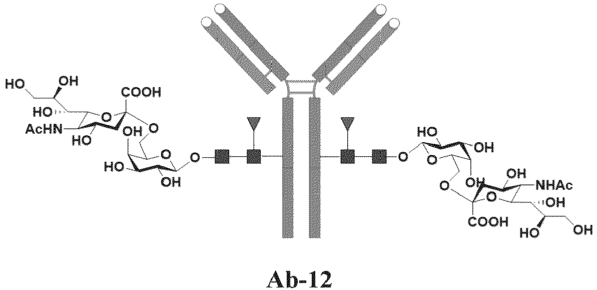

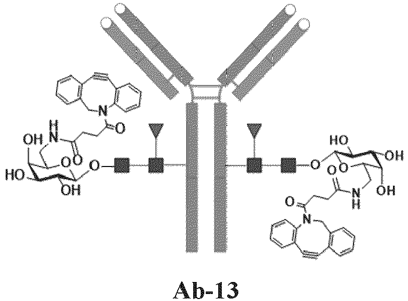

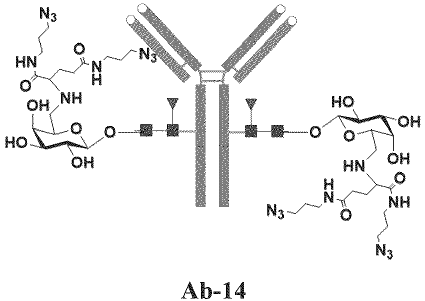

### General operation 2:

### Method 2 for preparing non natural glycoengineered antibodies:

The derivative disaccharide oxazoline (i.e. compounds **G3, G8, G10, G13**) and sialic acid-derived disaccharide oxazoline (i.e. compound **G12**) as prepared, the defucosylated antibody, and wild-type endoglycosidase Endo-S2 are incubated each at concentrations of 0.5 mM, 5 mg/mL, and 0.4 mg/mL, respectively, in a reaction system of pH 7.0 at 30°C for 0.5 to 12 hours. After purification by protein A, the desired non natural glycoengineered antibodies **Ab-15** to **Ab-19** are obtained, as shown in **examples 51-55** below.

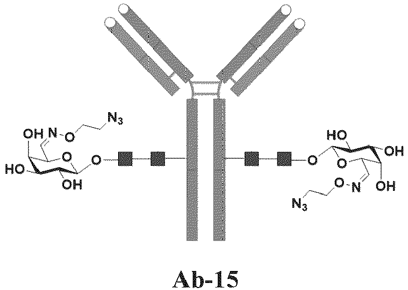

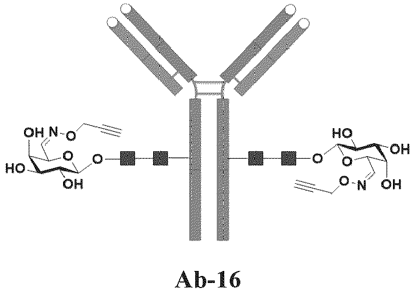

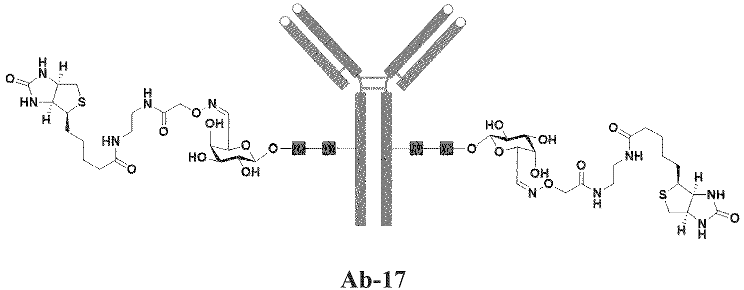

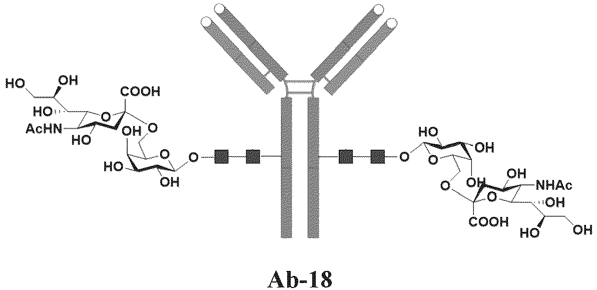

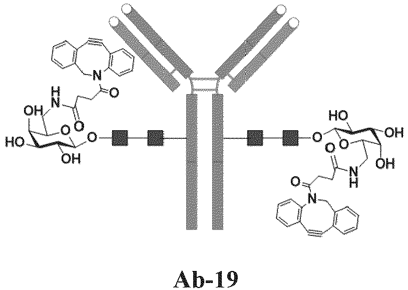

### General operation 3:

### Method for preparing glycosite-specific antibody-drug conjugate (ADC) in one step:

The drug-linker disaccharide oxazoline (i.e. compounds **DG-1 to DG-7, dDG1 to dDG3**) as prepared, wild-type antibody, and wild-type endoglycosidase Endo-S2 are incubated each at concentrations of 0.5 mM, 5 mg/mL, and 0.4 mg/mL, respectively, in a reaction system of pH 7.0 at 30°C for 0.5 to 12 hours. After it is converted into the product as confirmed by LC-MS, after purification by protein A, the desired glycosite-specific and quantitative antibody-drug conjugates **gsADC-30 to gsADC-37** are obtained, as shown in **examples 89-96** below.

### General operation 4:

### Method 1 for preparing site-specific ADC based on aldehyde disaccharide antibody:

The disaccharide antibody containing aldehyde group (i.e. non natural glycoengineered antibody **Ab-2**) as prepared, drug-linker containing 2-aminobenzamidoxime group (i.e. compound **D2**) are incubated each at concentrations of 5 mg/mL, and 0.3 mM, respectively, in a reaction system of pH 7.0-7.4 at 37°C. After it is completely converted into the product as confirmed by LC-MS, after purification by protein A, the desired glycosite-specific and quantitative antibody-drug conjugate **gsADC-1** is obtained, as shown in **example 56** below.

### General operation 5:

### Method 2 for preparing site-specific ADC based on aldehyde disaccharide antibody:

The disaccharide antibody containing aldehyde group (i.e. non natural glycoengineered antibody **Ab-2**) as prepared, drug-linker containing aminooxy group (i.e. compound **D1**) are incubated each at concentrations of 5 mg/mL, and 0.3 mM, respectively, in a reaction system of pH 7.5 at 37°C. After it is completely converted into the product as confirmed by LC-MS, after purification by protein A, the desired glycosite-specific and quantitative antibody-drug conjugate **gsADC-2** is obtained, as shown in **example 57** below.

### General operation 6:

### Method 3 for preparing site-specific ADC based on aldehyde disaccharide antibody:

The disaccharide antibody containing aldehyde group (i.e. non natural glycoengineered antibody **Ab-2**) as prepared, drug-linker containing thioPz group (i.e. compounds **D3** and **D4**) are added to concentrations of 5 mg/mL, and 0.3 mM, respectively, then adding EDTA, 10% TritonX-100 to the final concentrations of 0.5mM and 1%, the reaction system of pH 5.5 is incubated at 37°C. After it is completely converted into the product as confirmed by LC-MS, after purification by protein A, the desired glycosite-specific and quantitative antibody-drug conjugates **gsADC-3 and gsADC-4** are obtained as shown in **examples 58 and 59** below.

### General operation 7:

### Method 1 for preparing site-specific ADC based on azide disaccharide antibody:

The disaccharide antibody containing azide group (i.e. non natural glycoengineered antibody **Ab-3, Ab-4, Ab-6, Ab-9, Ab-15**) as prepared, DBCO drug-linker (i.e. compounds **D6-D9 and D13**) are incubated each at concentrations of 5 mg/mL, and 0.3 mM, respectively, in a reaction system of pH 7.4 at 37°C. After it is completely converted into the product as confirmed by LC-MS, after purification by protein A, the desired glycosite-specific and quantitative antibody-drug conjugates **gsADC-5~gsADC-20, gsADC-29** are obtained, as shown in **examples 60-76** below.

### General operation 8:

### Method 2 for preparing site-specific ADC based on azide disaccharide antibody:

The disaccharide antibody containing azide group (i.e. non natural glycoengineered antibody **Ab-3, Ab-4, Ab-6, Ab-9, Ab-14, Ab-15**) as prepared, BCN drug-linker (i.e. compounds **D5,**

**D11, D12)** are incubated each at concentrations of 5 mg/mL, and 0.3 mM, respectively, in a reaction system of pH 7.4 at 37°C. After it is completely converted into the product as confirmed by LC-MS, after purification by protein A, the desired glycosite-specific and quantitative antibody-drug conjugates **gsADC-21~ gsADC-24, gsADC-39, gsADC-41** ~ **gsADC-43** are obtained, as shown in **examples 77-84** below.

### General operation 9:

### Method 3 for preparing site-specific ADC based on azide disaccharide antibody:

The disaccharide antibody containing azide group (i.e. non natural glycoengineered antibody **Ab-3, Ab-4, Ab-6, Ab-9**) as prepared, straight chain alkyne drug-linker (i.e. compound D10) are added to concentrations of 5 mg/mL, and 0.3 mM, respectively, then adding 6 mM Cu(I)-BTTAA solution (preparation: 21 µL of ddH₂O, 3 µL of 60 mM CuSO₄ solution, 3 µL of 300 mM BTTAA solution, and 3 µL of 900 mM sodium ascorbate solution are added in sequence) to the final concentration of 0.5 mM, then the reaction system of pH 7.4 is incubated at 37°C. After it is completely converted into the product as confirmed by LC-MS, after purification by protein A, the desired glycosite-specific and quantitative antibody-drug conjugates **gsADC-25~ gsADC-28** are obtained, as shown in **examples 85-89** below.

### General operation 10:

### Method for preparing site-specific ADC based on defucosylated disaccharide:

The drug-linker disaccharide oxazoline (i.e. compound **DG-6**) as prepared, defucosylated antibody, and wild-type endoglycosidase Endo-S2 are incubated each at concentrations of 0.5 mM, 5 mg/mL, and 0.4 mg/mL, respectively, in a reaction system of pH 7.0 at 30°C for 1 hour.

After it is completely converted into the product as confirmed by LC-MS, after purification by protein A,the desired glycosite-specific and quantitative antibody-drug conjugate **gsADC-38** is obtained, as shown in **example 97** below.

The specific processes for preparing disaccharide linkers, glycoengineered antibodies, and glycosite-specific ADCs using the aforementioned general preparation method are described by the specific examples as below.

### I: Preparation of disaccharide linkers

### Example 1: Synthesis of compounds G1-G2

### The structures and synthesis methods of compounds G1-G2 are as follows:

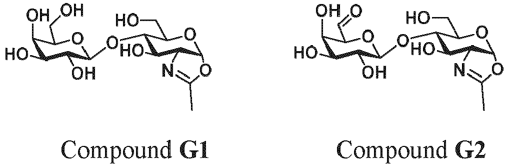

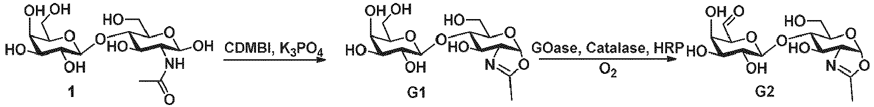

Step 1: Compound **1** (20 mg, 52.2 µmol) was weighed and dissolved in 800 µL of 50 mM PB, pH 7.0 buffer, and 2-chloro-1,3-dimethyl-1H-benzimidazole-3-chloride (CDMBI, 56.4 mg, 261 µmol) was added to the above reaction system, mixed well and cooled to 0 °C, adding potassium phosphate (166 mg, 0.783 mmoL), then adding ddH₂O to a total volume of 1044 µL and reacted at 0 °C for 2 hours. It was observed that plenty of precipitation was generated, which was removed by centrifugation, and a non-desalinated compound **G1** (dissolved in water to obtain a 50 mM stock) was obtained. After aliquoting, the stock was stored at -80 °C for use.

HRMS, calculated for C₁₄H₂₃NO₁₀ [M+H]⁺366.14, found 366.1322
Step 2: After blowing O₂ into Compound G1 (5 mg, 274 µL of 50 mM stock) obtained in step 1 for 10 min, 11.9 U of galactose oxidase GOase, 120 U of horseradish peroxidase HRP, and 2.38 kU of catalase were added to the reaction system to achieve a volume of 300 µL. The reaction system was kept at 30 °C, at 888 rpm for 4 h, and then the resultant was separated and purified by using a P2 column. After adding 1 equivalent of NaOH and freeze drying, Compound **G2** was obtained (which is dissolved to obtain a 50 mM stock, solvent 50 mM PB, pH 7.0), After aliquoting, the stock was stored at -80 °C for use. HRMS, calculated for C₁₄H₂₁NO₁₀ [M+H]⁺364.1243, found 364.1201.

### Example 2: Synthesis of compound G3

### The structure and synthesis method of compound G3 are as follows:

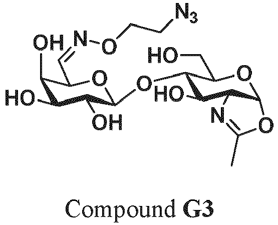

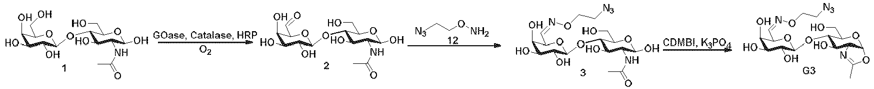

Step 1: Compound 1 (20 mg, 52.2 µmol) was weighed and dissolved in 1 mL of 50 mM PB, pH 7.0 buffer solution, followed by blowing O₂ for 10 min, and then 47.6 U of galactose oxidase GOase, 480 U of horseradish peroxidase HRP, and 9.52 kU of catalase were added to the above reaction system to a volume of 1.19 mL. The reaction system was kept at 30 °C, at 888 rpm for 4 h, and then the resultant was purified by using a P2 column and freeze dried to obtain compound **2** (18 mg, yield 90.5%). HRMS, calculated for C₁₄H₂₃NO₁₁ [M+H]⁺ 382.1349, found 382.1331
Step 2: Compound **2** (18 mg, 47.2 µmol) was weighed and dissolved in 200 µL of 50 mM PB, pH 7.4 buffer, and Compound **12** (5.3 mg, 51.9 µmol) was added to the above reaction system and reacted at 37 °C for 2 hours. When the reaction was complete as monitored by LC-MS, the resultant was separated and purified using a semi preparative C18 column to obtain compound **3** (20 mg, yield 91%). HRMS, calculated for C₁₄H₂₁NO₁₀ [M+H]⁺466.1785, found 466.1732. Step 3: Compound **3** (20 mg, 43 µmol) was weighed and dissolved in a 700 µL of 50 mM PB, pH 7.0 buffer. CDMBI (46.44 mg, 215 µmol) was added to the above reaction system, mixed well, and cooled to 0 °C. After adding potassium phosphate (137 mg, 0.645 mmoL), ddH₂O was added to a total volume 860 µL. The system was reacted at 0 °C for 2 hours. It was observed that plenty of precipitation was generated, which was removed by centrifugation to obtain a reaction system containing compound **G3.** HRMS, calculated for C₁₆H₂₅N₅O₁₀ [M+H]⁺ 448.1679, found 448.1616. ¹H NMR (600 MHz, Deuterium Oxide) δ 7.57 (d, *J* = 4.8 Hz, 0.65H), 6.91 (d, *J* = 4.6 Hz, 0.35H), 6.01 (dd, *J* = 7.3, 2.3 Hz, 1H), 4.43 (d, *J* = 7.8 Hz, 0.7H), 4.38 (d, *J* = 7.9 Hz, 0.3H), 4.34 (m, 0.3H), 4.33 - 4.3 (m, 1H), 4.25 - 4.15 (m, 3H), 4.11 (m, 1H), 3.98 (dd, *J=* 3.4, 1.1 Hz, 0.7H), 3.74 (m, 1H), 3.67 - 3.57 (m, 3H), 3.53 - 3.44 (m, 3H), 3.40 - 3.35 (m, 1H), 1.99 (t, *J* = 1.9 Hz, 3H).

### Example 3: Synthesis of compound G4

### The structure and synthesis method of compound G4 are as follows:

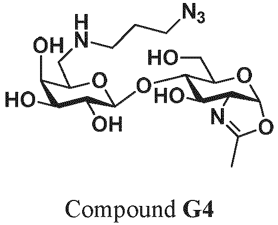

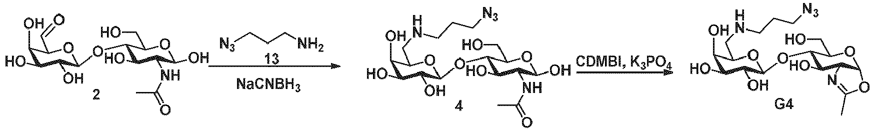

Step 1: Compound **2** (18 mg, 47.2 µmol) was weighed and dissolved in 200 µL of 0.2 M PB, pH 6.0 buffer, and compound **13** (23.6 mg, 236 µmol) and sodium cyanide borohydride (NaCNBH₃) (59.5 mg, 944 µmol) were added to the above reaction system, and reacted at 0 °C for 4 hours. When the reaction was complete as monitored by LC-MS, the resultant was purified by using a P2 column to obtain compound **4** (16 mg, yield 72.8%). HRMS, calculated for C₁₇H₃₁N₅O₁₀ [M+H]⁺466.2149, found 466.2132.

Step 2: Compound **4** (16 mg, 34.4 µmol) was weighed and dissolved in a 500 µL 50 mM PB, pH 7.0 buffer. CDMBI (37.2 mg, 172 µmol) was added to the above reaction system, mixed well, and cooled to 0 °C, adding potassium phosphate (109.6 mg, 0.516 mmoL), and ddH₂O to a total volume of 688 µL and reacted at 0 °C for 2 hours. It was observed that plenty of precipitation was generated, which was removed by centrifugation to obtain the supernatant containing compound **G4.** HRMS, calculated for C₁₇H₂₉N₅O₉ [M+H]⁺ 448.2043, found 448.2102.

¹H NMR (600 MHz, Deuterium Oxide) δ 5.97 (d, *J* = 7.3 Hz, 1H), 4.29 (d, *J* = 7.9 Hz, 1H), 4.27 (dd, *J* = 3.0, 1.6 Hz, 1H), 4.07 (m, 1H), 3.72 (d, *J* = 3.5 Hz, 1H), 3.69 (dd, *J* = 12.3, 2.5 Hz, 1H), 3.63 - 3.58 (m, 1H), 3.56 - 3.49 (m, 2H), 3.40 - 3.35 (m, 1H), 3.31 (m, 1H), 3.29 (t, *J* = 6.7 Hz, 3H), 2.93 (q, *J* = 7.4 Hz, 2H), 2.68 (q, *J* = 7.3 Hz, 2H), 1.95 (d, *J* = 1.7 Hz, 3H), 1.67 (p, *J* = 7.1 Hz, 2H).

### Example 4: Synthesis of compound G5

### The structure and synthesis method of compound G5 are as follows:

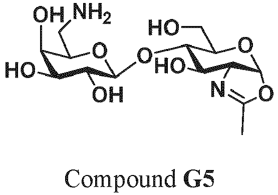

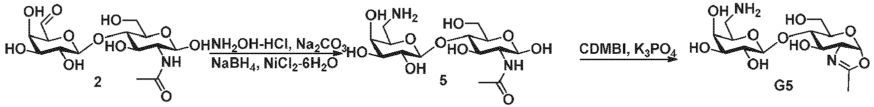

Step 1: Compound **2** (18 mg, 47.2 µmol) was weighed and dissolved in 200 µL of 50 mM PB, pH 7.0 buffer. Hydroxylamine hydrochloride (3.6 mg, 52 µmol) and 180 µL of methanol were added to the above reaction system, mixed well, and slowly added with sodium carbonate (2.6 mg, 23.6 µmol). After reacting at room temperature for 3 hours, nickel chloride hexahydrate (28 mg, 118 µmol/L) and sodium borohydride (26.8 mg, 0.7 mmoL) were added to the system. The reaction system was kept at 4 °C overnight, and then centrifuged to obtain a supernatant, and the precipitate was washed twice with water. The washing liquids were combined and purified using a P2 column, followed by freeze-drying to obtain compound **5** (15 mg, yield 83%). HRMS, calculated for C₁₄H₂₆N₂O₁₀ [M+H]⁺383.1665, found 383.1661.

Step 2: Compound **5** (15 mg, 39.25 µmol) was weighed and dissolved in a 600 µL of 50 mM PB, pH 7.0 buffer. CDMBI (42.4 mg, 196.3 µmol) was added to the above reaction system, mixed well, cooled to 0 °C, adding potassium phosphate (125 mg, 0.589 mmoL), and adding ddH₂O to a total volume of 785 µL, and reacted at 0 °C for 2 hours. It was observed that plenty of precipitation was generated, which was removed by centrifugation to obtain a supernatant containing compound **G5.** HRMS, calculated for C₁₄H₂₄N₂O₉ [M+H]⁺365.156, found 365.1521.

### Example 5: Synthesis of compound G6

### The structure and synthesis method of compound G6 are as follows:

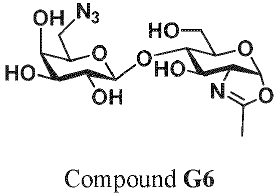

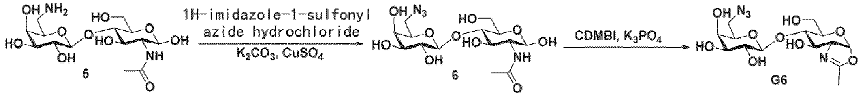

Step 1: Compound **5** (15 mg, 39.35 µmol) was weighed and dissolved in a 500 µL of CH₃OH/H₂O=1:4 system. 1H-imidazole-sulfonyl azide hydrochloride (12.3 mg, 58.9 µmol), potassium carbonate (16.3 mg, 117.75 µmol), and copper sulfate (6.3 mg, 39.25 µmol) were added to the above reaction system, and reacted at 37 °C for 4 hours. When the reaction was almost complete as monitored by LC-MS, the resultant was separated and purified using a P2 column and freeze dried to obtain compound **6** (13 mg, yield 81.3%). HRMS, calculated for C₁₄H₂₄N₄O₁₀ [M+H]⁺409.157, found 409.1526.

Step 2: Compound **6** (13 mg, 31.85 µmol) was weighed and dissolved in 500 µL of 50 mM PB, pH 7.0 buffer. CDMBI (34.4 mg, 159.3 µmol) was added to the above reaction system, mixed well, and cooled to 0 °C. adding potassium phosphate (101.5 mg, 0.478 µmol), and adding ddH₂O to a total volume of 637 µL and reacted for 2 hours at 0 °C. It was observed that plenty of precipitation was generated, which was removed by centrifugation to obtain a supernatant containing compound **G6.** HRMS, calculated for C₁₄H₂₂N₄O₉ [M+H]⁺ 391.1465, found 391.1432.

### Example 6: Synthesis of compound G7

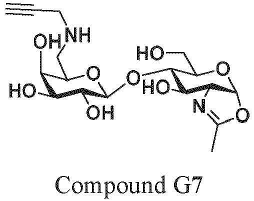

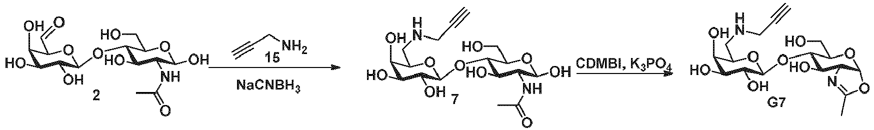

Step 1: Compound 2 (18 mg, 47.2 µmol) was weighed and dissolved in 200 µL 0.2 M PB, pH 6.0 buffer. Compound **15** (13 mg, 236 µmol) and sodium cyanide borohydride (59.5 mg, 944 µmol) were added to the above reaction system, and reacted 0 °C for 4 hours. When the reaction was complete as monitored by LC-MS, the resultant was separated and purified by using a P2 column to obtain compound **7** (14 mg, yield 70.5%). HRMS, calculated for C₁₇H₂₈N₂O₁₀ [M+H]⁺ 421.1822, found 421.1876

Step 2: Compound **7** (16 mg, 38 µmol) was weighed and dissolved in a 500 µL 50 mM PB, pH 7.0 buffer. CDMBI (41 mg, 190 µmol) was added to the above reaction system, mixed well, and cooled to 0 °C, adding potassium phosphate (121 mg, 0.57 mmoL), and ddH₂O to a total volume of 688 µL, and reacted at 0 °C for 2 hours. It was observed that plenty of precipitation was generated, which was removed by centrifugation to obtain a reaction system containing compound **G7.** HRMS, calculated for C₁₇H₂₆N₂O₉ [M+H]⁺403.1716, found 403.1733. ¹H NMR (600 MHz, Deuterium Oxide) δ 5.97 (d, *J* = 7.3 Hz, 1H), 4.33 - 4.23 (m, 2H), 4.11 - 4.03 (m, 1H), 3.74 (d, *J* = 3.6 Hz, 1H), 3.69 (dd, *J* = 12.3, 2.5 Hz, 1H), 3.63 - 3.58 (m, 1H), 3.56 - 3.48 (m, 2H), 3.40 - 3.35 (m, 1H), 2.90 - 2.86 (m, 3H), 2.67 - 2.62 (m, 2H), 1.95 (d, *J* = 1.8 Hz, 3H).

### Example 7: Synthesis of compound G8

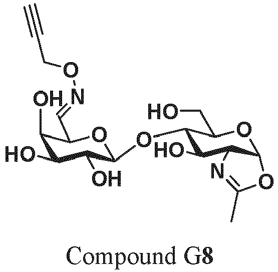

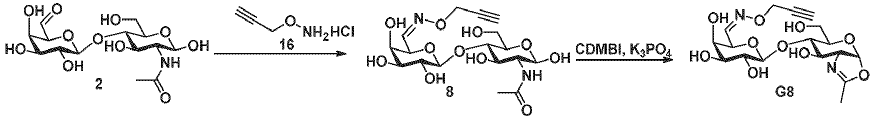

Step 1: Compound **2** (18 mg, 47.2 µmol) was weighed and dissolved in 200 µL of 50 mM PB, pH 7.4 buffer. Compound **16** (5.6 mg, 51.9 µmol) was added to the above reaction system and reacted at 37 °C for 2 hours. When the reaction was complete as monitored by LC-MS, the resultant was separated and purified by using a semi preparative C18 column to obtain compound **8** (18.1 mg, yield 88%). HRMS, calculated for C₁₇H₂₆N₂O₁₁ [M+H]⁺ 435.1615, found 435.1610.

Step 2: Compound **8** (18.1 mg, 41.6 µmol) was weighed and dissolved in a 700 µL of 50 mM PB, pH 7.0 buffer. CDMBI (45 mg, 208 µmol) was added to the above reaction system, mixed well, and cooled to 0 °C. adding potassium phosphate (132.5 mg, 0.624 mmoL), ddH₂O to a total volume of 860 µL, and reacted at 0 °C for 2 hours. It was observed that plenty of precipitation was generated, which was removed by centrifugation to obtain a supernatant containing compound **G8.** HRMS, calculated for C₁₇H₂₄N₂O₁₀ [M+H]⁺ 417.1509, found 417.1505.

¹H NMR (600 MHz, Deuterium Oxide) δ 7.53 (d, *J* = 4.9 Hz, 0.65H), 6.95 (d, *J* = 4.8 Hz, 0.35H), 5.98 (d, *J* = 7.3 Hz, 1H), 4.61 (d, *J* = 9.9 Hz, 2H), 4.43 - 4.25 (m, 3H), 4.15 - 4.05 (m, 1.3H), 3.95 (d, *J* = 3.4 Hz, 0.7H), 3.76 - 3.67 (m, 1H), 3.65 - 3.53 (m, 3H), 3.46 (dd, *J* = 10.1, 7.8 Hz, 1H), 3.36 (m, 1H), 1.96 (d, *J* = 1.6 Hz, 3H).

### Example 8: Synthesis of compound G9

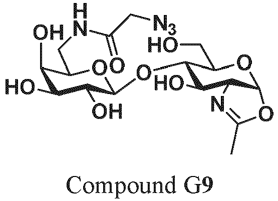

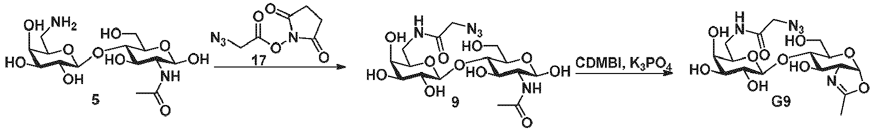

Step 1: Compound **5** (15 mg, 39.3 µmol) was weighed and dissolved in 200 µL of 50 mM PB, pH 7.4 buffer. Compound **17** (23.3 mg, 117.9 µmol) was added to the above reaction system and reacted at room temperature for 4 hours. When the reaction was complete as monitored by LC-MS, the resultant was separated and purified by using a P2 gel column to obtain compound **9** (14 mg, yield 76.7%), HRMS, calculated for C₁₆H₂₇N₅O₁₁ [M+H]⁺466.1785, found 466.1725. Step 2: Compound **9** (14 mg, 30.1 µmol) was weighed and dissolved in 500 µL of D₂O. CDMBI (32.5 mg, 150.5 µmol) was added to the above reaction system, mixed well, and cooled to 0 °C, adding potassium phosphate (96 mg, 0.45 mmoL), D₂O to a total volume of 602 µL, and reacted at 0 °C for 2 hours. It was observed that plenty of precipitation was generated, which was removed by centrifugation to obtain a supernatant containing compound **G9.** HRMS, calculated for C₁₆H₂₅N₅O₁₀ [M+H]⁺448.1679, found 448.1666.

### Example 9: Synthesis of compound G10

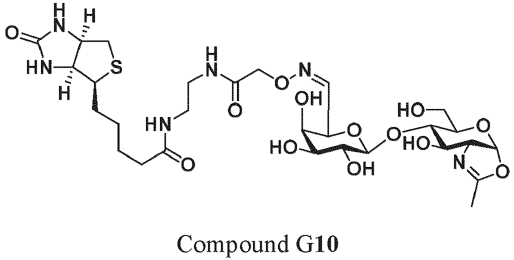

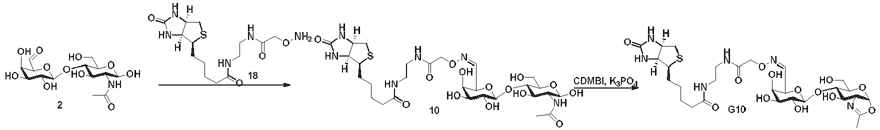

Step 1: Compound **2** (20 mg, 52.48 µmol) was weighed and dissolved in a 200 µL of 50 mM PB buffer, pH 7.4. Compound **18** (22.6 mg, 63 µmol) was added to the above reaction system, and kept at room temperature for 8 hours. Thereafter, the reaction was complete as monitored by LC-MS, the resultant was separated and purified by using a semi preparative C18 column to obtain compound **10** (32.6 mg, yield 86%). HRMS, calculated for C₂₈H₄₆N₆O₁₄S [M+H]⁺ 723.2871, found 723.2877.

Step 2: Compound **10** (10 mg, 13.85 µmol) was weighed and dissolved in D₂O. CDMBI (15 mg, 69.3 µmol) was added to the above reaction system, mixed well, and cooled to 0 °C, adding potassium phosphate (44 mg, 208 µmol), and D₂O to a total volume of 277 µL, and reacted at 0 °C for 2 hours. It was observed that plenty of precipitation was generated, which was removed by centrifugation to obtain a supernatant containing compound **G10.** HRMS, calculated for C₂₈H₄₄N₆O₁₃S [M+H]⁺705.2765, found 705.2771.

### Example 10: Synthesis of compound G11

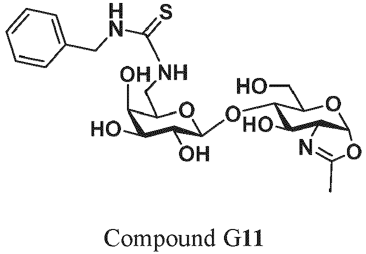

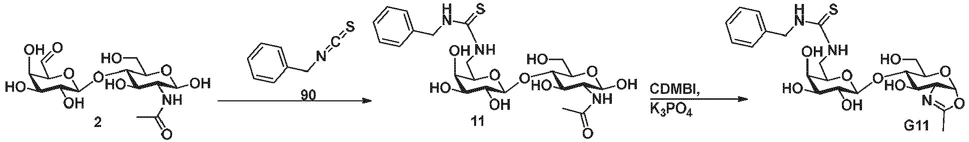

Step 1: Compound **2** (20 mg, 52.48 µmol) was weighed and dissolved in 200 µL of 50 mM PB, pH 7.4 buffer. Compound **90** Benzyl-NCS (9.4 mg, 63 µmol) was added to the above reaction system, which was kept at room temperature for 8 hours. When the reaction was complete as monitored by LC-MS, the resultant was separated and purified by using a semi preparative C18 column to obtain compound **11** (17 mg, 62%). HRMS, calculated for C₂₂H₃₃N₃O₁₀S [M+H]⁺ 532.1965, found 532.1911.

Step 2: Compound **11** (17 mg, 32.4 µmol) was weighed and dissolved in D₂O. CDMBI (35 mg, 162 µmol) was added to the above reaction system, mixed well, and cooled to 0 °C, adding potassium phosphate (103 mg, 0.486 mmoL), and D₂O to a total volume of 650 µL, and reacted at 0 °C for 2 hours. It was observed that plenty of precipitation was generated, which was removed by centrifugation to obtain a supernatant containing compound **G11.** HRMS, calculated for C₂₂H₃₁N₃O₉S [M+H]⁺ 514.1859, found 514.1880.

¹H NMR (600 MHz, Deuterium Oxide) δ 7.32 (t, *J* = 7.6 Hz, 2H), 7.25 (dd, *J* = 7.9, 5.7 Hz, 3H), 5.97 (d, *J* = 7.3 Hz, 1H), 4.7 (m, 2H), 4.39 - 4.15 (m, 2H), 4.04 (m, 1H), 3.70 (dd, *J* = 12.3, 2.5 Hz, 4H), 3.56 (dd, *J* = 12.3, 6.4 Hz, 3H), 3.51 - 3.43 (m, 1H), 3.43 - 3.31 (m, 2H), 1.97 (m, 3H).

### Example 11: Synthesis of compound G12

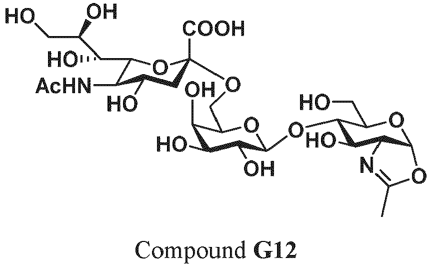

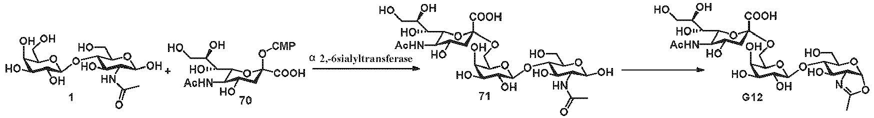

Step 1: Compound **1** (10 mg, 26.1 µmol) and compound **70** (16 mg, 26.1 µmol) were weighed and dissolved in 1.5 mL of 100 mM Tris buffer at pH 8.0. 30 µg of α 2,6-sialyltransferase (α 2,6-sialyltransferase (Pd2,6ST) was added to the above reaction system. When the reaction was complete as monitored by TLC plate, the resultant was separated and purified by using a P2 column to obtain compound **71** (12.6 mg, yield 72%). HRMS, calculated for C₂₅H₄₂N₂O₁₉ [M+H]⁺ 675.246, found 675.2433.

Step 2: Compound **71** (2 mg, 2.97 µmol) was weighed and dissolved in D₂O. CDMBI (3.2 mg, 14.83 µmol) was added to the above reaction system, mixed well, and cooled to 0 °C, adding potassium phosphate (9.5 mg, 44.55 µmol), and D₂O to a total volume of 148 µL, and reacted at 0 °C for 2 hours. It was observed that plenty of precipitation was generated, which was removed by centrifugation to obtain a supernatant containing compound **G12.** HRMS, calculated for C₂₅H₄₀N₂O₁₈ [M+H]⁺657.2354, found 657.2301.

¹H NMR (600 MHz, Deuterium Oxide) δ 5.71 (d, *J* = 7.3 Hz, 1H), 4.07 - 3.99 (m, 2H), 3.83 - 3.77 (m, 1H), 3.60 - 3.47 (m, 4H), 3.46 - 3.35 (m, 3H), 3.35 - 3.18 (m, 7H), 3.15 - 3.05 (m, 3H), 2.63 (m, 1H), 1.69 (d, *J* = 1.9 Hz, 3H), 1.66 (s, 3H), 1.30 (t, *J* = 12.1 Hz, 1H).

### Example 12: Synthesis of compound G13

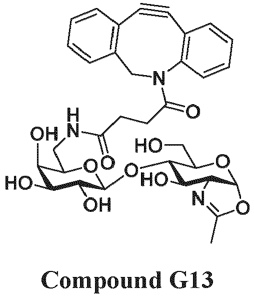

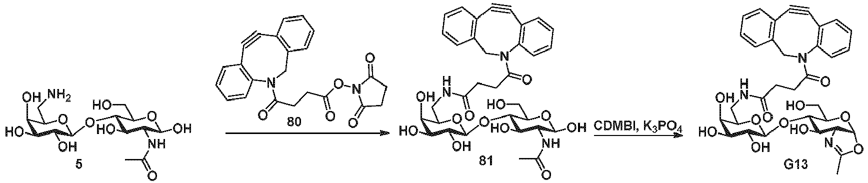

Step 1: Compound **5** (15 mg, 39.3 µmol) was weighed and dissolved in 200 µL of 50 mM PB, pH 7.4 buffer. Compound **80** (23.7 mg, 59 µmol) was added to the above reaction system and reacted at room temperature for 4 hours. When the reaction was complete as monitored by LC-MS, the resultant was separated and purified by using a semi preparative column to obtain compound **81** (22 mg, yield 83.7%). HRMS, calculated for C₃₃H₃₉N₃O₁₂ [M+H]⁺ 670.2612, found 670.2661.

Step 2: Compound **81** (10 mg, 14.9 µmol) was weighed and dissolved in 500 µL of D₂O. CDMBI (16.2 mg, 74.7 µmol) was added to the above reaction system, mixed well, and cooled to 0 °C, adding potassium phosphate (31.6 mg, 149 µmol), and D₂O to a total volume of 600 µL, and reacted at 0 °C for 2 hours. It was observed that plenty of precipitation was generated, which was removed by centrifugation to obtain a supernatant containing compound **G13.** HRMS, calculated for C₃₃H₃₇N₃O₁₁ [M+H]⁺ 652.2506, found 652.2501.

### Example 13: Synthesis of compound G14

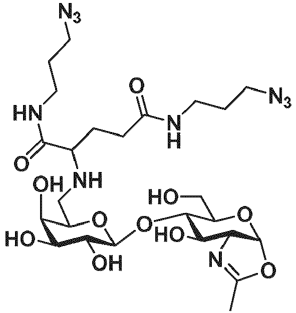

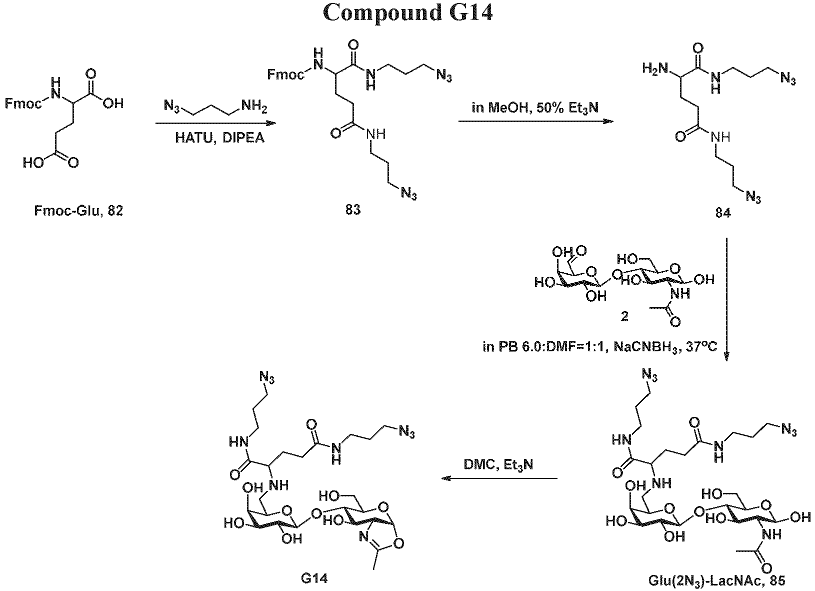

Step 1: Compound **82** (369 mg, 1 mmol) was weighed and dissolved in 2 mL of DMF. HATU (1.52 g, 4 mmol) and compound 3-azidopropylamine (500 µL. 50 mmol) and N,N-diisopropylethylamine DIPEA (1 mL, 5.9 mmol) were added in sequence, mixed well and reacted at room temperature for 1 hour. When the reaction was complete as monitored by LC-MS, the resultant was separated and purified by using a semi preparative C18 column, followed by freeze drying to obtain a white powder compound **83** (yield 92%). HRMS, calculated for C₂₆H₃₁N₉O₄ [M+H]⁺ 534.2499, found 534.6498.

Step 2: Compound **83** (54 mg, 0.1 mmol) was weighed and dissolved in 500 µL of methanol, added with 250 µL of triethylamine , and reacted at room temperature for 2 hours. After being freeze-dried, compound **84** was obtained (yield 94%). HRMS, calculated for C₁₁H₂₁N₉O₂ [M+H]⁺ 312.1818, found 312.8763.

Step 3: Compound **84** (9 mg, 0.028 mmol) was weighed and dissolved in 500 µI of DMF, compound **2** (16.8 mg, 0.044 mmol) and NaCNBH₃ (18.4 mg, 0.29 mmol) were sequentially added. After mixing, the reaction was carried out at 37 °C for 6 hours. When the reaction was complete as monitored by LC-MS, the resultant was separated and purified by using a semi preparative C18 column, followed by freeze drying to obtain a white powder compound **85** (yield 62%). HRMS, calculated for C₂₅H₄₃N₁₀O₁₁ [M+H]⁺ 659.3113, found 659.7651.

Step 4: Compound **85** (18 mg, 0.027 mmol) was weighed and dissolved in 500 µL of 50 mM PB buffer at pH 7.4. DMC (65.91 mg, 0.39 mmol) and triethylamine (18 µL. 0.13 mmol) were added thereto and reacted at 0 °C for 2 hours. When the reaction was complete as monitored by LC-MS, the resultant was separated and purified by using a semi preparative column, followed by freeze drying to obtain compound **G14** (yield 79%). HRMS, calculated for C₂₅H4₂N₁₀O₁₀: [M+H]⁺ 643.3119, found 643.3786.

### II: Preparation of small molecule drug-linkers

### Example 14: Synthesis of Compound D1

**The structure and synthesis method of compound D1 are as follows:**

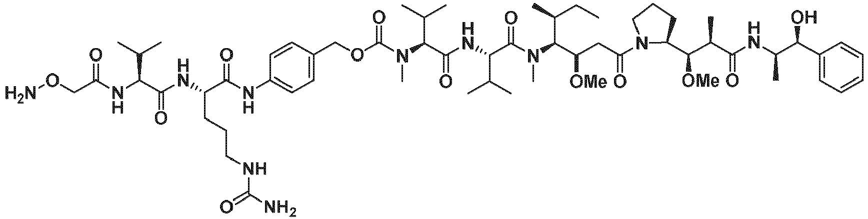

### NH₂O-VC-PAB-MMAE (Compound D1)

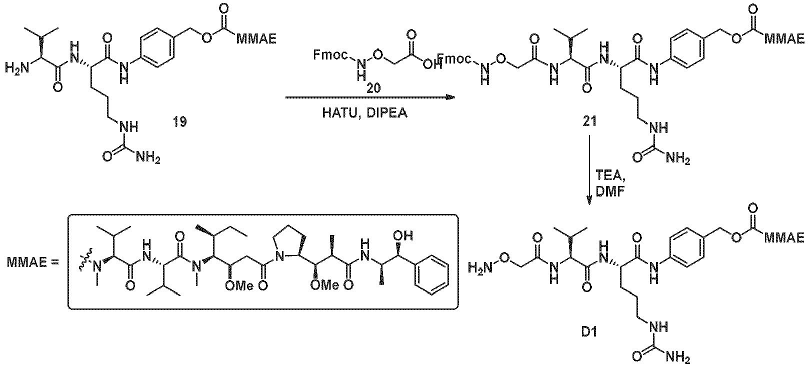

Step 1: Compound **20** (5.6 mg, 17.8 µmol) was weighed and dissolved in 100 µL of DMF. HATU (13.5 mg, 35.6 µmol), compound **19** (NH₂-VC-PAB-MMAE, 20 mg, 17.8 µmol), and N,N-diisopropylethylamine DIPEA (9.3 µL, 53.4 µmol) were added to the above system in sequence, and reacted at 37 °C for 2 hours. When the reaction was complete as monitored by LC-MS, the resultant was separated and purified by using a semi preparative C18 column, followed by freeze drying to obtain compound **21** (20 mg, yield 87%). HRMS, calculated for C₇₅H₁₀₇N₁₁O₁₆ [M+H]⁺ 1418.7975 [M+2H]²⁺709.9026, found 1418.7913, 709.9021
Step 2: Compound **21** (20 mg, 14.1 µmol) was weighed and dissolved in 100 µL of DMF. 100 µL of triethylamine was added to the above system, mixed well, and reacted at room temperature for 1 hour. When the reaction was complete as monitored by LC-MS, the resultant was separated and purified by using a semi preparative C18 column, followed by freeze drying to obtain compound **D1** (16.5 mg, yield 89%). HRMS, calculated for C₆₀H₉₇N₁₁O₁₄ [M+H]⁺ 1196.7294, [M+2H]²⁺ 598.8685, found 1196.7263, 598.8622.

### Example 15: Synthesis of Compound D2

**The structure and synthesis method of compound D2 are as follows:**

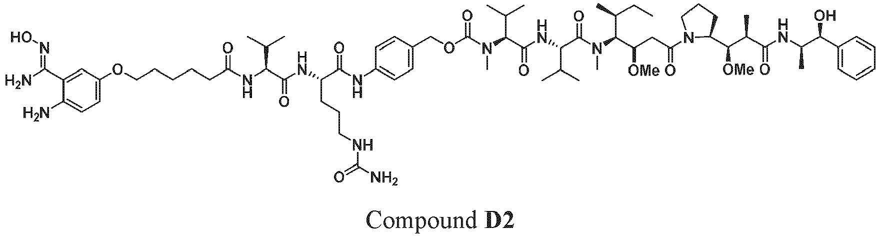

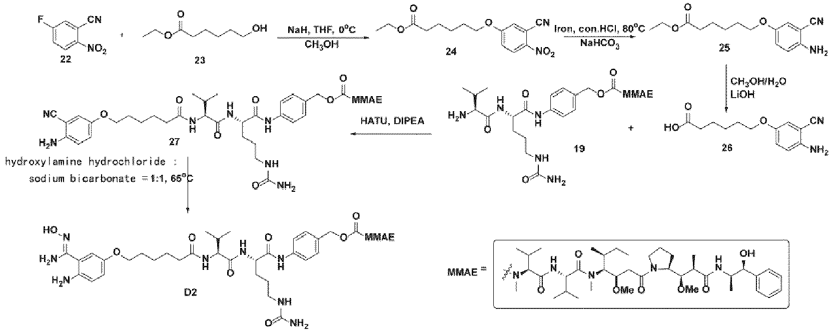

Step 1: Compound **23** (352 mg, 2.2 mmol) was weighed and dissolved in 10 mL of tetrahydrofuran. NaH (60% in oil, 88 mg, 2.2 mmol) was added to the above system at 0 °C. The reaction system was stirred at 0 °C for 15 minutes and slowly added with compound **22** (332 mg, 2 mmol). The system was stirred at room temperature for 1 hour. After quenching reaction with methanol, the reaction system was concentrated and purified on a silica gel column (petroleum ether: ethyl acetate=4:2) to obtain compound **24** (199 mg, 65%).

Step 2: Compound **24** (153 mg, 0.5 mmol) was weighed and dissolved in 10 mL of methanol. Iron (300 mg) and concentrated hydrochloric acid (0.5 ml) were added to the above system. The reaction system was diluted with 10 ml of water and stirred vigorously at 80 °C for 1 hour. After filtering and neutralizing with sodium bicarbonate, the reaction system was filtered and concentrated. Compound **25** (55 mg, 40%) was obtained by silica gel column purification (petroleum ether: ethyl acetate 2: 1).

Step 3: Compound **25** (55 mg, 0.2 mmol) was weighed and dissolved in 5 mL of methanol/water=1:1. LiOH (10 mg, 0.42 mmoL) was added to the above reaction system, which was stirred at room temperature for 4 hours. Compound **26** (44.6 mg, 90%) was obtained by concentrating and purifying on a silica gel column (petroleum ether: ethyl acetate=1:1).

Step 4: Compound **26** (5.6 mg, 0.0225 mmol) was weighed and dissolved in 100 µL of DMF. HATU (8.5 mg, 0.0225 mmol), compound **19** (8.5 mg, 0.0225 mmol), and DIPEA (7.83µL. 0.045 mmol) were added to the above reaction system in sequence, and reacted at 37 °C for 1 hour. When the reaction was complete as monitored by LC-MS, the resultant was separated and purified by using a semi preparative C18 column, followed by freeze drying to obtain compound **27** (24 mg, 80%). HRMS, calculated for C₇₁H₁₀₈N₁₂O₁₄ [M+H]⁺ 1353.8186, [M+2H]²⁺677.413, found 1353.8172, 677.4121
Step 5: Compound **27** (13.2 mg, 0.01 mmol) was weighed and dissolved in 1 mL of methanol. 5 equivalent of hydroxylamine hydrochloride and sodium bicarbonate at a ratio of 1:1(dissolved in 0.5 mL of water) were added to the above reaction system, and reacted under stirring at 65 °C for 24 hours. When the reaction was complete as monitored by LC-MS, the resultant was separated and purified by using a semi preparative C18 column, followed by freeze drying to obtain compound **D2** (13 mg, 94%). HRMS, calculated for C₇₁H₁₁₁N₁₃O₁₅ [M+H]⁺ 1386.8401, [M+2H]²⁺693.9235, found 1386.8395, 693.9264

### Example 16: Synthesis of Compound D3

**The structure and synthesis method of compound D3 are as follows:**

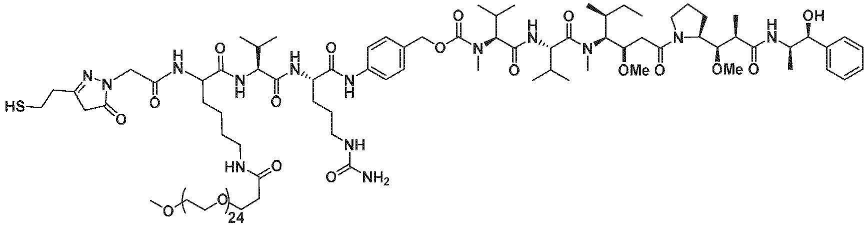

### ThioPz-Lys (PEG₂₄)-VC-PAB-MMAE (Compound D3)

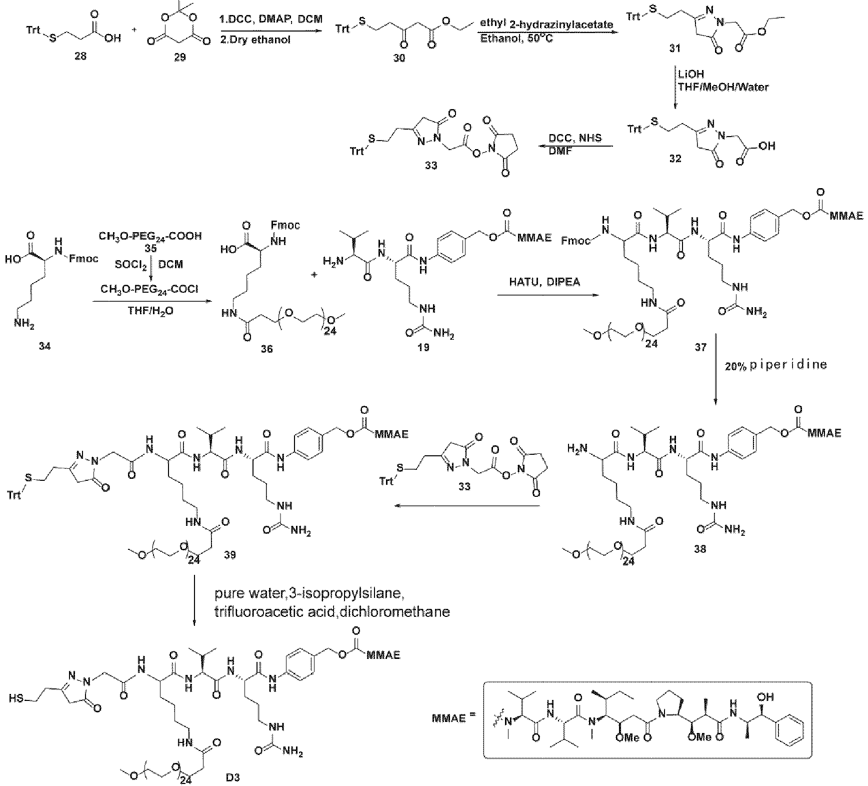

Step 1: Compound **28** (574 mg, 1.65 mmoL), compound **29** (237.4 mg, 1.65 mmoL), and DMAP (201.4 mg, 1.65 mmoL) were weighed and dissolved in 10 mL of anhydrous dichloromethane. The reaction system was cooled on ice to 0 °C. DCC (337.4 mg, 1.638 mmoL) was added to the above solution, which was stirred at 0 °C for 30 minutes, returned to room temperature, and stirred for 6 hours. The solution was diluted with dichloromethane and filtered, washed with 1N HCl and saturated brine, and the organic layer was dried over MgSO₄, and dried by rotary evaporation. The obtained oily substance was redissolved in 30 mL of anhydrous ethanol and refluxed for 4 hours. Compound **30** (560 mg, yield 81%) was obtained by silica gel column purification (hexane: ether=10:1).

Step 2: Compound **30** (499 mg, 1.24 mmoL), ethyl hydrazinylacetate hydrochloride (191 mg, 1.23 mmoL) were weighed and dissolved in 10 mL of ethanol, added with triethylamine (17.3 µL, 0.124 mmoL), and reacted at 50 °C for 2 hours. The reaction system was concentrated and purified by a silica gel column (hexane: ether=1:1) to obtain compound **31** (402 mg, yield 71%). Step 3: Compound **31** (236 mg, 0.5 mmoL) was weighed and dissolved in 10 mL of THF: MeOH: Water=2:3:1. LiOH (25 mg, 1.04 mmoL) was added to the above system and stirred for 4 hours. 40 mL of water and 40 mL of ether were poured into the above system, the water layer was adjusted to pH 2, and washed with dichloromethane. The organic layer was dried over anhydrous magnesium sulfate, concentrated, and purified by a silica gel column (dichloromethane: methanol=4:1) to obtain compound **32** (160 mg, yield 72%).

Step 4: Compound **32** (50 mg, 112.6 µmol) was weighed and dissolved in 200 µL of DMF. DCC (34.7 mg, 168.7 µmol) and NHS (19.4 mg, 168.7 µmol) were added to the above system, and reacted at 37 °C for 4 hours. When the reaction was complete as monitored by LC-MS, the resultant was separated and purified by using a semi preparative C18 column, followed by freeze drying to obtain compound **33** (55 mg, yield 90.3%).

Step 5: Compound **35** (CH₃O-PEG₂₄-COOH, 50 mg, 43 µmol) was weighed in a 25 mL round bottom flask, dissolved in 2 mL of anhydrous dichloromethane, added with two drops of dichlorosulfoxide dropwise, and refluxed at 50 °C for about 2 hours under nitrogen protection. When the reaction was complete as analyzed by thin layer chromatography using a developing agent of methanol and dichloromethane in a ratio of 1:8, the reaction system was dried by rotary evaporation, vacuum-pumped with an oil pump for 30 minutes, ensuring that the reaction system was free of dichlorosulfoxide.

Step 6: Compound **34** (Fmoc Lys OH, 16 mg, 43 µmol/L) and NaHCOs (18 mg, 215 µmol/L) were weighed in a round bottom flask, and dissolved in 900 µL tetrahydrofuran and 300 µL pure water such that it became clear.

Step 7: The system obtained by drying the solution from the first step under rotary evaporation was weighed and dissolved in 400 µL anhydrous tetrahydrofuran, the resultant was slowly added to the reaction system in step 2 in an ice bath with stirring, and reacted at room temperature for 30 minutes. When the reaction was complete as monitored by LC-MS, the resultant was separated and purified by using a semi preparative column, followed by freeze drying to obtain compound **36** (42 mg, yield 63.6%). HRMS, calculated for C₇₃H₁₂₆N₂O₃₀ [M+H]⁺ 1511.8473, [M+2H]²⁺ 756.4275, found 1511.8401, 756.4233.

Step 8: Compound **36** (10 mg, 6.62 µmol) and HATU (5 mg, 13.24 µmol) were weighed and dissolved in 100 µL of anhydrous DMF. Compound **19** (NH₂-VC-PAB-MMAE, 8.2 mg, 7.28 µmol) was added to the above reaction system, adding DIPEA (3.44 µL, 20 µmol) dropwise with stirring, and the reaction was carried out at room temperature for 1 hour. When the reaction was complete as monitored by LC-MS, the resultant was separated and purified by using a semi preparative column, followed by freeze drying to obtain compound **37** (15 mg, yield 87%). HRMS, calculated for C₁₃₁H₂₁₈N₁₂O₄₁ [M+2H]²⁺ 1308.7745, [M+3H]³⁺ 872.852, found 1308.7761, 872.8542.

Step 9: Compound **37** (15 mg, 3.8 µmol) was weighed and dissolved in 160 µL of DMF. 40 µL of piperidine was added to the above reaction system and stirred at room temperature for 20 minutes. When the reaction was complete as monitored by LC-MS, the resultant was separated and purified by using a semi preparative column, followed by freeze drying to obtain compound **38** (12.8 mg, yield 92%). HRMS, calculated for C₁₁₆H₂₀₈N₁₂O₃₉ [M+2H]²⁺ 1197.7405, [M+3H]³⁺ 798.8293, found 1197.7375, 798.8234.

Step 10: Compound **33** (2.9 mg, 5.37 µmol) was weighed and dissolved in 100 µL of DMF. Compound **38** (12.8 mg, 5.37 µmol) and triethylamine (1.5 µL, 10.74 µmol) were added to the above reaction system, and reacted at 37 °C for 2 hours. When the reaction was complete as monitored by LC-MS, the resultant was separated and purified by using a semi preparative C18 column, followed by freeze drying to obtain compound **39** (12 mg, yield 80%). HRMS, calculated for C₁₄₂H₂₃₀N₁₄O₄₁S [M+2H]²⁺ 1410.8105, [M+3H]³⁺ 940.876, found 1410.8123, 940.8771

Step 11: Compound **39** (39 mg, 4.28 µmol) was weighed and dissolved in 130 µL of dichloromethane, then the reaction system was cooled to 0 °C. 10 µL of water, 10 µL of triisopropylsilane, and 80 µL of trifluoroacetic acid were added to the above reaction system, and stirred at room temperature for 30 minutes. When the reaction was complete as monitored by LC-MS, the resultant was separated and purified by using a semi preparative C18 column, followed by freeze drying to obtain compound **D3** (6 mg, yield 54.7%). HRMS, calculated for C₁₂₃H₂₁₆N₁₄O₄₁S [M+2H]²⁺ 1289.756, [M+3H]³⁺ 860.173, found 1289.7552, 860.1741

### Example 17: Synthesis of Compound D4

**The structure and synthesis method of compound D4 are as follows:**

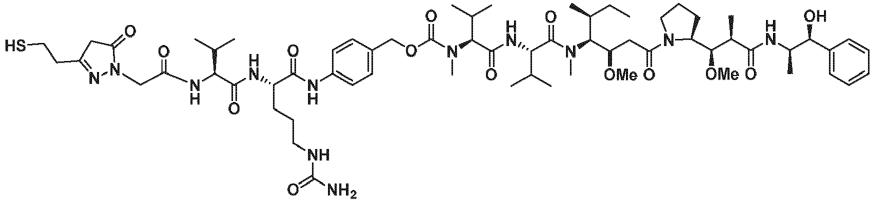

### ThioPz- VC-PAB-MMAE < Compound D4)

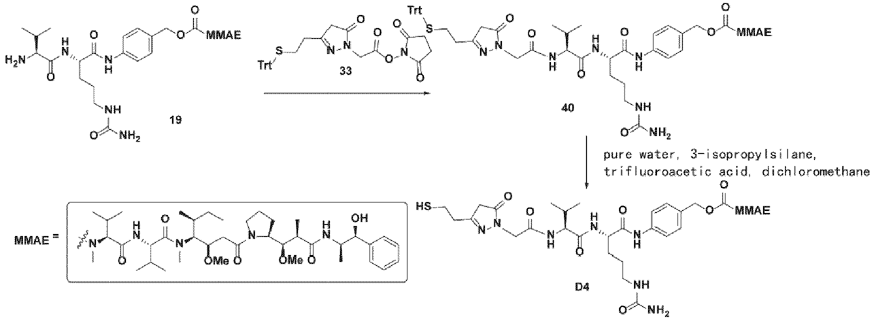

Step 1: Compound **33** (11.6 mg, 21.4 µmol) was weighed and dissolved in 100 µL of DMF. Compound **19** (20 mg, 17.8 µmol) and triethylamine (3 µL, 21.4 µmol) were added to the above reaction system, and reacted at 37 °C for 2 hours. When the reaction was complete as monitored by LC-MS, the resultant was separated and purified by using a semi preparative C18 column, followed by freeze drying to obtain compound **40** (25 mg, yield 90%). HRMS, calculated for C₈₄H₁₁₆N₁₂O₁₄S [M+2H]²⁺775.4305, found 775.4331.

Step 2: Compound **40** (20 mg, 12.9 µmol) was weighed and dissolved in 130 µL of dichloromethane, then the reaction system was cooled to 0 °C. 10 µL of water, 10 µL of triisopropylsilane, and 80 µL of trifluoroacetic acid were added to the above reaction system, and stirred at room temperature for 30 minutes. When the reaction was complete as monitored by LC-MS, the resultant was separated and purified by using a semi preparative C18 column, followed by freeze drying to obtain compound **D4** (12.8 mg, yield 76%). HRMS, calculated for C₆₅H₁₀₂N₁₂O₁₄S [M+H]⁺ 1307.7437, found 1307.7437

### Example 18: Synthesis of Compound D5

**The structure and synthesis method of compound D5 are as follows:**

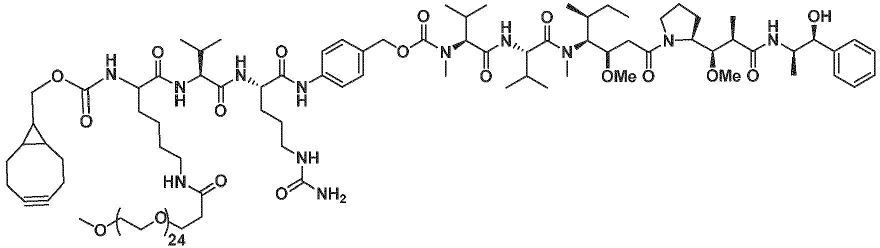

### BCN-Lys (PEG₂₄)-VC-PAB-MMAE (Compound D5)

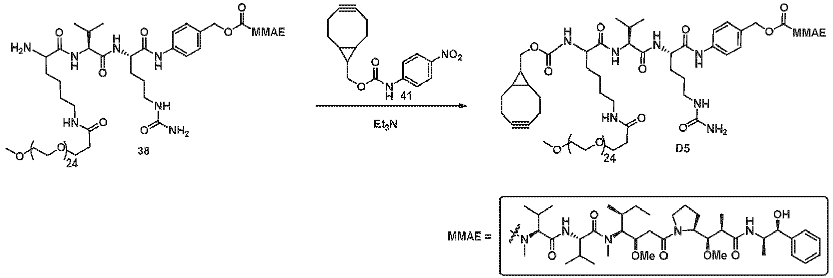

Compound **38** (12.8 mg, 5.38 µmol) was weighed and dissolved in 200 µL of DMF. Compound **41** (BCN-O-PNP, 3.38 mg, 10.76 µmol) and triethylamine (1.5 µL, 10.76 µmol) were added thereto, and let stand at 37 °C for 3 hours. When the reaction was complete as monitored by LC-MS, the resultant was separated and purified by using a semi preparative C18 column, followed by freeze drying to obtain compound **D5** (10 mg, yield 72.7%). HRMS, calculated for C₁₂₇H₂₂₀N₁₂O₄₁ [M+2H]²⁺ 1285.7825, [M+3H]³⁺ 857.524, found 1285.7848, 857.5232.

### Example 19: Synthesis of Compound D6

**The structure and synthesis method of compound D6 are as follows:**

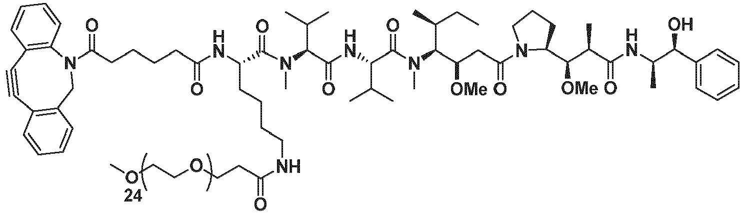

### DBCO-Lys (PEG₂₄)-MMAE (Compound D6)

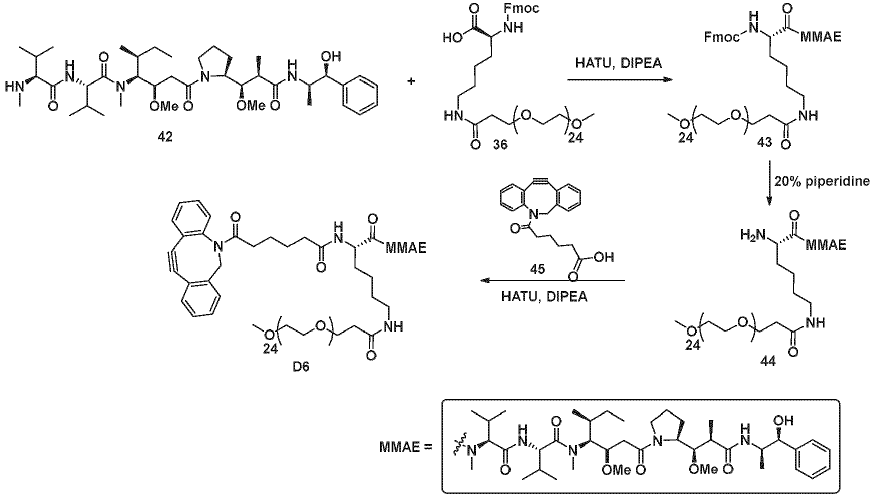

Step 1: Compound **36** (21 mg, 13.93 µmol) and HATU (10.6 mg, 27.86 µmol) were weighed and dissolved in 100 µL of anhydrous DMF. Compound **42** (MMAE, 10 mg, 13.93 µmol) and DIPEA (7.26 mL, 41.79 µmol) were added to the above reaction system, and reacted at room temperature for 1 hour. When the reaction was complete as monitored by LC-MS, the resultant was separated and purified by using a semi preparative column, followed by freeze drying to obtain compound **43** (26 mg, yield 84.7%). HRMS, calculated for C₁₁₂H₁₉₁N₇O₃₆ [M+2H]²⁺ 1106.174, [M+3H]³⁺ 737.785, found 1106.1722, 737.7832

Step 2: Compound **43** (26 mg, 11.76 µmol) was weighed and dissolved in 160 µL of DMF. 40 µL of piperidine was added to the above reaction system and stirred at room temperature for 20 minutes. When the reaction was complete as monitored by LC-MS, the resultant was separated and purified by using a semi preparative column, followed by freeze drying to obtain compound **44** (21 mg, yield 90%). HRMS, calculated for C₉₇H₁₈₁N₇O₃₄ [M+2H]²⁺ 995.14, found 995.1442 Step 3: Compound **45** (DBCO-COOH, 3.5 mg, 10.56 µmol) and HATU (8 mg, 21.12 µmol) were weighed and dissolved in 100 µL of anhydrous DMF. Compound **44** (21 mg, 10.56 µmol) and DIPEA (5.5 µL, 31.68 µmol) were added to the above reaction system, and reacted at room temperature for 1 hour. When the reaction was complete as monitored by LC-MS, the resultant was separated and purified by using a semi preparative column, followed by freeze drying to obtain compound **D6** (20.2 mg, yield 84%). HRMS, calculated for C₁₁₈H₁₉₈N₈O₃₆ [M+2H]²⁺ 1152.7033, [M+3H]³⁺ 768.8048, found 1152.7022, 768.8031.

### Example 20: Synthesis of Compound D7

**The structure and synthesis method of compound D7 are as follows:**

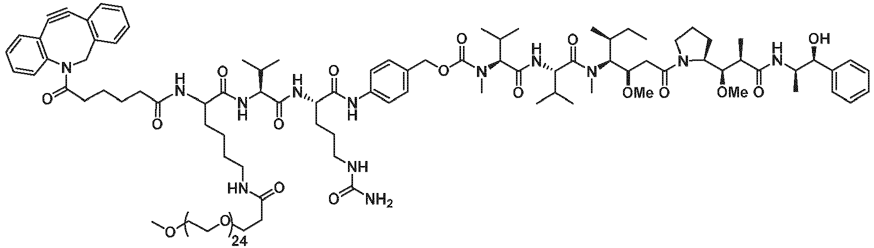

### DBCO-Lys (PEG₂₄)-VC-PAB-MMAE (Compound D7)

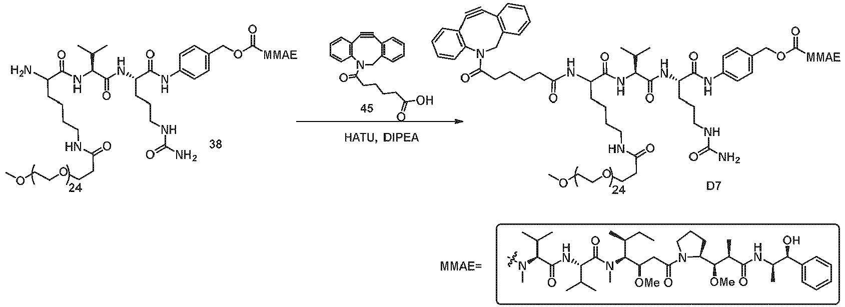

Compound **45** (DBCO-COOH, 1.8 mg, 5.38 µmol) and HATU (4.1 mg, 10.76 µmol) were weighed and dissolved in 100 µL of anhydrous DMF. Compound **38** (12.8 mg, 5.38 µmol) and DIPEA (2.8 µL, 16.14 µmol) were added to the above reaction system, and reacted at room temperature for 1 hour. When the reaction was complete as monitored by LC-MS, the resultant was separated and purified by using a semi preparative column, followed by freeze drying to obtain compound **D7** (12 mg, 84%). HRMS, calculated for C₁₃₇H₂₂₅N1₃O₄₁ [M+2H]²⁺ 1355.3039, [M+3H]³⁺ 903.8718, found 1355.302, 903.8707.

### Example 21: Synthesis of Compound D8

**The structure and synthetic method of compound D8 are as follows:**

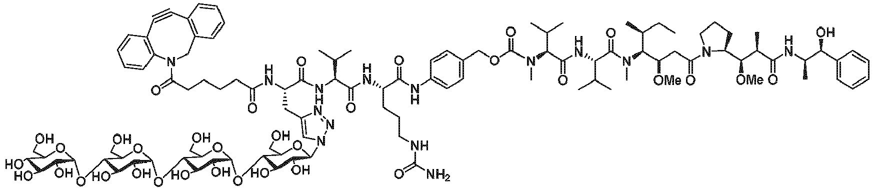

### DBCO-Gly (maltotetraose)-VC-PAB-MMAE (Compound D8)

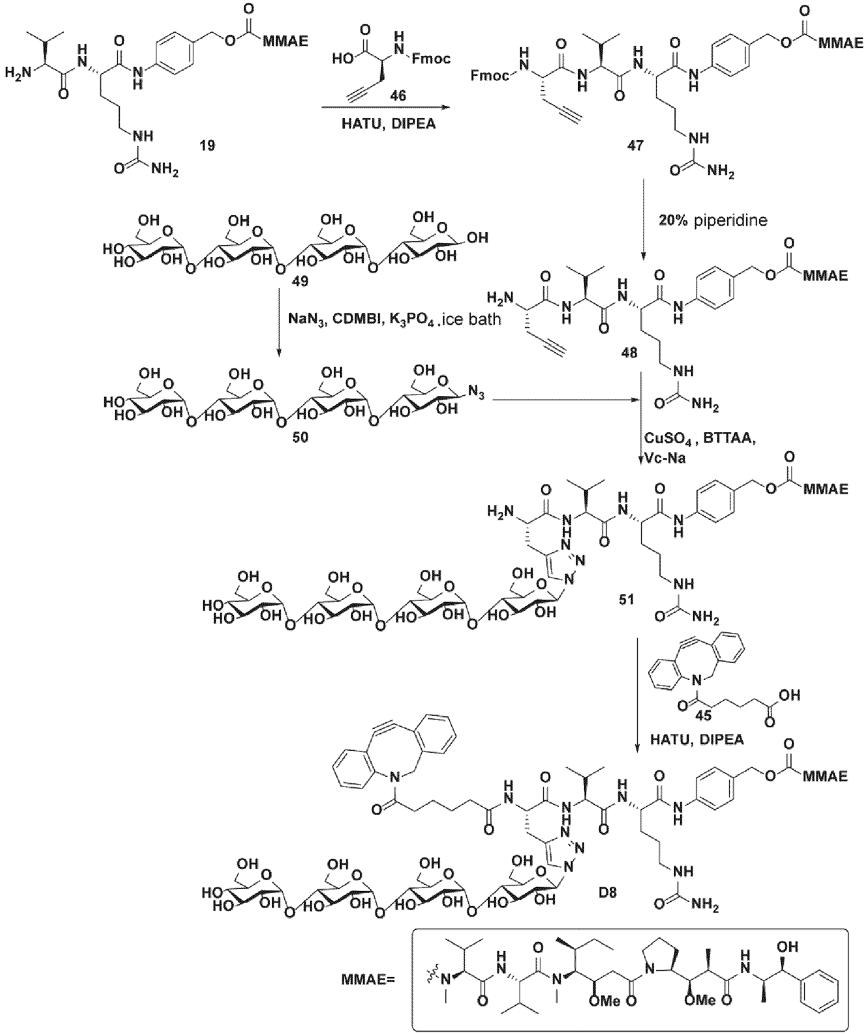

Step 1: Compound **49** (maltotetraose, 20 mg, 30 µmol) was weighed and dissolved in 600 µL of ddH₂O, and sodium azide (96 mg, 1.5 mmoL) and CDMBI (32.4 mg, 150 µmol) were added to the above reaction system, which was then placed on ice and cooled to 0 °C. Potassium phosphate (96 mg, 450 µmol) was then added and reacted at 0 °C for 4 h to generate compound **50**. The reaction system was not processed, and stored at -80 °C after aliquoting for use.

Step 2: Compound **46** (6 mg, 17.9 µmol) was weighed and dissolved in 100 µL of DMF. HATU (13.6 mg, 35.8 µmoL), compound **19** (NH₂-VC-PAB-MMAE, 20 mg, 17.9 µmol) and DIPEA (0.88 mL, 53.7 µmol) were added in sequence to the above reaction system and reacted at 37 °C for 2 h. When the reaction was complete as monitored by LC-MS, the resultant was separated and purified by a semi preparative C18 column, followed by freeze drying to obtain Compound **47** (21 mg, yield 82%). HRMS calculated for C₇₈H₁₀₉N₁₁O₁₅ [M+2H]²⁺ 720.913, found 720.9121.

Step 3: Compound **47** (21 mg, 14.58 µmol) was weighed and dissolved in 80 µL of DMF, 20 µL of piperidine was added to the above reaction system, and reacted at room temperature for 20 min. When the reaction was complete as monitored by LC-MS, the resultant was separated and purified by a semi preparative C18 column to obtain Compound **48** (16 mg, yield 90%). HRMS calculated for C₆₃H₉₉N₁₁O₁₃ [M+H]⁺ 1218.7502, [M+2H]²⁺ 609.879, found 1218.7552, 609.8776.

Step 4: Preparation of Cu (I)-BTTAA solution: 32.5 µL of 60 mM CuSO₄, 39 µL of 300 mM BTTAA and 286 µL of 0.9M aodium ascorbate were mixed in turn for use.

Step 5: Compound **48** (16 mg, 13.1 µmoL) was added to the reaction system in step 1 (calculated by 100% yield, compound **50** (17.9 mg, 25.9 µmoL)), mixed well and then the whole volume of Cu(I)-BTTAA solution in step 4 was added thereto and reacted at 37 °C for 4 h. When the reaction was complete as monitored by LC-MS, the resultant was separated and purified by a semi preparative C18 column, followed by freeze drying to obtain compound **51** (20 mg, yield 80%). HRMS calculated for C₈₇H₁₄₀N₁₄O₃₃ [M+2H]²⁺ 955.493, found 955.4887.

Step 6: Compound **45** (DBCO-COOH, 3.5 mg, 10.47 µmoL) was weighed and dissolved in 100 µL of DMF. HATU (8 mg, 20.94 µmoL), compound **51** (20 mg, 10.47µmol) and DIPEA (5.46 mL, 31.41 µmol) were added to the above reaction system in sequence and reacted at 37 °C for 2 h. When the reaction was complete as monitored by LC-MS, the resultant was separated and purified by a semi preparative C18 column, followed by freeze drying to obtain compound **D8** (21 mg, yield 91%). HRMS calculated for C₁₀₈H₁₅₇N₁₅O₃₅ [M+2H]²⁺ 1113.0562, [M+3H]³⁺ 742.3734, found 1113.0505, 742.3704.

### Example 22: Synthesis of Compound D9

**The structure and synthetic method of compound D9 are as follows:**

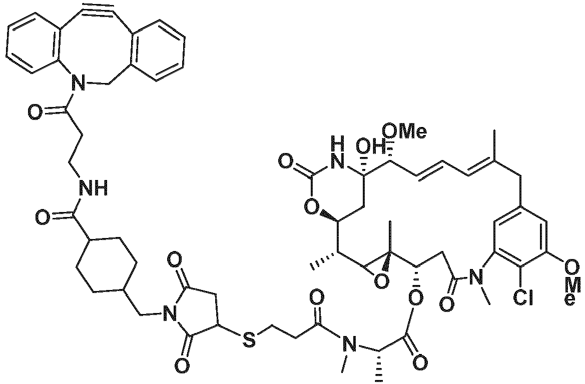

### DBCO-SMCC-DM1 (Compound D9)

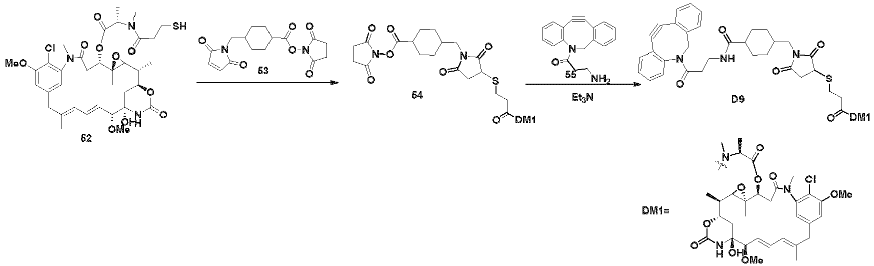

Step 1: Compound **52** (SH-DM1, 20 mg, 27.13 µmol) was weighed and dissolved in 100 µL of DMF, compound **53** (SMCC, 9 mg, 27.13 µmol) was added to the above reaction system, and reacted at 37 °C for 2 hours. When the reaction was complete as monitored by LC-MS, the resultant was separated and purified by a semi preparative C18 column, followed by freeze drying to obtain compound **54** (24 mg, yield 82.7%). HRMS calculated for C₅₁H₆₆ClN₅O₁₆S [M+H]⁺ 1072.3992, found 1072.3966.

Step 2: Compound **54** (SMCC-DM1, 24 mg, 22.4 µmoL) was weighed and dissolved in 100 µL of DMF, compound **55** (DBCO-NH₂, 6.2 mg, 22.4 µmol) and triethylamine (6.3 µL, 44.8 µmol) were added to the above reaction system and reacted at 37 °C for 1 h. When the reaction was complete as monitored by LC-MS, the resultant was separated and purified by a semi preparative C18 column, followed by freeze drying to obtain compound **D9** (20 mg, yield 72.6%). HRMS calculated for C₆₅H₇₇ClN₆O₁₄S [M+H]⁺ 1233.4985, [M+2H]²⁺ 617.253, found 1233.4923, 617.2555.

### Example 23: Synthesis of Compound D10

**The structure and synthetic method of compound D10 are as follows:**

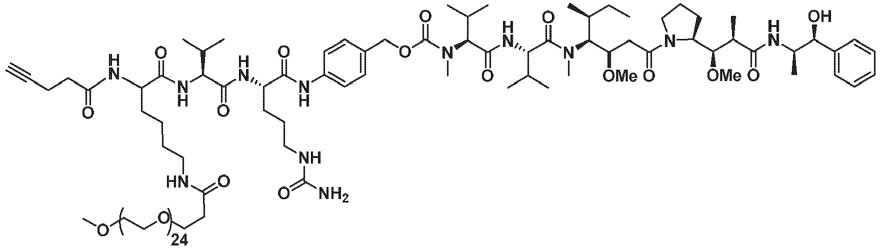

### straight chain alkynyl-Lys (PEG₂₄)-VC-PAB-MMAE (Compound D10)

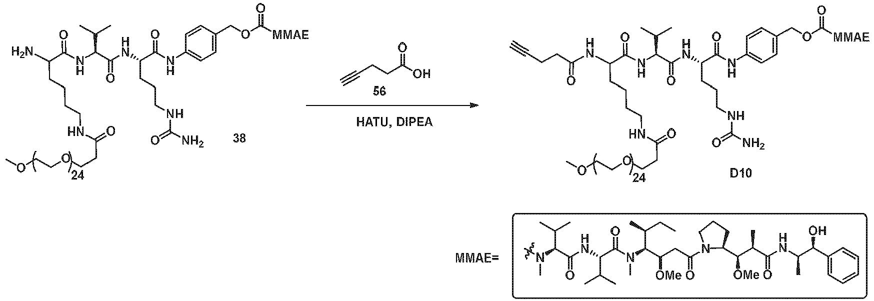

Compound **56** (0.6 mg, 6.12 mol) was weighed and dissolved in 100 µL of DMF. HATU (4.1 mg, 10.7 µmol) was added to the above reaction system and mixed well, and then compound **38** (12.8 mg, 5.35 µmol) and DIPEA (2.8 µL, 16.05 µmol) were added in sequence and reacted at 37 °C for 2 h. When the reaction was complete as monitored by LC-MS, the resultant was separated and purified by a semi preparative C18 column, followed by freeze drying to obtain compound **D10** (10 mg, yield 75.6%). HRMS calculated for C₁₂₁H₂₁2N₁₂O₄₀ [M+2H]²⁺ 1237.7535, [M+3H]3+ 825.505, found 1237.7532, 825.5060.

### Example 24: Synthesis of Compound D11

**The structure and synthetic method of compound D11 are as follows:**

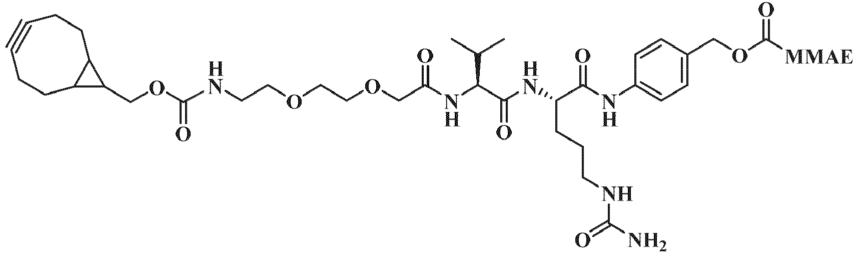

### BCN-PEG₂-CH₂- VC-PAB-MMAE < Compound D11)

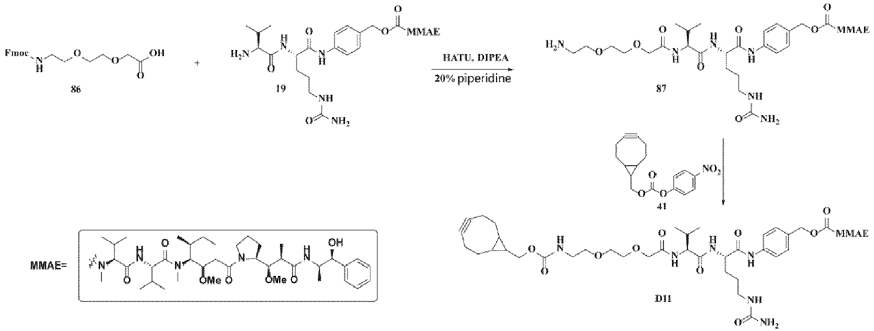

Step 1: Compound **86** (8.24 mg, 0.0356 mmol) was weighed and dissolved in 82.4 µL of DMF. HATU (13.6 mg, 0.0356 mmol), compound **19** (20 mg, 0.0178 mmol) and DIPEA (9.34 µL, 0.0536 mmol) were added to the above reaction system in sequence and reacted at 37 °C for 2 h. When the reaction was almost complete as monitored by LC-MS, 107 µL of triethylamine was added, mixed well and reacted at room temperature for 15 min. The resultant was separated and purified by a semi preparative C18 column, and the target product was collected and freeze dried to obtain compound **87** (20.8 mg, yield 92%). HRMS, calculated for C₆₄H₁₀₅N₁₁O₁₅ [M+H]⁺ 1268.787, found 1268.7815.

Step 2: Compound **87** (20.8 mg, 0.0164 mmol) was weighed and dissolved in 208 µL of DMF, and compound **41** (10.35 mg, 0.0328 mmol) and triethylamine (9.13 µL, 0.0657 mmol) were added thereto and reacted at 37 °C for 2 h. When the reaction was almost complete as monitored by LC-MS, the resultant was separated and purified by a semi preparative C18 column, and the target product was collected and freeze dried to obtain compound **D11** (14.7 mg, yield 62%). HRMS, calculated for C₇₅H₁₁₇N₁₁O₁₇ [M+H]⁺ 1444.8707, found 1444.8662.

### Example 25: Synthesis of Compound D12

**The structure and synthetic method of compound D12 are as follows:**

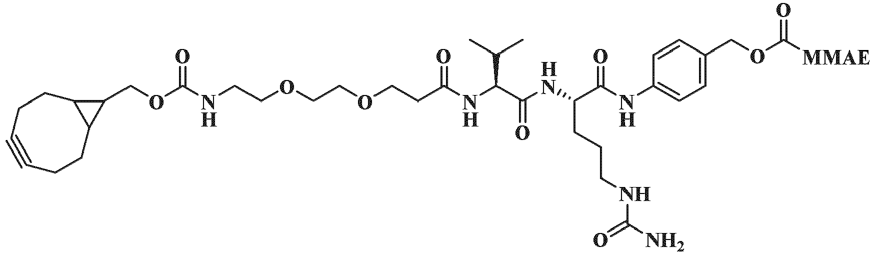

BCN-PEG₂-CH₂-CH₂-VC-PAB-MMAE (Compound **D12**)

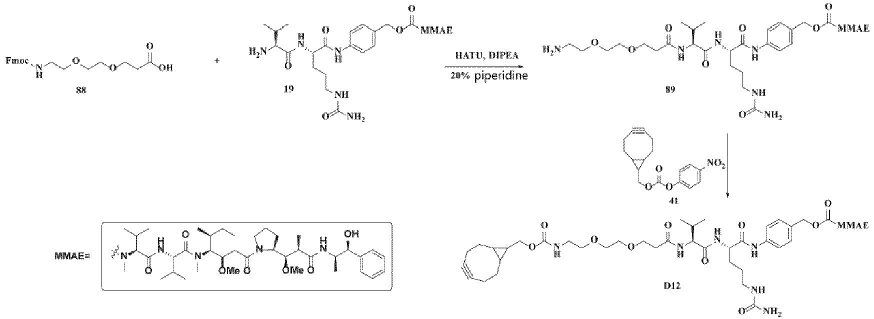

Step 1: Compound **88** (10.68 mg, 0.0267 mmol) was weighed and dissolved in 106.8 µL of DMF, HATU (20.34 mg, 0.0534 mmol), compound **19** (30 mg, 0.0267 mmol) and DIPEA (9.34 µL, 0.0802 mmol) were added to the above reaction system in sequence and reacted at 37 °C for 2 h. When the reaction was almost complete as monitored by LC-MS, 156 µL of triethylamine was added, mixed well and reacted at room temperature for 15 min. The resultant was separated and purified by a semi preparative C18 column, and the target product was collected and freeze dried to obtain compound **89** (31.1 mg, yield 90.7%). HRMS, calculated for C₆₅H₁₀₇N₁₁O₁₅ [M+H]⁺ 1282.8026, found 1282.8041.

Step 2: The above product compound **89** (31.1 mg, 0.0234 mmol) was weighed and dissolved in 208 µL of DMF, and compound **41** (10.35 mg, 0.0351 mmol) and triethylamine (9.13 µL, 0.0936 mmol) were added and reacted at 37 °C for 2 h. When the reaction was almost complete as monitored by LC-MS, the resultant was separated and purified by a semi preparative C18 column, and the target product was collected and freeze dried to obtain compound **D12** (28.5 mg, yield 83.5%). HRMS, calculated for C₇₆H₁₁₉N₁₁O₁₇ [M+H]⁺ 1458.8864, found 1458.8792.

### Example 26: Synthesis of Compound D13

**The structure and synthetic method of compound D13 are as follows:**

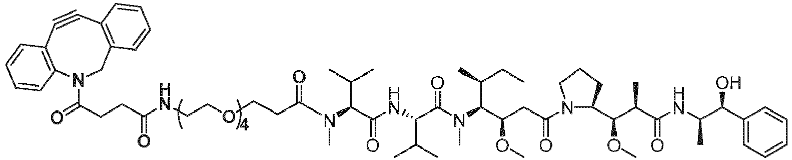

### DBCO-PEG₄- MMAE (Compound D13)

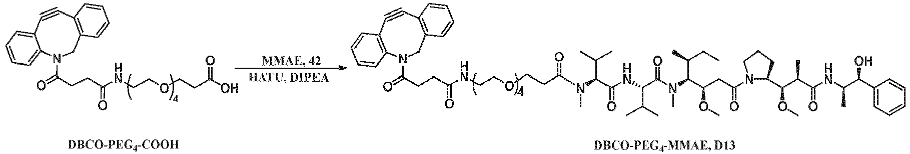

Compound DBCO-PEG₄-COOH (9.8 mg, 17.8 µmoL) was weighed and dissolved in 100 µL of DMF, and HATU (13.5 mg, 35.6 µmoL), MMAE (12.8 mg, 17.8 µmoL) and DIPEA (18.6 µL, 106.8 µmoL) were added to the above system and reacted at 37 °C for 2 h. When the reaction was complete as monitored by LC-MS, the resultant was separated and purified by a semi preparative C18 column, and the target product was collected and freeze dried to obtain compound **D13** (15.8 mg, yield 71%). HRMS, calculated for C₆₉H₁₀₁N₇O₁₄ [M+H]⁺ 1252.7485, found 1252.7479.

### III: Synthesis of disaccharide-small molecule drug conjugates DG-1 to DG5 and dDG-1

### Example 27: Synthesis of Compound DG-1

**The structure and synthesis method of compound DG-1 are as follows:**

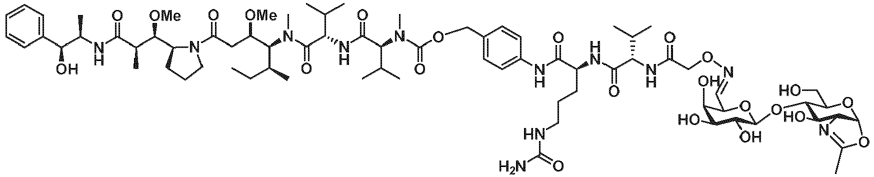

### MMAE-PAB-VC-ON=CH-LacNAc-ox <Compound DG-1)

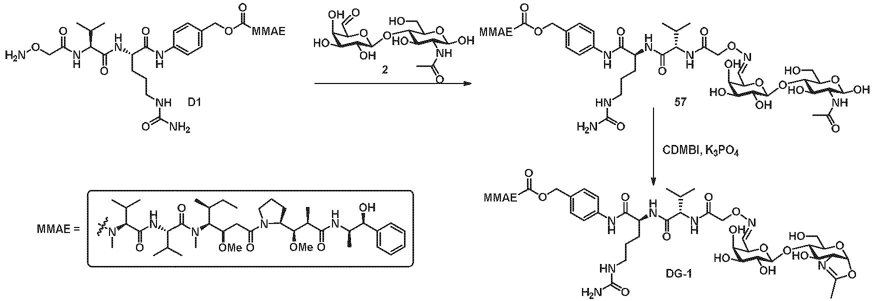

Step 1: Compound **D1** (10 mg, 8.5 µmoL) was weighed and dissolved in 100 µL of DMF, and compound **2** (3.3 mg, 8.5 µmoL) was weighed and dissolved in 100 µL of 0.2 M PB, pH 7.5 buffer. The above two systems were mixed and reacted at 37 °C for 2 h. When the reaction was complete as monitored by LC-MS, the resultant was separated and purified by a semi preparative C18 column, followed by freeze drying to obtain compound **57** (10 mg, yield 74%). HRMS calculated for C₇₄H₁₁₈N₁₂O₂₄ [M+2H]²⁺ 780.4265, found 780.4221.

Step 2: Compound **57** (10 mg, 6.4 µmol) was weighed and dissolved in 100 mL of DMF/50 mM PB, pH 7.5=1:1. CDMBI (6.9 mg, 32 moL) was added to the above system, mixed well and cooled to 0 °C on ice. Potassium phosphate (20.4 mg, 96 µmol) was added and reacted at 0 °C for 12 h. A cyclized product was generated as monitored LC-MS, which was separated and purified by an alkaline semi-preparative C18 column to obtain compound **DG-1** (7.2 mg, yield 72%). HRMS calculated for C₇₄H₁₁₆N₁₂O₂₃ [M+2H]²⁺771.4215, found 771.4221.

### Example 28: Synthesis of Compound DG-2

**The structure and synthesis method of compound DG-2 are as follows:**

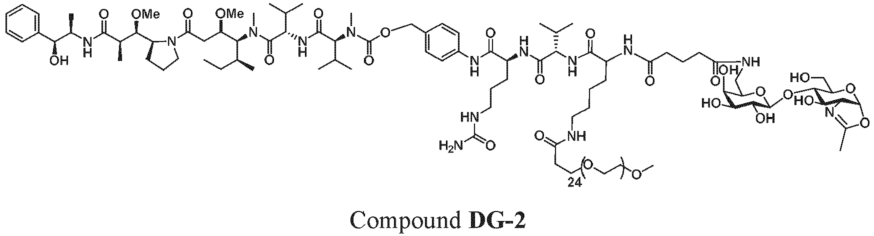

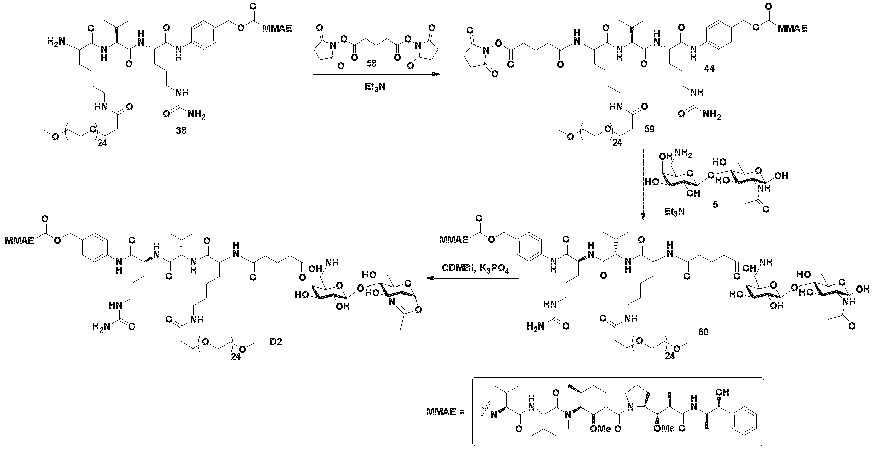

Step 1: Compound **38** (20 mg, 8.36 µmol) was weighed and dissolved in 100 µL of DMF, compound **58** (13.6 mg, 41.8 µmol) and triethylamine (3.5 µL, 25.1 µmol) were added to the above system, and reacted at 37 °C for 2 h. When the reaction was complete as monitored by LC-MS, the resultant was separated and purified by a semi preparative C18 column, followed by freeze drying to obtain compound **59** (18 mg, yield 83%). HRMS calculated for C₁₂₅H₂₁₇N₁₃O₄₄ [M+2H]²⁺ 1303.2645, [M+3H]³⁺ 869.1792, Found 1303.2667, 869.1799.

Step 2: Compound **59** (18 mg, 6.9 µmoL) was weighed and dissolved in 100 µL of DMF, compound **5** (2.64 mg, 6.9 µmoL) and triethylamine (2.9 µL, 20.8 µmoL) were added to the above reaction system, and reacted at room temperature for 3 h. Compound **5** (2.64 mg, 6.9 µmol) was further added, and the reaction was continued at room temperature for 3 h. When the reaction was complete as monitored by LC-MS, the resultant was separated and purified by a semi preparative C18 column, followed by freeze drying to obtain compound **60** (14.2 mg, yield 71%). HRMS calculated for C₁₃₅H₂₃₈N₁₄O₅₁ [M+3H]³⁺ 958.2231, [M+4H]⁴⁺ 718.9193 Found 958.2233, 718.9192.

Step 3: Compound **60** (14.2 mg, 4.95 µmoL) was weighed and dissolved in 100 µL of 50 mM PB, pH 7.5. CDMBI (5.4 mg, 24.8 µmoL) was added to the above reaction system, mixed well. After standing, it was cooled to 0 °C on ice, and then potassium phosphate (15.8 mg, 74.25 µmol) was added thereto and reacted at 0 °C for 12 h. When the reaction was almost complete as monitored by LC-MS, the resultant was separated and purified by an alkaline semi preparative C18 column, followed by freeze drying to obtain compound **DG-2** (10 mg, yield 70.5%). HRMS calculated for C₁₃₅H₂₃₆N₁₄O₅₀ [M+3H]³⁺ 952.2196, found 952.2112.

### Example 29: Synthesis of Compound DG-3

**The structure and synthesis method of compound DG-3 are as follows:**

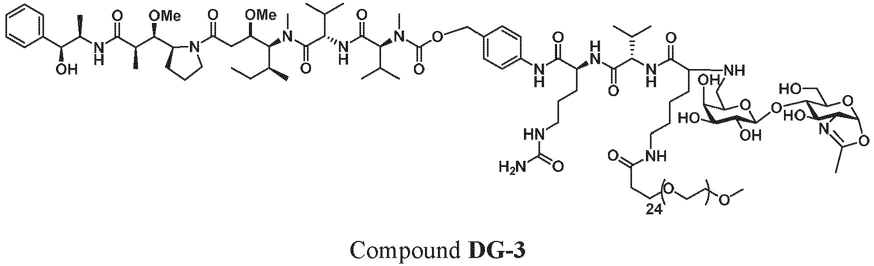

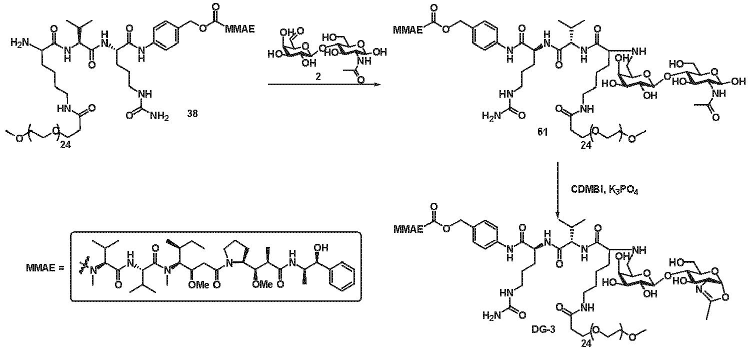

Step 1: Compound **38** (20 mg, 8.36 µmoL) was weighed and dissolved in 100 µL of DMF/0.2M PB, pH 6.0=1:1, compound **2** (9.6 mg, 25.1 µmoL) was added to the above reaction system, which was adjusted to pH 6.0 with NaOH/HCl, then sodium cyanoborohydride (5.3 mg, 83.6 µmoL) was added thereto and reacted at 37 °C for 3 h. When the reaction was almost complete as monitored by LC-MS, the resultant was separated and purified by a semi preparative C18 column, followed by freeze drying to obtain compound **61** (18 mg, yield 78.3%). HRMS calculated for C₁₃₀H₂₃₁N₁₃O₄₉ [M+3H]³⁺ 920.5406, found 920.5353.

Step 2: Compound **61** (18 mg, 6.53 µmoL) was weighed and dissolved in 100 µL of 50 mM PB, pH 7.5=1: 1, CDMBI (7 mg, 32.63 µmoL) was added to the above reaction system, mixed well, and then cooled to 0 °C on ice. Potassium phosphate (20.8 mg, 98 mol) was added and reacted at 0 °C for 12 h. When the reaction was almost complete as monitored by LC-MS, the resultant was separated and purified by an alkaline C18 column, followed by freeze drying to obtain compound **DG-3** (11 mg, yield 61.5%). HRMS calculated for C₁₃₀H₂₂₉N₁₃O₄₈ [M+3H]³⁺ 914.537, found 914.5310.

### Example 30: Synthesis of Compound DG-4

**The structure and synthesis method of compound DG-4 are as follows:**

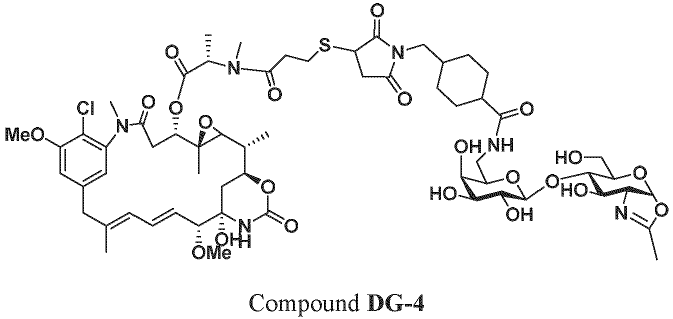

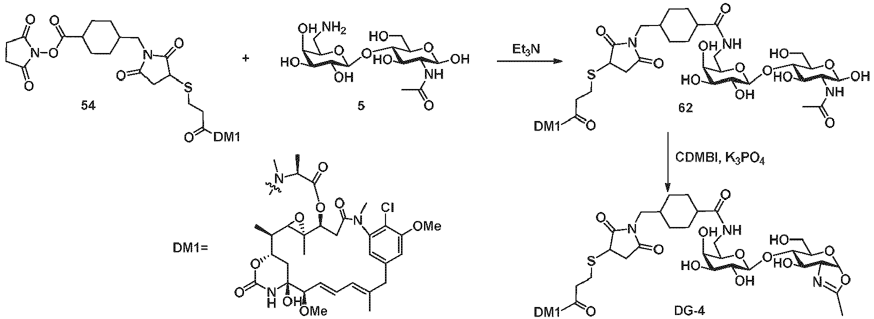

Step 1: Compound **54** (10 mg, 9.3 µmoL) was weighed and dissolved in 100 µL of DMF, compound **5** (3.6 mg, 9.3 µmoL) and triethylamine (3.8 µL, 27.9 µmol) were added to the above system, and reacted at 37 °C for 2 h. After compound **5** (3.6 mg, 9.3 µmol) was added, the reaction was continued at 37 °C for 2 h. When the reaction was almost complete as monitored by LC-MS, the resultant was separated and purified by a semi preparative C18 column, followed by freeze drying to obtain compound **62** (10 mg, 80%). HRMS calculated for C₆₁H₈₇ClN₆O₂₃S [M+2H]²⁺670.2694, found 670.2669.

Step 2: Compound **62** (10 mg, 7.47 µmoL) was weighed and dissolved in 100 µL of DMF/50 mM PB, pH 7.5, CDMBI (8 mg, 37.35 µmoL) was added to the above reaction system, mixed well and then the reaction system was cooled to 0 °C on ice, and potassium phosphate (23.8 mg, 112 µmol) was added and reacted at 0 °C for 12 h. When the reaction was almost complete as monitored by LC-MS, the resultant was separated and purified by an alkaline C18 column, followed by freeze drying to obtain compound **DG-4** (7 mg, yield 71%). HRMS calculated for C₆₁H₈₅ClN₆O₂₂S [M+2H]²⁺ 661.2641, found 661.2660.

### Example 31: Synthesis of Compound DG-5

**The structure and synthesis method of compound DG-5 are as follows:**

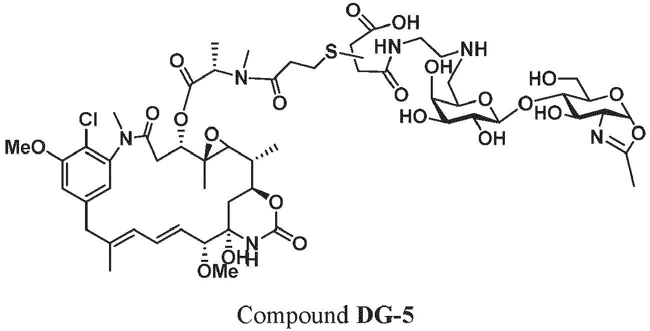

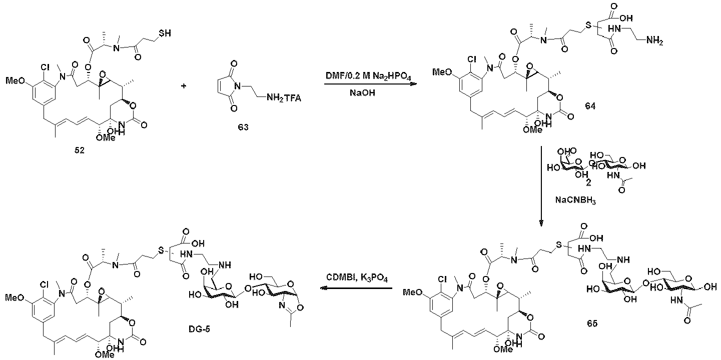

Step 1: Compound **52** (20 mg, 27.12 µmol) was weighed and dissolved in 200 µL of DMF, compound **63** (6.5 mg, 27.12 µmol) and 100 µL of 0.2 M Na₂HPO₄ were added to the above system, and reacted at room temperature for 2 h. After the reaction was almost complete as monitored by LC-MS, 200 µL of 1% NaOH was added to the above system, the reaction solution changed from light yellow to light red. The reaction was complete after 1 h. The resultant was separated and purified by a semi preparative C18 column, followed by freeze drying to obtain compound **64.** (19 mg, 78%). HRMS calculated for C₄₁H₅₈ClN₅O₁₃S [M+H]⁺ 896.3518, found 896.3533.

Step 2: Compound **64** (19 mg, 21.2 µmoL) was weighed and dissolved in 400 µL of DMF/0.2M PB, pH 6.0=1:1, compound **2** (32.3 mg, 84.8 µmol) was added to the above reaction system, which was adjusted to pH 6.0 with NaOH/HCl, then sodium cyanoborohydride (10.7 mg, 169.6 µmol) was added, and the reaction was carried out at 37 °C for 3 h. The reaction was almost complete as monitored by LC-MS. The resultant was separated and purified by a semi preparative C18 column, followed by freeze drying to obtain compound **65** (20 mg, yield 75%). HRMS calculated for C₅₅H₈₁ClN₆O₂₃S [M+2H]²⁺ 631.2459, found 631.2424.

Step 3: Compound **65** (20 mg, 15.86 µmoL) was weighed and dissolved in 500 µL of 50 mM PB, pH 7.5, CDMBI (17.2 mg, 79.3 µmoL) was added to the above reaction system, mixed well and then the reaction system was cooled to 0 °C on ice, and then potassium phosphate (50.5 mg, 237.9 µmol) was added thereto and reacted at 0 °C for 12 h. When the reaction was almost complete as monitored by LC-MS, the resultant was separated and purified by an alkaline C18 column, followed by freeze drying to obtain compound **DG-5** (12.6 mg, yield 64%). HRMS calculated for C₅₅H₇₉ClN₆O₂₂S [M+2H]²⁺ 622.2406, found 622.2409.

### Example 32: Synthesis of Compound DG-6

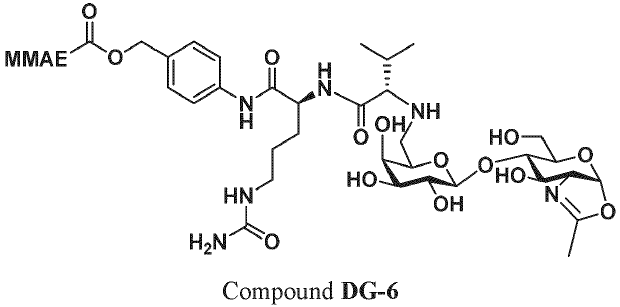

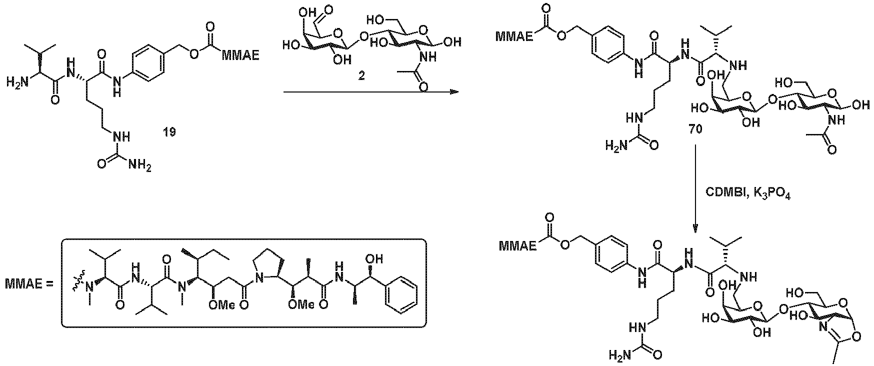

Step 1: Compound **19** (10 mg, 8.9 µmoL) was weighed and dissolved in 100 µL of DMF, compound **2** (13.8 mg, 35.6 µmoL) was weighed and dissolved in 100 µL of 0.2 M PB, pH 6.0 buffer, After the pH of the reaction system was 6.0 as detected, NaCNBH₃ (5.3 mg, 89 µmol) was added, and the reaction system was mixed well, and reacted at 37 °C for 2 h. When most product was generated as monitored by LC-MS, the resultant was separated and purified by a semi preparative C18 column, followed by freeze drying to obtain compound **70** (10 mg, yield 75.4%). HRMS calculated for C₇₂H₁₁₇N₁₁O₂₂ [M+2H]²⁺ 744.9187, found 744.9110.

Step 2: Compound **70** (10 mg, 6.7 µmoL) was weighed and dissolved in 100 µL of DMF/50 mM PB, pH 7.5=1:1, CDMBI (7.2 mg, 33.5 µmol) was added to the above system, mixed well and cooled to 0 °C on ice, and then potassium phosphate (21.4 mg, 100.5 µmol) was added thereto and reacted at 0 °C for 12 h. As monitored by LC-MS, the reaction was almost complete to generate a cyclized product, which was separated and purified by an alkaline semi preparative C18 column, followed by freeze drying to obtain compound **DG-6** (7.2 mg, yield 73.4%). HRMS calculated for C₇₂H₁₁₅N₁₁O₂₁ [M+2H]²⁺ 735.9134, found 735.9133.

### Example 33: Synthesis of Compound DG-7

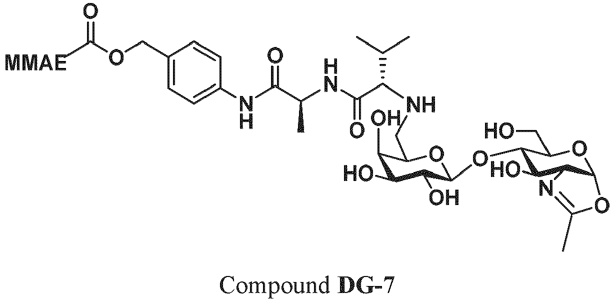

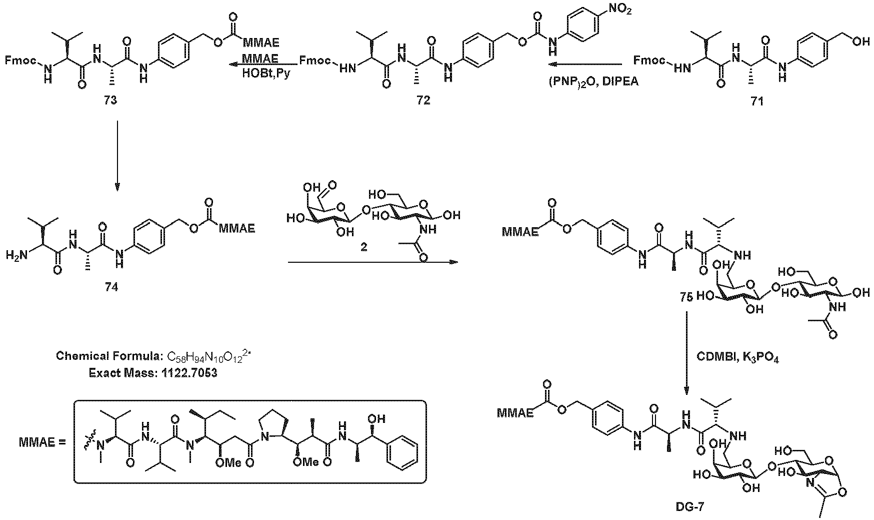

Step 1: Compound **71** (Fmoc-VA-PAB-OH, 20 mg, 38.8 µmoL) was weighed and dissolved in 400 µL of DMF, and compound (PNP)₂O (23.6 mg, 77.6 µmoL) was weighed and dissolved in the above mixture system, and 3.2 µL of DIPEA was added thereto, mixed well and reacted overnight at room temperature. As monitored by LC-MS, most product was generated, which was separated and purified by a semi preparative C18 column, followed by freeze drying to obtain compound **72** (23 mg, yield 87%). HRMS calculated for C₃₇H₃₇N₅O₈ [M+H]⁺ 680.272, found 680.2712.

Step 2: Compound **72** (23 mg, 33.8 µmoL) was weighed and dissolved in 400 µL of DMF, MMAE (24.3 mg, 33.8 µmoL) was added to the above system, mixed well and HOBt (0.92 mg, 6.76 µmoL) and 82 µL of pyridine were added thereto and reacted at room temperature for 12 h. As monitored by LC-MS, reaction was almost complete to generate a cyclized product, which was separated and purified by an alkaline semi-preparative C18 column to obtain compound **73** (35.3 mg, yield 83%). HRMS calculated for C₇₀H₉₈N₈O₁₃ [M+2H]²⁺ 630.3705, found 630.3701. Step 3: Compound 73 (30 mg, 23.8 µmol) was weighed and dissolved in 100 µL of DMF, 20 µL of piperidine was added to the above reaction system, and reacted at room temperature for 20 min. When the reaction was complete as monitored by LC-MS, the resultant was separated and purified by a semi preparative C18 column, followed by freeze drying to obtain Compound **74** (22.9 mg, yield 93%). HRMS calculated for C₅₅H₈₈N₈O₁₁ [M+H]⁺ 1037.6651, found 1037.6559.

Step 4: Compound **74** (22.9 mg, 22.1 µmoL) was weighed and dissolved in 100 µL of DMF, and compound **2** (34.2 mg, 88.4 µmoL) was weighed and dissolved in 100 µL of 0.2 M PB, pH 6.0 buffer, then adding to the above system. After the pH of the reaction system was 6.0 as detected, NaCNBH₃ (131.6 mg, 221 µmol) was added thereto. The reaction system was mixed well and reacted at 37 °C for 6 h. As monitored by LC-MS, most product was generated, which was separated and purified by a semi preparative C18 column, followed by freeze drying to obtain Compound **75** (23.5 mg, yield 76%). HRMS calculated for C₆₉H₁₁₁N₉O₂₁ [M+2H]²⁺ 701.9025, found 701.9022.

Step 5: Compound **75** (10 mg, 6.7 µmoL) was weighed and dissolved in 100 µL of DMF/50 mM PB, pH 7.5=1:1, CDMBI (7.6 mg, 35.5 µmol) was added to the above system, mixed well and cooled to 0 °C on ice. Potassium phosphate (22.7 mg, 106.5 µmol) was added thereto and reacted at 0 °C for 12 h. As monitored by LC-MS, reaction was almost complete to generate a cyclized product, which was separated and purified by an alkaline semi-preparative C18 column to obtain compound **DG-7** (6.8 mg, yield 69%). HRMS calculated for C₆₉H₁₀₉N₉O₂₀ [M+2H]²⁺ 692.8973, found 692.8910.

### Example 34: Synthesis of Compound dDG-1

**The structure and synthetic method of compound dDG-1 are as follows:**

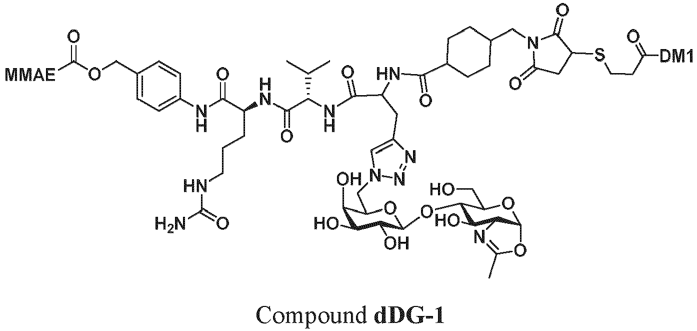

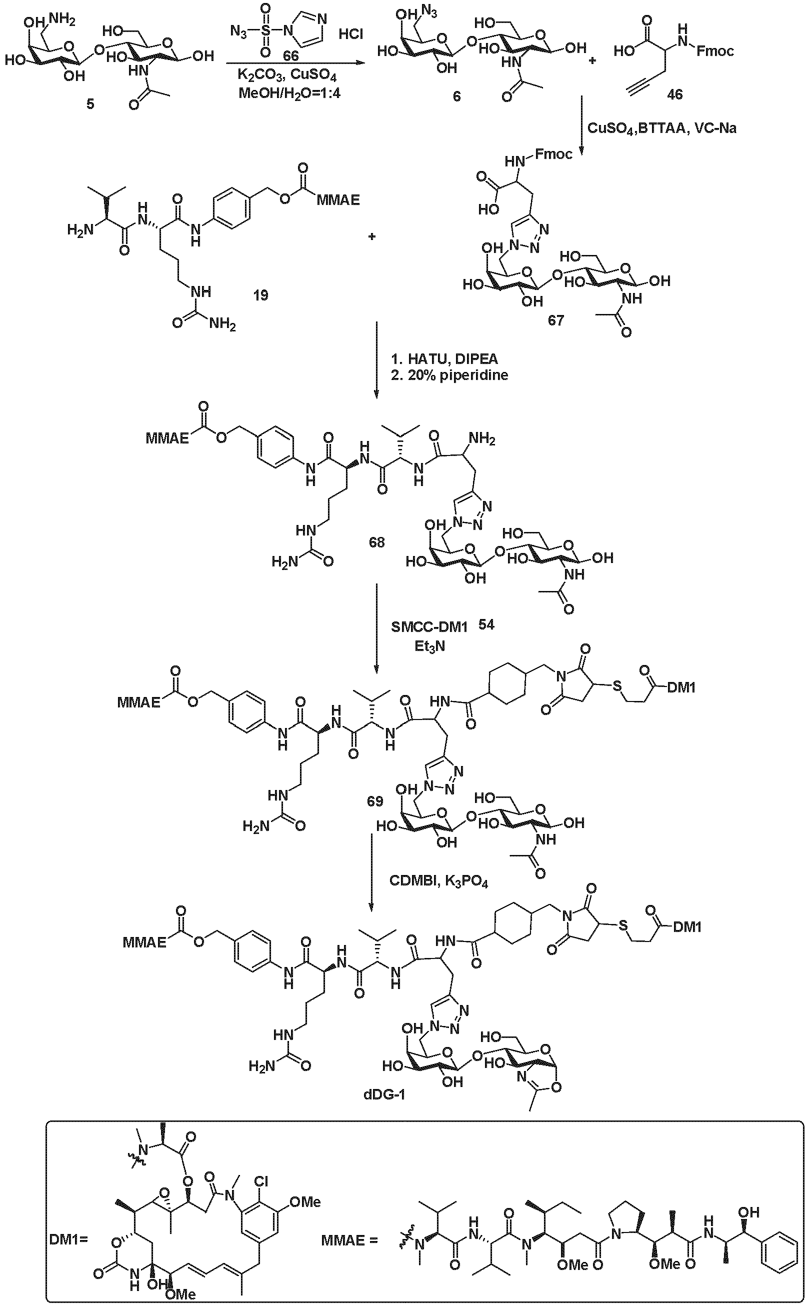

Step 1: Compound **5** (10 mg, 26.17 µmol) was weighed and dissolved in a 500 µL of CH₃OH/H₂O=1:4 system. 1H-imidazole sulfonyl azide hydrochloride (8.2 mg, 39.3 µmol), potassium carbonate (10.9 mg, 78.6 µmol), and copper sulfate (6.2 mg, 39.3 µmol) were added to the above reaction system, and reacted at 37 °C for 4 hours. When the reaction was almost complete as monitored by LC-MS, the resultant was separated and purified by a P2 column, followed by freeze drying to obtain compound **6** (9 mg, yield 85%).

Step 2: Preparation of Cu (I) - BTTAA solution: 55 µL of 60 mM CuSO₄, 66 µL of 300 mM BTTAA, and 490 µL of 0.9M sodium ascorbate were mixed well for use.

Step 3: Compound **6** (9 mg, 22 µmol) was weighed and dissolved in a 50 µL of 50 mM PB, pH 7.5 buffer. Compound **46** (7.4 mg, 22 µmol) was added to the above reaction system, mixed well, and then added with all volumes of Cu (I) - BTTAA solution from step 2 and reacted at 37 °C for 4 hours. When the reaction was complete as monitored by LC-MS, the resultant was separated and purified by a semi preparative C18 column, followed by freeze drying to obtain compound **67** (14.2 mg, yield 88%).

Step 4: Compound **67** (14.2 mg, 19.1 µmol) was weighed and dissolved in 100 µL of DMF. HATU (14.6 mg, 38.2 µmol), compound **19** (21.5 mg, 19.1 µmol), and DIPEA (10 µL, 57.3 µmol) were added to the above reaction system, and reacted at 37 °C for 2 hours. After the reaction was complete as monitored by LC-MS, 20 µL of piperidine was added. After 15 minutes, LC-MS showed that the reaction was complete. Compound **68** (26 mg, yield 83%) was obtained by freeze-drying after separation and purification on a semi preparative C18 column.

Step 5: Compound **68** (20 mg, 12.3 µmol) was weighed and dissolved in 100 µL of DMF. Compound **54** (SMCC-DM1, 13.2 mg, 12.3 µmol) and triethylamine (5 µL, 36.9 µmol) were added to the above reaction system, and reacted at 37 °C for 2 hours. When the reaction was complete as monitored by LC-MS, the resultant was separated and purified by a semi preparative C18 column, followed by freeze drying to obtain compound **69** (24 mg, yield 76%). Step 6: Compound **69** (24 mg, 9.3 µmol) was weighed and dissolved in 100 µL of DMF/50 mM PB, pH 7. =1:1. CDMBI (10 mg, 46.5 µmol) was added to the above reaction system, mixed well, and cooled to 0 °C on ice, and potassium phosphate (29 mg, 139.5 µmol) was added and reacted at 0 °C for 12 hours. When the reaction was almost complete as monitored by LC-MS, the resultant was separated and purified by an alkaline C18 column, followed by freeze drying to obtain compound **dDG-1** (15.5 mg, yield 65%).

### Example 35: Synthesis of Compound dDG-2

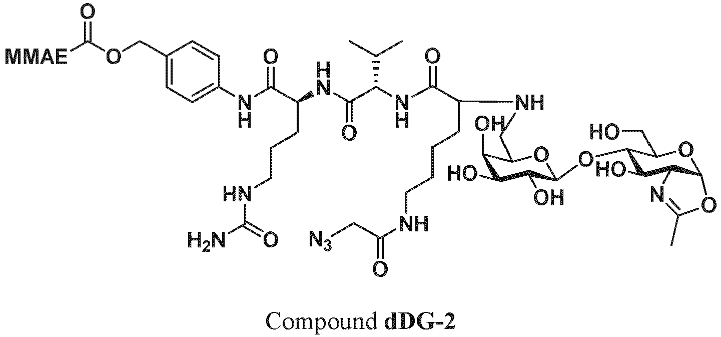

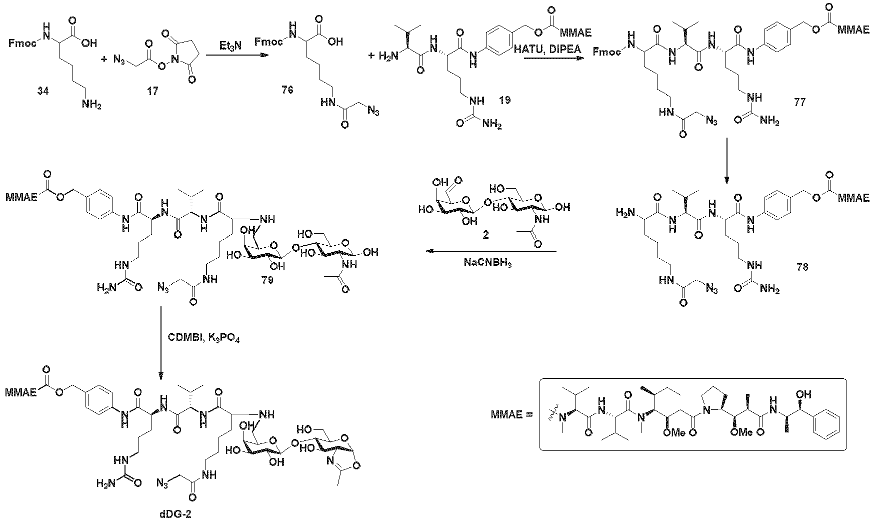

Step 1: Compound **34** (Fmoc Lys OH, 20 mg, 54.3 µmol) was weighed and dissolved in 200 µL of DMF. Compound **17** (11.82 mg, 59.8 µmol) and 22.6 µL of trimethylamine were added to the above system, mixed well, and reacted at room temperature for 2 hours. When the reaction was complete as monitored by LC-MS, the resultant was separated and purified by a semi preparative C18 column, followed by freeze drying to obtain compound **76** (22.5 mg, yield 92%). HRMS calculated for C₂₃H₂₅N₅O₅ [M+H]⁺ 452.1934, found 452.1991

Step 2: Compound **76** (20 mg, 44.3 µmol) and HATU (33.5 mg, 88.6 µmol) were weighed and dissolved in 100 µL of anhydrous DMF. Compound **19** (NH₂-VC-PAB-MMAE, 49.9 mg, 44.3 µmol) was added to the above reaction system, added with DIPEA (22.8 µL, 132.9 µmol) dropwise with stirring, and reacted at room temperature for 1 hour. When the reaction was complete as monitored by LC-MS, the resultant was separated and purified by a semi preparative column, followed by freeze drying to obtain compound **77** (60 mg, yield 88%) HRMS, calculated for C₈₁H₁₁₇N₁₅O₁₆ [M+2H]²⁺ 778.9479, found 778.9479, found 778.9477.

Step 3: Compound **77** (20 mg, 12.8 µmol) was weighed and dissolved in 100 µL of DMF. 20 µL of piperidine was added to the above reaction system, and reacted at room temperature for 20 minutes. When the reaction was complete as monitored by LC-MS, the resultant was separated and purified by a semi preparative C18 column, followed by freeze drying to obtain compound **78** (15.8 mg, yield 92%). HRMS calculated for C₆₆H₁₀₇N₁₅O₁₄ [M+2H]²⁺ 667.9139, found 667.9140.

Step 4: Compound **78** (15.8 mg, 11.8 µmol) was weighed and dissolved in 100 µL of DMF, and compound **2** (18.3 mg, 47.2 µmol) was weighed and dissolved in 100 µL of 0.2 M PB, pH 6.0 buffer, and added to the above system. After the pH of the reaction system was 6.0 as detected, NaCNBH₃ (70.3 mg, 118 µmol) was added thereto. The reaction system was mixed well and reacted at 37 °C for 6 hours. As monitored by LC-MS, most product was generated, which was separated and purified by a semi preparative C18 column, followed by freeze drying to obtain compound **79** (15 mg, yield 75%). HRMS calculated for C₈₀H₁₃₀N₁₆O₂₄ [M+2H]²⁺ 850.48, found 850.4721.

Step 5: Compound **79** (10 mg, 5.9 µmol) was weighed and dissolved in 100 µL of DMF/50 mM PB, pH 7.5=1:1. CDMBI (6.7 mg, 31.2 µmol) was added to the above system, mixed well, and cool to 0 °C on ice. Potassium phosphate (20 mg, 93.7 µmol) was added thereto and reacted at 0 °C for 12 hours. A cyclized product was generated as monitored LC-MS, which was separated and purified by an alkaline semi-preparative C18 column to obtain compound **dDG-2** (7 mg, yield 71%). HRMS calculated for C₆₉H₁₀₉N₉O₂₀ [M+2H]²⁺ 842.4747, found 842.4721.

### Example 36: Synthesis of Compound dDG-3

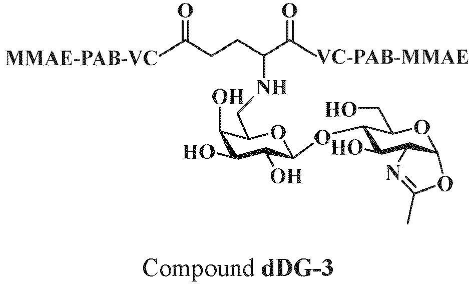

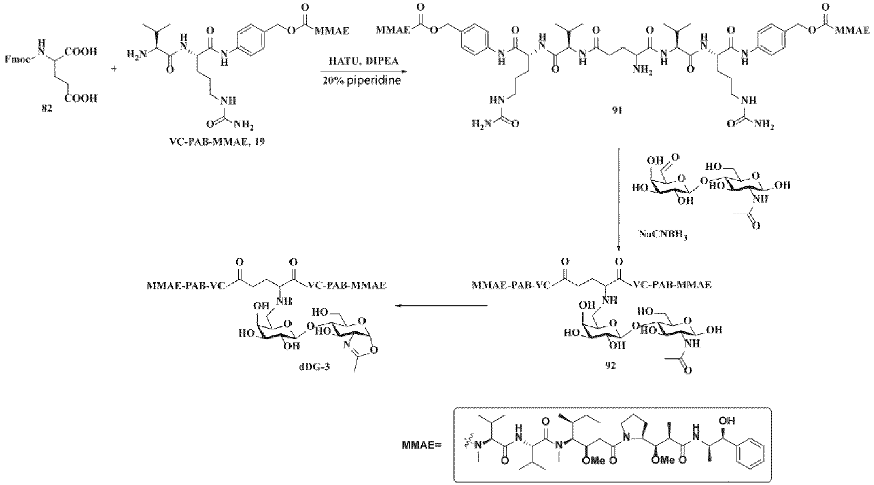

Step 1: Compound **82** (6 mg, 0.0162 mmol) was weighed and dissolved in 60 µL of DMF, HATU (17.9 mg, 0.0486 mmol), compound **19** (33 mg, 0.0292 mmol), and DIPEA (8.86µL, 0.0649 mmol) were sequentially added to the above reaction system, and reacted at room temperature for 2 hours. As monitored by LC-MS, the reaction was almost complete, and 128.5 µL of trimethylamine was added, mixed well and reacted at room temperature for 15 minutes. The resultant was separated and purified by a semi preparative C18 column, and the target product was collected and freeze-dried to obtain compound **91** (21.5 mg, yield 57.15%). HRMS, calculated for C₁₂₁H₁₉₃N₂₁O₂₆ [M+2H]²⁺ 1179.2291, [M+3H]³⁺ 786.4887, found 1179.221178.484.

Step 2: Compound **91** (21.5 mg, 9.12 µmol) was weighed and dissolved in 210 µL of DMF, CHO-LacNAc (10.44 mg, 0.0273 mmoL) was added to the above reaction system, and DMF was added such that DMF/0.2M PB=1:1. The pH of the reaction system was adjusted to 6.0 using NaOH/HCl, and then sodium cyanide borohydride (5.74 mg, 0.0912 mmoL) was added and reacted at room temperature for 3-4 hours. The reaction is almost complete as monitored by LC-MS. The resultant was separated and purified by a semi preparative C18 column, and the target product was collected and freeze-dried to obtain compound **92** (10.5 mg, yield 50.5%). HRMS, calculated for C₁₃₅H₂₁₆N₂₂O₃₆ [M+2H]²⁺ 1361.7952, [M+3H]³⁺ 908.1994, found 1361.7889, 908.1909.

Step 3: Compound **92** (10.5 mg, 3.86 µmol) was weighed and dissolved in 210 µL of DMF/H₂O=1:1, DMC (13 mg, 32.63µmoL) and triethylamine (32.2 µL, 0.231 mmol) were added to the above reaction system, which was mixed well, cooled to 0 °C on ice and reacted for 2 hours. When the reaction was almost complete as monitored by LC-MS, the resultant was separated and purified by an alkaline C18 column, and the target product was collected and freeze-dried to obtain compound **dDG-3** (7.1 mg, yield 68%). HRMS, calculated for C₁₃₅H₂₁₄N₂₂O₃₅ [M+2H]²⁺ 1352.7899, [M+3H]³⁺ 902.1959, found 1352.7881, 902.1952.

### IV: Synthesis of glycoengineered antibodies Ab-1 to Ab-19

### Example 37: Synthesis of Ab-1

The non-natural glycoengineered antibody **Ab-1** was obtained from compound **G1** and the wild-type antibody Herceptin through general operation 1. The HRMS measured value after deconvolution is 146536.

### Example 38: Synthesis of Ab-2

The non-natural glycoengineered antibody **Ab-2** was obtained from compound **G2** and the wild-type antibody Herceptin through general operation 1.

### Example 39: Synthesis of Ab-3

The non-natural glycoengineered antibody **Ab-3** was obtained from compound **G3** and the wild-type antibody Herceptin through general operation 1. The HRMS measured value after deconvolution is 146702.

### Example 40: Synthesis of Ab-4

The non-natural glycoengineered antibody **Ab-4** was obtained from compound **G4** and the wild-type antibody Herceptin through general operation 1. The HRMS measured value after deconvolution is 146699.

### Example 41: Synthesis of Ab-5

The non-natural glycoengineered antibody **Ab-5** was obtained from compound **G5** and the wild-type antibody Herceptin through general operation 1. The HRMS measured value after deconvolution is 146170.

### Example 42: Synthesis of Ab-6

The non-natural glycoengineered antibody **Ab-6** was obtained from compound **G6** and the wild-type antibody Herceptin through general operation 1.

### Example 43: Synthesis of Ab-7

The non-natural glycoengineered antibody **Ab-7** was obtained from compound **G7** and the wild-type antibody Herceptin through general operation 1. The HRMS measured value after deconvolution is 146642.

### Example 44: Synthesis of Ab-8

The non-natural glycoengineered antibody **Ab-8** was obtained from compound **G8** and the wild-type antibody Herceptin through general operation 1. The HRMS measured value after deconvolution is 146642.

### Example 45: Synthesis of Ab-9

The non-natural glycoengineered antibody **Ab-9** was obtained from compound **G9** and the wild-type antibody Herceptin through general operation 1. The HRMS measured value after deconvolution is 146701.

### Example 46: Synthesis of Ab-10

The non-natural glycoengineered antibody **Ab-10** was obtained from compound **G10** and the wild-type antibody Herceptin through general operation 1. The HRMS measured value after deconvolution is 147218.

### Example 47: Synthesis of Ab-11

The non-natural glycoengineered antibody **Ab-11** was obtained from compound **G11** and the wild-type antibody Herceptin through general operation 1. The HRMS measured value after deconvolution is 146831.

### Example 48: Synthesis of Ab-12

The non-natural glycoengineered antibody **Ab-12** was obtained from compound **G12** and the wild-type antibody Herceptin through general operation 1. The HRMS measured value after deconvolution is 147120.

### Example 49: Synthesis of Ab-13

The non-natural glycoengineered antibody **Ab-13** was obtained from compound **G13** and the wild-type antibody Herceptin through general operation 1. The HRMS measured value after deconvolution is 147110.

### Example 50: Synthesis of Ab-14

The non-natural glycoengineered antibody **Ab-14** was obtained from compound **G14** and the wild-type antibody Herceptin through general operation 1. The HRMS measured value after deconvolution is 147152.

### Example 51: Synthesis of Ab-15

The non-natural glycoengineered antibody **Ab-15** was obtained from compound **G3** and the defucosylated antibody Herceptin through general operation 2. The HRMS measured value after deconvolution is 146423.

### Example 52: Synthesis of Ab-16

The non-natural glycoengineered antibody **Ab-16** was obtained from compound **G8** and the defucosylated antibody Herceptin through general operation 2. The HRMS measured value after deconvolution is 146362.

### Example 53: Synthesis of Ab-17

The non-natural glycoengineered antibody **Ab-17** was obtained from compound **G10** and the defucosylated antibody Herceptin through general operation 2. The HRMS measured value after deconvolution is 146939.

### Example 54: Synthesis of Ab-18

The non-natural glycoengineered antibody **Ab-18** was obtained from compound **G12** and the defucosylated antibody Herceptin through general operation 2. The HRMS measured value after deconvolution is 146819.

### Example 55: Synthesis of Ab-19

The non-natural glycoengineered antibody **Ab-19** was obtained from compound **G13** and the defucosylated antibody Herceptin through general operation 2.

### V: Synthesis of glycosite-specific antibody-drug conjugates gsADC-1 to gsADC-43

### Example 56: Synthesis of gsADC-1 (gsADC means sugar site-specific antibody-drug conjugate)

Antibody-drug conjugate **gsADC-1** was obtained from compound **D2** and non-natural glycoengineered antibody **Ab-2** through general operation 4.

### Example 57: Synthesis of gsADC-2

Antibody-drug conjugate **gsADC-2** was obtained from compound **D1** and non-natural glycoengineered antibody **Ab-2** through general operation 5. The HRMS measured value after deconvolution is 148892.

### Example 58: Synthesis of gsADC-3

Antibody-drug conjugate **gsADC-3** was obtained from compound **D3** and non-natural glycoengineered antibody **Ab-2** through general operation 6.

### Example 59: Synthesis of gsADC-4

Antibody-drug conjugate **gsADC-4** was obtained from compound **D4** and non-natural glycoengineered antibody **Ab-2** through general operation 6.

### Example 60: Synthesis of gsADC-5

Antibody-drug conjugate **gsADC-5** was obtained from compound **D6** and non-natural glycoengineered antibody **Ab-3** through general operation 7. The HRMS measured value after deconvolution is 151464.

### Example 61: Synthesis of gsADC-6

Antibody-drug conjugate **gsADC-6** was obtained from compound **D7** and non-natural glycoengineered antibody **Ab-3** through general operation 7.

### Example 62: Synthesis of gsADC-7

Antibody-drug conjugate **gsADC-7** was obtained from compound **D8** and non-natural glycoengineered antibody **Ab-3** through general operation 7.

### Example 63: Synthesis of gsADC-8

Antibody-drug conjugate **gsADC-8** was obtained from compound **D9** and non-natural glycoengineered antibody **Ab-3** through general operation 7.

### Example 64: Synthesis of gsADC-9

Antibody-drug conjugate **gsADC-9** was obtained from compound **D6** and non-natural glycoengineered antibody **Ab-4** through general operation 7.

### Example 65: Synthesis of gsADC-10

Antibody-drug conjugate **gsADC-10** was obtained from compound **D7** and non-natural glycoengineered antibody **Ab-4** through general operation 7.

### Example 66: Synthesis of gsADC-11

Antibody-drug conjugate **gsADC-11** was obtained from compound **D8** and non-natural glycoengineered antibody **Ab-4** through general operation 7.

### Example 67: Synthesis of gsADC-12

Antibody-drug conjugate **gsADC-12** was obtained from compound **D9** and non-natural glycoengineered antibody **Ab-4** through general operation 7.

### Example 68: Synthesis of gsADC-13

Antibody-drug conjugate **gsADC-13** was obtained from compound **D6** and non-natural glycoengineered antibody **Ab-6** through general operation 7.

### Example 69: Synthesis of gsADC-14

Antibody-drug conjugate **gsADC-14** was obtained from compound **D7** and non-natural glycoengineered antibody **Ab-6** through general operation 7.

### Example 70: Synthesis of gsADC-15

Antibody-drug conjugate **gsADC-15** was obtained from compound **D8** and non-natural glycoengineered antibody **Ab-6** through general operation 7.

### Example 71: Synthesis of gsADC-16

Antibody-drug conjugate **gsADC-16** was obtained from compound **D9** and non-natural glycoengineered antibody **Ab-6** through general operation 7.

### Example 72: Synthesis of gsADC-17

Antibody-drug conjugate **gsADC-17** was obtained from compound **D6** and non-natural glycoengineered antibody **Ab-9** through general operation 7.

### Example 73: Synthesis of gsADC-18

Antibody-drug conjugate **gsADC-18** was obtained from compound **D7** and non-natural glycoengineered antibody **Ab-9** through general operation 7.

### Example 74: Synthesis of gsADC-19

Antibody-drug conjugate **gsADC-19** was obtained from compound **D8** and non-natural glycoengineered antibody **Ab-9** through general operation 7.

### Example 75: Synthesis of gsADC-20

Antibody-drug conjugate **gsADC-20** was obtained from compound **D9** and non-natural glycoengineered antibody **Ab-9** through general operation 7.

### Example 76: Synthesis of gsADC-29

Antibody-drug conjugate **gsADC-29** was obtained from compound **D13** and non-natural glycoengineered antibody **Ab-3** through general operation 7.

### Example 77: Synthesis of gsADC-21

Antibody-drug conjugate **gsADC-21** was obtained from compound **D5** and non-natural glycoengineered antibody **Ab-3** through general operation 8. The HRMS measured value after deconvolution is 151832.

### Example 78: Synthesis of gsADC-39

Antibody-drug conjugate **gsADC-39** was obtained from compound **D11** and non-natural glycoengineered antibody **Ab-3** through general operation 8. The HRMS measured value after deconvolution is 149589.

### Example 79: Synthesis of gsADC-41

Antibody-drug conjugate **gsADC-41** was obtained from compound **D12** and non-natural glycoengineered antibody **Ab-3** through general operation 8. The HRMS measured value after deconvolution is 149617.

### Example 80: Synthesis of gsADC-22

Antibody-drug conjugate **gsADC-22** was obtained from compound **D5** and non-natural glycoengineered antibody **Ab-4** through general operation 8. The HRMS measured value after deconvolution is 151833.

### Example 81: Synthesis of gsADC-23

Antibody-drug conjugate **gsADC-23** was obtained from compound **D5** and non-natural glycoengineered antibody **Ab-6** through general operation 8.

### Example 82: Synthesis of gsADC-24

Antibody-drug conjugate **gsADC-24** was obtained from compound **D5** and non-natural glycoengineered antibody **Ab-9** through general operation 8.

### Example 83: Synthesis of gsADC-42

Antibody-drug conjugate **gsADC-42** was obtained from compound **D11** and non-natural glycoengineered antibody **Ab-14** through general operation 8. The HRMS measured value after deconvolution is 152801.

### Example 84: Synthesis of gsADC-43

Antibody-drug conjugate **gsADC-43** was obtained from compound **D5** and non-natural glycoengineered antibody **Ab-15** through general operation 8.

### Example 85: Synthesis of gsADC-25

Antibody-drug conjugate **gsADC-25** was obtained from compound **D10** and non-natural glycoengineered antibody **Ab-3** through general operation 9.

### Example 86: Synthesis of gsADC-26

Antibody-drug conjugate **gsADC-26** was obtained from compound **D10** and non-natural glycoengineered antibody **Ab-4** through general operation 9.

### Example 87: Synthesis of gsADC-27

Antibody-drug conjugate **gsADC-27** was obtained from compound **D10** and non-natural glycoengineered antibody **Ab-6** through general operation 9.

### Example 88: Synthesis of gsADC-28

Antibody-drug conjugate **gsADC-28** was obtained from compound **D10** and non-natural glycoengineered antibody **Ab-9** through general operation 9.

### Example 89: Synthesis of gsADC-30

Antibody-drug conjugate **gsADC-30** was obtained from compound **DG-1** and wild-type antibody Herceptin through general operation 3. The HRMS measured value after deconvolution is 148892.

### Example 90: Synthesis of gsADC-31

Antibody-drug conjugate **gsADC-31** was obtained from compound **DG-2** and wild-type antibody Herceptin through general operation 3.

### Example 91: Synthesis of gsADC-32

Antibody-drug conjugate **gsADC-32** was obtained from compound **DG-3** and wild-type antibody Herceptin through general operation 3.

### Example 92: Synthesis of gsADC-33

Antibody-drug conjugate **gsADC-33** was obtained from compound **DG-4** and wild-type antibody Herceptin through general operation 3.

### Example 93: Synthesis of gsADC-34

Antibody-drug conjugate **gsADC-34** was obtained from compound **DG-5** and wild-type antibody Herceptin through general operation 3. The HRMS measured value after deconvolution is 148295.

### Example 94: Synthesis of gsADC-35

Antibody-drug conjugate **gsADC-35** was obtained from compound **DG-6** and wild-type antibody Herceptin through general operation 3. The HRMS measured value after deconvolution is 148751.

### Example 95: Synthesis of gsADC-36

Antibody-drug conjugate **gsADC-36** was obtained from compound **DG-7** and wild-type antibody Herceptin through general operation 3. The HRMS measured value after deconvolution is 148576.

### Example 96: Synthesis of gsADC-37

Antibody-drug conjugate **gsADC-37** was obtained from compound **dDG-1** and wild-type antibody Herceptin through general operation 3.

### Example 97: Synthesis of gsADC-38

Antibody-drug conjugate **gsADC-38** was obtained from compound **DG-6** and defucosylated antibody Herceptin through general operation 10. The HRMS measured value after deconvolution is 148448.

### VI: Screening of sugar substrates and endoglycosidases

### Preparation of sugar oxazoline

Different sugar substrates (1 equivalent, including monosaccharide, disaccharide and trisaccharide structures) were weighed and dissolved in 50 mM PB, pH 7.0 buffer, CDMBI (5 equivalents) was added to the above system, mixed well and cooled to 0 °C. Potassium phosphate (15 eq) was added, the final concentration of the sugar substrates in the reaction system was 10 mM, and the reaction was carried out at 0 °C for 2 h. Plenty of precipitate was observed, which was removed by centrifugation. The supernatant was oxazoline substrates **G1**, **G12, G15-G20** containing salts, which were directly used for the next step of screening.

### Synthesis of compound G15

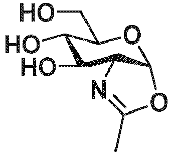

HRMS, calculated for C₈H₁₃NO₅ [M+H]⁺ 204.0872, found 204.0809_{∘} ¹H NMR (600 MHz, Deuterium Oxide) δ 6.01 (d, *J* = 7.3 Hz, 1H), 4.04 (ttd, *J=* 6.2, 4.4, 3.9, 2.4 Hz, 1H), 3.90 (t, *J* = 3.6 Hz, 1H), 3.74 - 3.70 (m, 1H), 3.60 (dd, *J* = 12.5, 6.3 Hz, 1H), 3.57 - 3.51 (m, 1H), 3.29 (ddd, *J* = 8.9, 6.3, 2.5 Hz, 1H), 1.97 - 1.95 (m, 3H).

### Synthesis of Compound G16

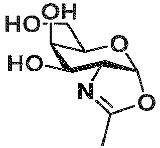

HRMS, calculated for C₈H₁₃NO₅ [M+H]⁺ 204.0872, found 204.0859_{∘} ¹H NMR (600 MHz, Deuterium Oxide) δ 6.00 (d, *J* = 7.2 Hz, 0.85H), 5.14 (d, *J* = 3.7 Hz, 0.15H), 4.02 - 3.97 (m, 1H), 3.86 - 3.84 (m, 1H), 3.81 (ddd, *J* = 7.0, 4.9, 1.8 Hz, 1H), 3.76 (td, *J =* 7.1, 1.3 Hz, 1H), 3.70 - 3.59 (m, 2H), 1.94 (d, *J=* 1.3 Hz, 3H).

### Synthesis of Compound G17

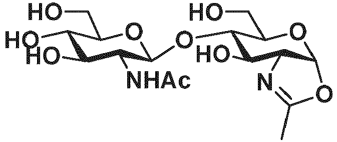

HRMS, calculated for C₁₆H₂₆N₂O₁₀ [M+H]⁺ 407.1665, found 407.1636_{∘} ¹H NMR (600 MHz, Deuterium Oxide) δ 6.00 (d, *J* = 7.3 Hz, 1H), 4.49 (d, *J* = 8.4 Hz, 1H), 4.33 (dd, *J* = 3.2, 1.6 Hz, 1H), 4.11 (ddp, *J* = 6.7, 3.5, 1.7 Hz, 1H), 3.84 (dd, *J* = 12.5, 2.1 Hz, 1H), 3.69 (dd, *J* = 12.5, 5.0 Hz, 1H), 3.63 - 3.54 (m, 3H), 3.53 - 3.45 (m, 2H), 3.42 - 3.34 (m, 2H), 3.20 (ddd, *J* = 8.9, 6.5, 2.4 Hz, 1H), 1.98 - 1.94 (m, 6H).

### Synthesis of Compound G18

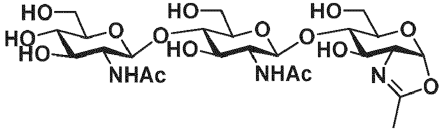

HRMS, calculated for C₂₄H₃₉N₃O₁₅ [M+H]⁺ 610.2459, found 610.2451. ¹H NMR (600 MHz, Deuterium Oxide) δ 5.99 (d, *J* = 7.3 Hz, 1H), 4.52 - 4.45 (m, 2H), 4.34 - 4.32 (m, 1H), 4.10 (ddq, *J* = 7.0, 3.3, 1.8 Hz, 1H), 3.85 -3.20 (m, 16H), 2.00 - 1.90 (m, 9H).

### Synthesis of Compound G19

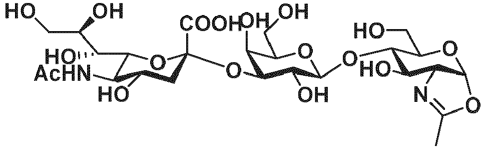

HRMS, calculated for C₂₅H₄₀N₂O₁₈ [M+H]⁺ 657.2354, found 657.2351_{∘} ¹HNMR (600 MHz, Deuterium Oxide) δ 5.97 (d, *J* = 7.3 Hz, 1H), 4.39 (d, *J* = 7.8 Hz, 1H), 3.96 (dd, *J* = 9.8, 3.2 Hz, 1H), 3.83 (d, *J* = 3.2 Hz, 1H), 3.79 - 3.32 (m, 17H), 2.51 (1H), 1.97 - 1.90 (m, 6H), 1.67 (t, *J* = 12.1 Hz, 1H).

### Synthesis of Compound G20

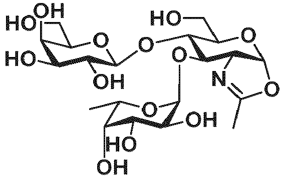

HRMS, calculated for C₂₀H₃₃NO₁₄ [M+H]⁺ 512.1979, found 512.1966_{∘} ¹H NMR (600 MHz, Deuterium Oxide) δ 6.01 (d, *J* = 7.3 Hz, 1H), 5.10 (d, *J* = 4.0 Hz, 1H), 4.38 (dd, *J* = 3.0, 1.2 Hz, 1H), 4.34 (d, *J* = 7.8 Hz, 1H), 4.28 - 4.24 (m, 1H), 4.11 (q, *J* = 6.5 Hz, 1H), 3.96 (dt, *J* = 8.7, 1.4 Hz, 1H), 3.84 - 3.32 (m, 12H), 1.98 (d, *J* = 1.8 Hz, 3H), 1.12 (3H).

### Screening of endoglycosidases

**G14,** deglycosylated antibody (Fuc α1,6) GlcNAc-Herceptin and different endoglycosidases were sequentially added to 50 mM Tris-HCl, pH 7.2 buffer, such that the final concentrations of each were 1.5 mM, 5 mg/mL and 0.2 mg/mL, respectively, which was incubated at 30 °C for 3 h for detection by mass spectrometry. At the same time, a control group without endoglycosidase was set to eliminate the influence of non-enzymatic reactions. According to the screening conditions, a total of ten endoglycosidases were screened, namely Endo-S, Endo-S D233Q, Endo-S2, Endo-S2 D184M, Endo-F3, Endo-F3 D165A, Endo-D, Endo-D Q431A, Endo-D N322Q and Endo-A. It was found that Endo-S2 had weak transfer activity only for G14, and the transfer yield was about 5.6%, as shown in Figure 1A.

### Screening of sugar substrates

Oxazoline substrates (**G1, G12, G15-G20**), deglycosylated antibody (Fuc α1,6) GlcNAc-Herceptin and endoglycosidase Endo-S2 were sequentially added to 50 mM Tris-HCl, pH 7.2 buffer, such that the final concentrations of each were 1.5 mM, 5 mg/mL, and 0.2 mg/mL, respectively, which was incubated at 30 °C for 3 h for detection by mass spectrometry. At the same time, a control group without endoglycosidase was set to eliminate the influence of non-enzymatic reactions. The results show that Endo-S2 could recognize **G1** and its transfer efficiency to the deglycosylated antibody was as high as 68%; **G12** obtained by introducing sialic acid to position 6 of galactose of **G1** could also be well recognized by Endo-S2; while for the sugar substrates **G19** and **G20** which were modified at position 3 of galactose or at position 3 of N-acetylglucosamine, the transfer activity of Endo-S2 was greatly reduced; other sugar structures could not be well recognized, either. As shown in Figure 1B.

### Pharmacological Example 1

### Experimental process of in vitro activity and data result analysis

To evaluate the activity of some disaccharide ADCs above at the cell level, three cell lines were selected, among which SK-Br-3 cells and NCI-N87 cells were Her2-positive cells, MDA-MB-231 was Her2-negative cells, and MTT was used to test the cellular viability and toxicity of ADC molecules. The specific operation was performed as follows: 100 µL of PBS was added to the outermost circle of the 96-well plate, another three wells were selected to add medium only, and the remainders were added with about 6000 corresponding cells for each well, and the plate was incubated overnight in a CO₂ incubator at 37°C. 10 µL of each ADC molecule was added (various ADC molecules were diluted 5-fold from the highest concentration of 100 nM, a total of 9 concentrations were diluted, and each concentration had 3 replicate wells), in each 96-well plate, the remaining 3 wells were plated with cells and another 3 wells were added with only 10 µL of medium as the control group and the blank group, and the 96-well plate was incubated in a CO₂ incubator at 37°C for 72 h. After adding 10 µL of 5 mg/mL MTT to each well, it was incubated at 37 °C for 4 h, then 90 µL of SDS lysate was added to each well, and incubated at 37 °C for 7 h to fully lyse the cells. Finally, the OD value of each well at 570 nm was measured, the data was processed by GraphPad Prism 6, and the results are shown in Figure 2.

In Figure 2, **gsADC-40** was prepared from N₃-NH-SCT-Her (see patent application CN107778372A for details, prepared by using the oligosaccharide structure Az-NH-SCT, as shown in the figure below) and the BCN-containing drug-linker **D5** via ring tension-promoted click chemical reaction. Specific reaction conditions: N₃-NH-SCT-Her 5 mg/mL, compound **D5** 0.55 mM, pH 7.4, purified by using protein A after the reaction was complete and the product was generated as confirmed by LC-MS.

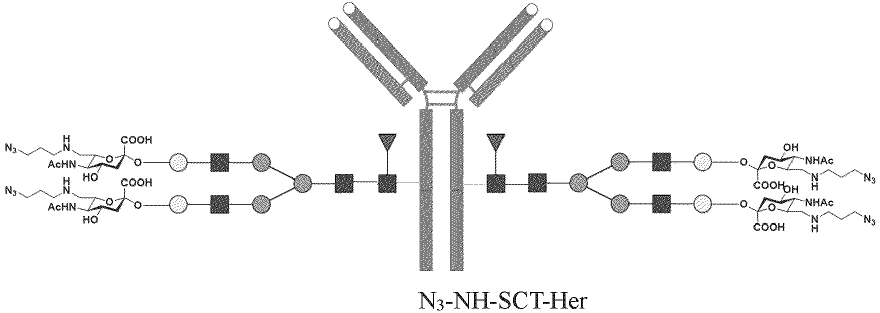

It can be seen from the results in Figure 2 that the disaccharide ADC has comparable *in vitro* cell activity to the marketed T-DM1, without toxicity in negative cells.

### Pharmacological Example 2:

### Experimental process and result analysis of anti-tumor activity in vivo

Gastric cancer cell NCI-N87 was used to construct a BALB/c nude mouse xenograft tumor model, and the mice were divided into large, medium and small groups by ear hole markings, with five mice in each group.

Four disaccharide ADC compounds **gsADC-21, gsADC-30, gsADC-35** and **gsADC-36** were evaluated for animal level activity, Cys random conjugated ADC compound (DAR≈4) was used as positive control, and PBS was used as negative control. All samples were diluted to 0.2 mg/mL with 1 x PBS before administration, and sterilized with a 0.22 mm filter membrane before use.

All samples were intraperitoneally administered at a concentration of 3 mg/kg, administered once every three days, and administered three times in total. The tumor size and mouse body weight were measured every three days using a caliper after the first administration. The experimental process complied with animal ethics requirements. The measured data were plotted and analyzed using GraphPad Prism 6 software. As shown in Figure 3, the glycosite-specific ADC compounsd prepared using the technology of the present application have good *in vivo* activity.

## Claims

1. A disaccharide linker represented by the following general formula I: 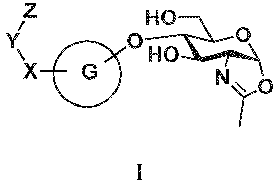 In general formula I,
G ring represents a structure derived from a monosaccharide molecule, which is connected to the 4-position of N-acetyl-D-glucosamine ring closed in 1,2 positions through a glycosidic bond, wherein the monosaccharide molecule is selected from the group consisting of galactose, N-acetyl-galactose, glucose, mannose, fucose, sialic acid sugar; the glycosidic bond is 1,4-glycosidic bond, 2,4-glycosidic bond or 3,4-glycosidic bond;
Z-Y-X- represents a substituent on the G ring, and the substitution position of Z-Y-X- is any position other than position 1 of the G ring derived from the monosaccharide molecule,
Wherein, in the structure Z-Y-X-, Z-Y- may or may not exist,
When Z-Y- does not exist, X is an aldehyde group, a phosphoric acid group, -NH₂, -CH₂-NH₂, -COOH, -CH₂SRₚ, -CH₂SeRₚ, -N₃, -CH₂-N₃, wherein Rₚ is a protecting group;
When Z-Y- exists, X is selected from the group consisting of -CH₂-, -CH₂-O-, -CH₂-S-, - CH₂-Se-, -CO-NH-, -ON=CH-, -CONH-N=CH-, -NHCH₂-, -CH=CH-, and the following structures: 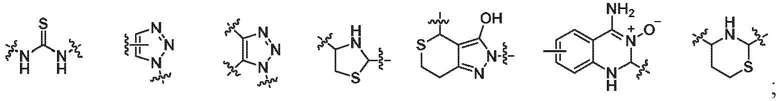
Y is a divalent linker or a multivalent linker connecting X and Z,
preferably, Y is selected from the following groups: -(CH₂)ₘ-(CH-w)ₙ-, -(CH₂-CH₂-O)ₘ-(CH-w)ₙ-, -(PO₄)ₙ-, wherein m and n are independently selected from an integer between 0-30, w is a hydrogen atom or a polyethylene glycol structure with different lengths; or a combination of cleavable fragments and the above-mentioned linking fragments;
Z is selected from the following cases i)-iv):
i) reactive groups with bioorthogonal reactivity or fragments of functional molecules,
Preferably, Z is selected from the following reactive groups: azide residues, aldehyde residues, thiol residues, alkyne residues, alkene residues, halogen residues, tetrazine residues, nitrone residues, hydroxylamine residues, nitrile residues, hydrazine residues, ketone residues, boronic acid residues, cyanobenzothiazole residues, allyl residues, phosphine residues, maleimide residues, disulfide residues, thioester residues, α-halogenated carbonyl residues, isonitrile residues, sydnones residues, selenium residues, conjugated diene residues, phosphoric acid residues, cycloalkyne residues and cycloalkene residues,
Alternatively, Z is selected from the following groups: 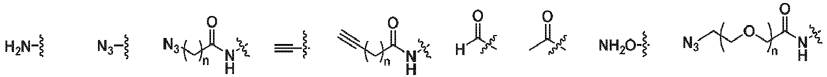 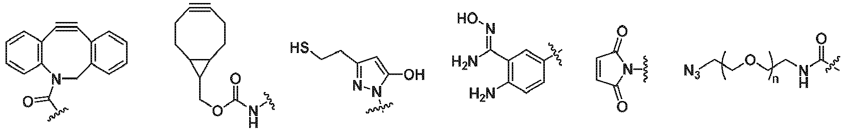 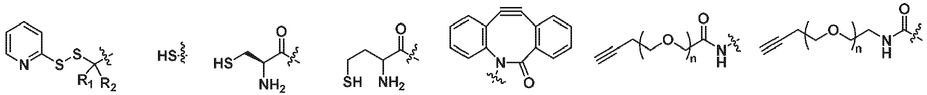 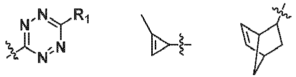
Wherein, n is an integer of 1-30, R₁ and R₂ are independently selected from H, -CH₃, - CH₂CH₃, cyclopropyl or cyclobutyl;
Preferably, the functional molecules are selected from: toxins, drugs, fluorescent probes, polyethylene glycol, lipids, polypeptides, nanobodies, DNA and related drugs, RNA and related drugs, cholesterol, antibiotics or radioisotope labels, contrast agents and MRI agents;
ii) 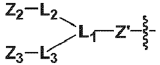
Wherein, L₁ is a trivalent linker with three reactive groups,
Preferably, L₁ is a branched-chain amino acid with reactive functional groups which is derived from lysine, aspartic acid, glutamic acid, propargylglycine, cysteine, and the following structures: 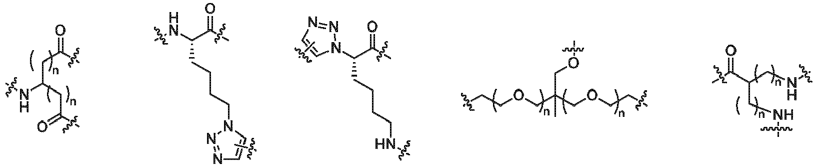 Wherein,
n is an integer of 1-30,
L₂ and L₃ are divalent or multivalent linkers connecting L₁ with Z₂ and Z₃, Preferably, L₂ and L₃ are independently selected from the following structures: -(CH₂)ₘ-(CH-w)ₙ-, -(CH₂-CH₂-O)ₘ-(CH-w)ₙ-, -(PO₄)ₙ-, wherein m and n are independently selected from integers between 0-30, w is a hydrogen atom or other side chain structures, such as polyethylene glycol with different lengths; or a combination of cleavable fragments and the above-mentioned linking fragments,
Z' is a linking fragment coupling L₁ to the sugar linker, independently is absent or -(CH₂)ₚ-, wherein p is an integer from 1 to 5, or is a group that can react with the Z group in case i), For example, Z' is selected from the following groups: 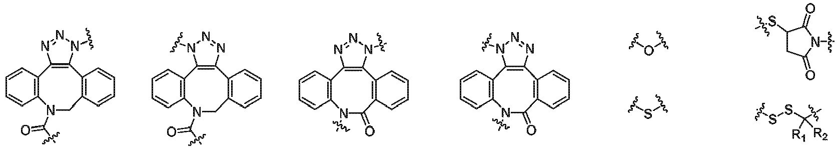 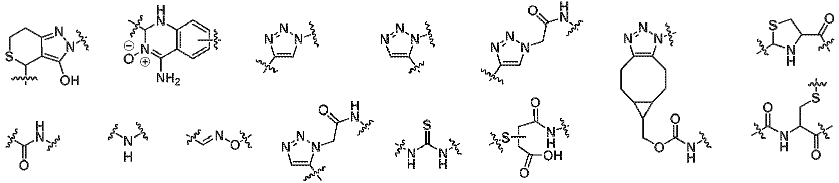 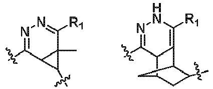
Wherein, R₁ and R₂ are each independently selected from H, -CH₃, -CH₂CH₃, cyclopropyl or cyclobutyl;
The definitions of Z₂ and Z₃ are the same as the definitions of Z in case i);
iii) 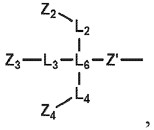
Wherein, L₆ is a tetravalent linker with four reactive groups,
Preferably, L₆ is selected from dimerized lysine, dimerized glutamic acid, dimerized aspartic acid, aspartic acid-glutamic acid dipeptide structure, aspartic acid-lysine dipeptide structure, glutamate-lysine structure, or is a structure selected from the following: 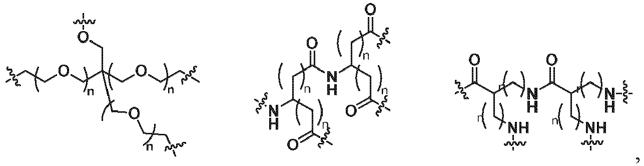
Wherein, n is an integer of 1-30, the definitions of L₂, L₃, L₄ are the same as the definitions of L₂, L₃ in ii), the definition of Z' is the same as the definition of Z' in ii), and the definitions of Z₂, Z₃, Z₄ are the same as the definitions of Z₂ and Z₃ in ii);
iv) 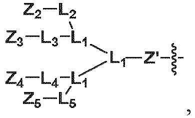
wherein, the definition of L₁ is the same as the definition of L₁ in ii), the definitions of L₂, L₃, L₄, L₅ are the same as the definitions of L₂, L₃ in ii), the definitions of Z' is the same as the definition of Z' in ii), and the definitions of Z₂, Z₃, Z₄, Z₅ are the same as the definitions of Z₂ and Z₃ in ii);
Or, when Y, Z are absent, X is selected from: 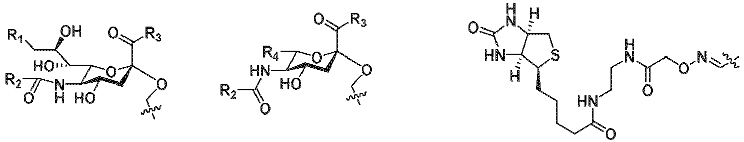
Wherein, R₁ is hydroxyl -OH or azido -N₃, R₂ is any group, R₃ is hydroxyl -OH or any group containing -NH-, R₄ is any group, 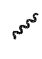 represents the connection position.

2. The disaccharide linker according to claim 1, wherein the disaccharide linker of general formula I is represented by the following general formula II: 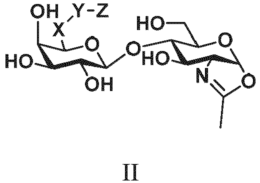 In general formula II, X, Y and Z are each as defined as those in claim 1.

3. The disaccharide linker according to claim 1 or 2, wherein the disaccharide linker is selected from the following specific compounds: 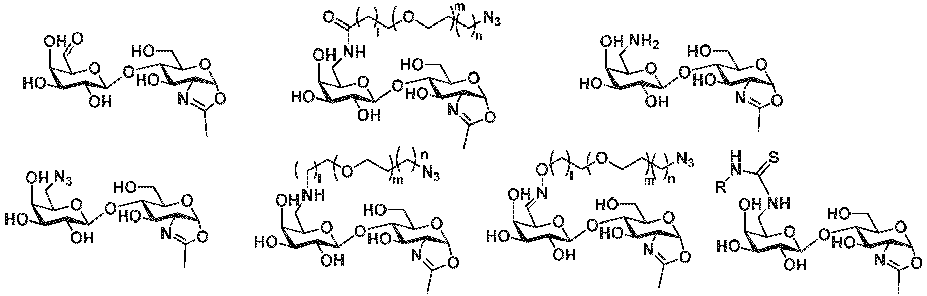 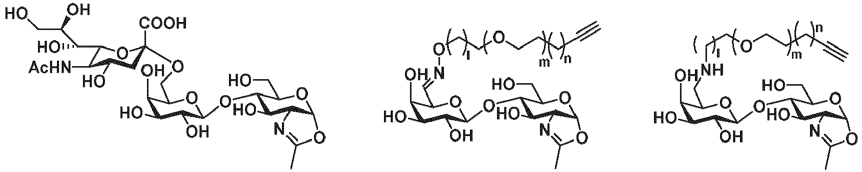 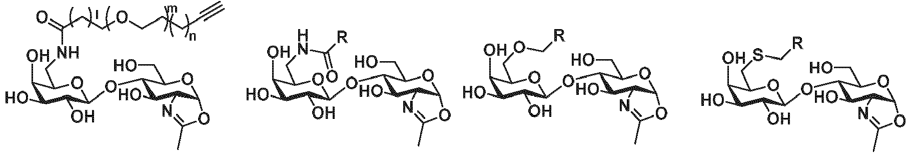 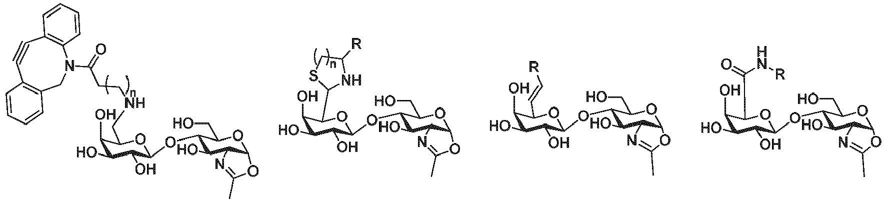 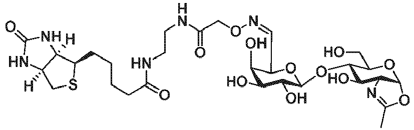 Wherein, R is a fragment involved in Y and Z of claim 1 or a combination thereof, l, m and n are each independently integers of 0-30.

4. A method for preparing the disaccharide linker according to any one of claims 1 to 3, as shown in the following reaction scheme: 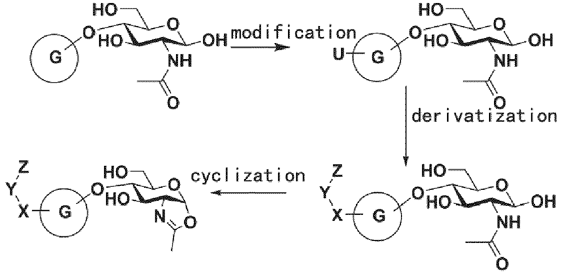
In the above reaction scheme, G ring is as defined as those in any one of claims 1 to 3, and the modification position of the monosaccharide is any modifiable position other than position 1; U is an introduced active group, and is selected from aldehyde group, amino group, azido group, alkyne group; the definitions of X, Y, and Z are as defined as those in any one of claims 1 to 3,
the method comprises the steps of:
1) The disaccharide structure with an acetylglucosamine structure at the end is modified under the action of enzymes or other small molecular compounds to obtain a disaccharide structure with an active group U, and a Z-Y-X- having orthogonal reactivity or containing a functional molecular fragment is introduced to the disaccharide structure with an active group U via derivatization; and
2) The disaccharide structure introduced with Z-Y-X- having orthogonal reactivity or containing a functional molecular fragment is converted into the disaccharide linker of general formula I by a cyclization reaction.

5. The method according to claim 4, wherein
the modification reaction in step 1) is an oxidation reaction, the enzyme is galactose oxidase, and U is an aldehyde group, or
the derivatization reaction in step 1) is an oxime-forming reaction, reductive amination, a reaction involving an amino group, or a reaction involving an azido group;
in step 2), the cyclization reaction is carried out by using 2-chloro-1,3-dimethylimidazoline chloride or 2-chloro-1,3-dimethyl-1H- benzimidazole-3-chloro.

6. A disaccharide-small molecule drug conjugate represented by the following general formulas III, IV or V: 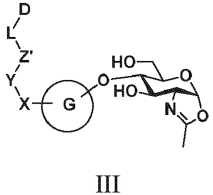 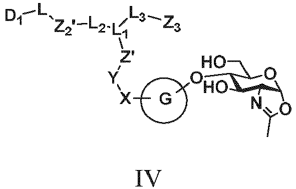 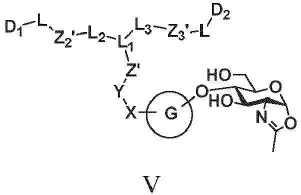
In the above general formulas III, IV and V, ring G, X, Y, Z₃, Z', L₁, L₂, L₃ are as defined as those in any one of claims 1 to 3, respectively, and in the structures of general formula IV or V, each L may be the same or different from each other, Z₂', Z₃' are linker structures formed by bioorthogonal groups and Z₂, Z₃, and each Z' can be the same or different from each other, and can also coexist or not independently;
L is a divalent linker connecting D, D₁ or D₂ with the remaining part of general formulas III-V;
Preferably, L is selected from -(CH₂)a-(OCH₂CH₂)b-(NHCO)n-(CH₂)c-,
or selected from the following groups: 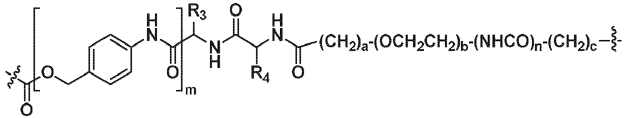 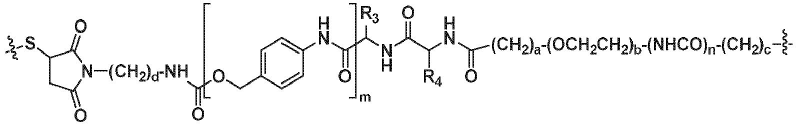 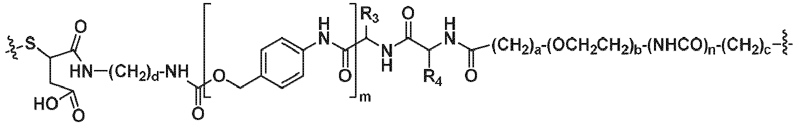 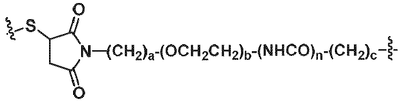 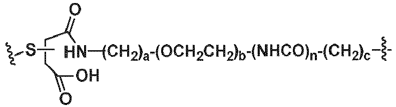 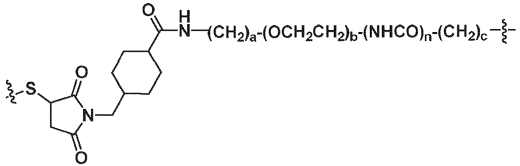 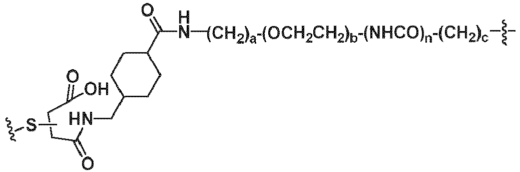 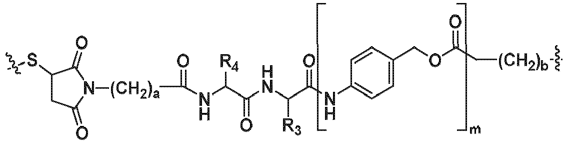 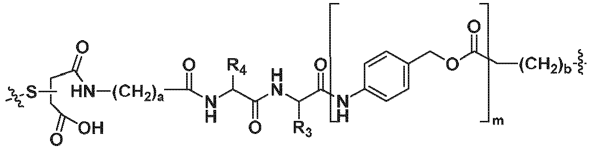 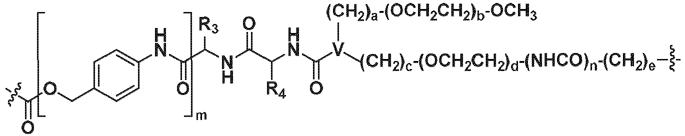 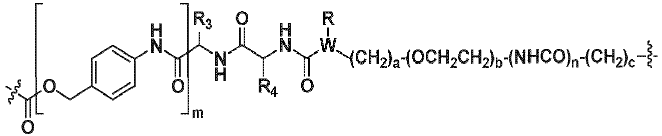
Wherein V and W are bifunctional linkers, including a structure with lysine and propargylglycine as bifunctional linkers, for example, L is selected from: 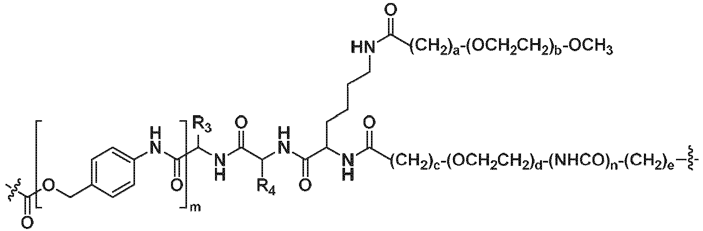 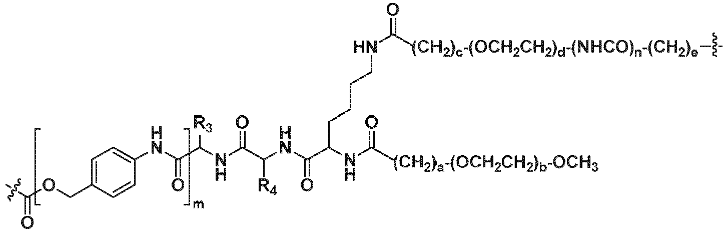 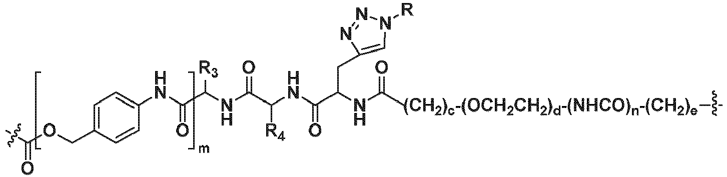
Wherein, a, b, c, d and e are each independently selected from integers between 0-30, m and n are 0 or 1, R₃ and R₄ are each independently selected from CH₃-, (CH₃)₂CH-, PhCH₂, NH₂(CH₂)₄-, NH₂CONH(CH₂)₃-, R is selected from azidizable monosaccharides, disaccharides, oligosaccharides or PEG structures with different lengths with azido groups, or combinations of PEG and chain or cyclic monosaccharides, disaccharides, and oligosaccharides, wherein the oligosaccharides include branched oligosaccharide chains; 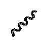
represents connection position;
D, D₁ and D₂ each independently represent a group derived from a cytotoxic compound, a small-molecule drug, or a fluorescent group, and the small-molecule drug is preferably selected from maytansine, DM-1, DM-4, MMAE, MMAF, SN-38, Dxd, duocamycin, amanitin, PBDs, vincristine, vinblastine, vinorelbine, VP-16, camptothecin, paclitaxel, docetaxel, epothilone A, epothilone B, nocodazole, colchicine, estramustine, cemadotin, eleutherobin, fluorescent reagents, monosaccharides, disaccharides, oligosaccharides, and derivatives thereof, or
The small molecule drug is a radiotherapeutic agent;
Preferably, D, D₁ and D₂ are each independently selected from the following groups:

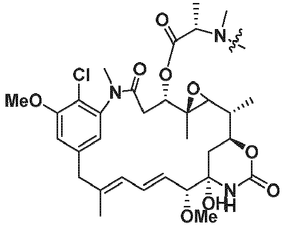

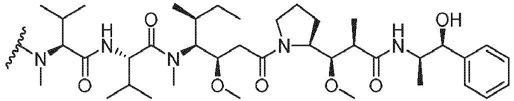

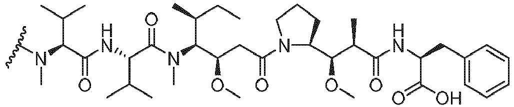

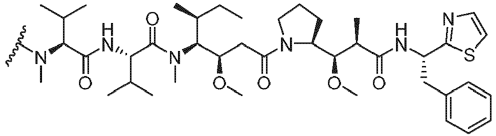

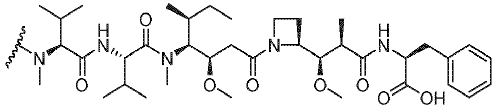

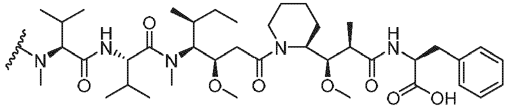

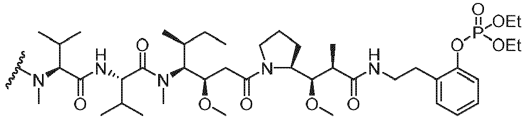

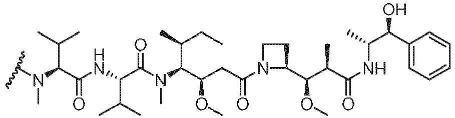

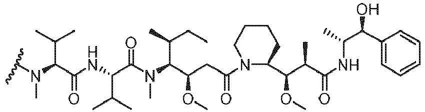

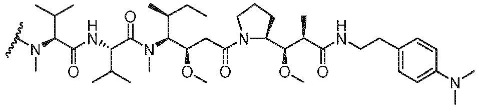

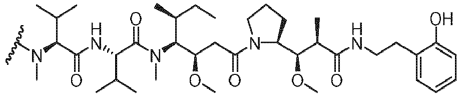

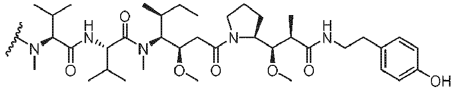

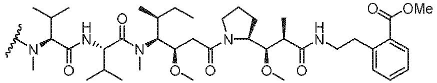

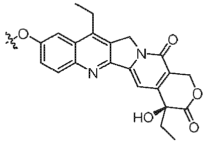

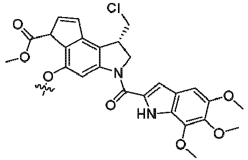

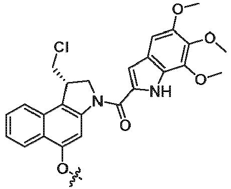

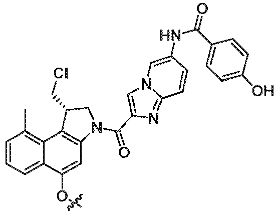

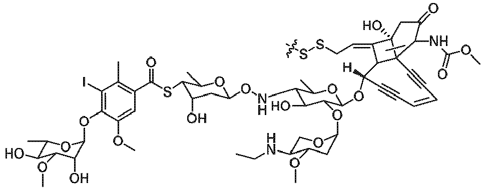

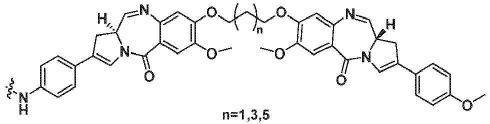

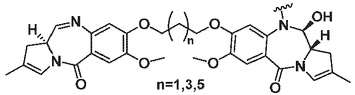

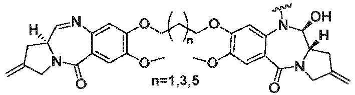

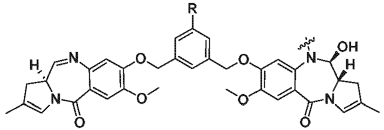

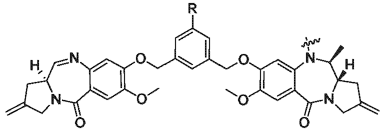

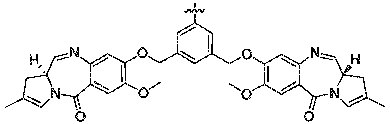

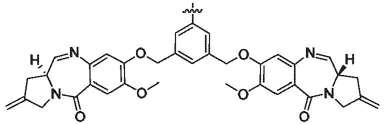

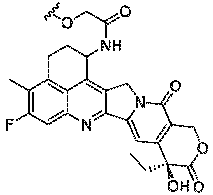

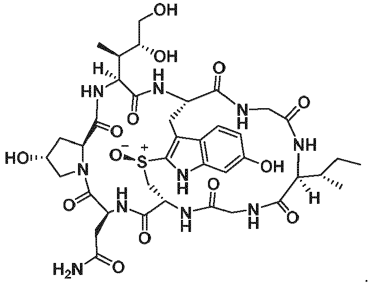

7. The disaccharide-small molecule drug conjugate according to claim 6, wherein, the disaccharide-small molecule drug conjugate is represented by the following general formulas VI, VII, or VIII: 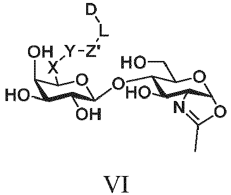 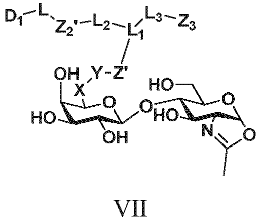 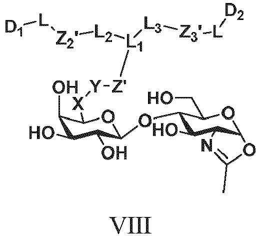 The respective substituents in the general formulas VI, VII and VIII are as defined as those in claim 6, respectively.

8. The disaccharide-small molecule drug conjugate according to claim 6 or 7, wherein the disaccharide-small molecule drug conjugate is selected from any of the following compounds: 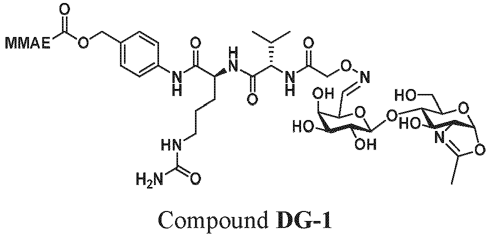 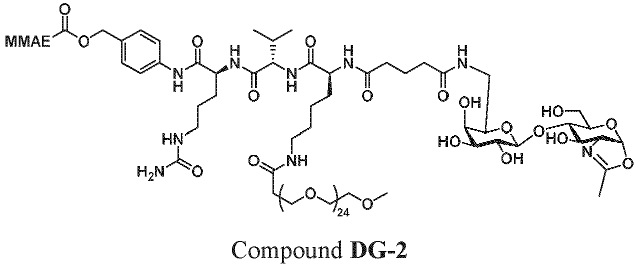 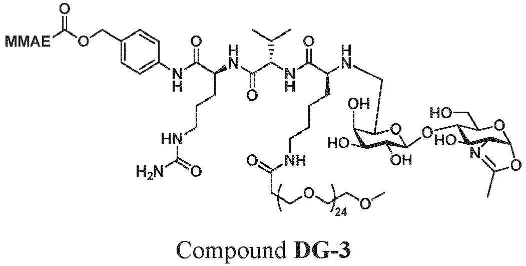 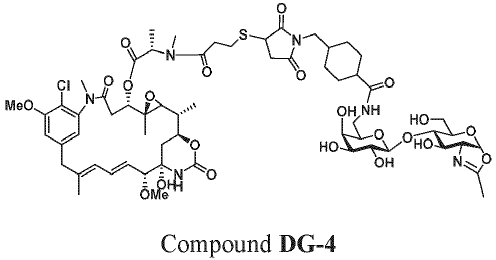 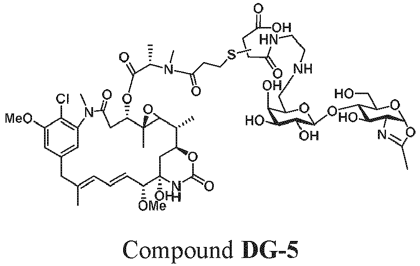 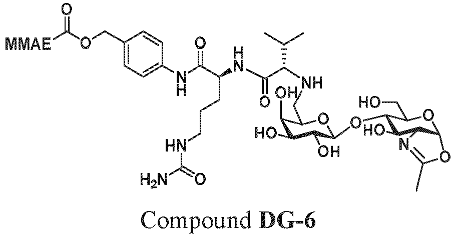 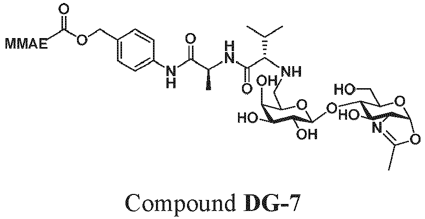 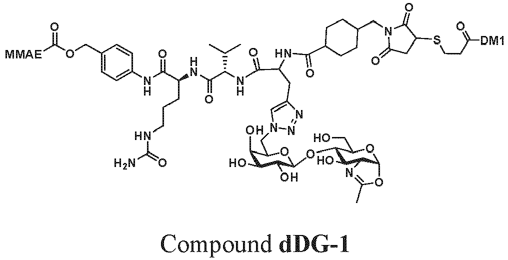 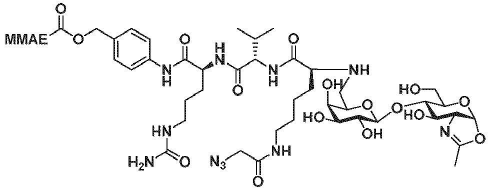 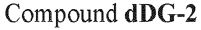 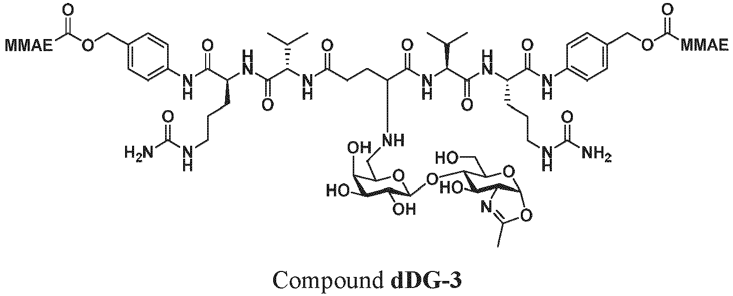 In each of the above structures, the structure of the MMAE moiety is:

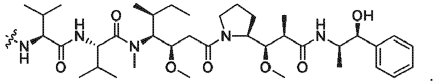

9. A glycoengineered antibody with the site-specific linkage at the N-glycosylation site of the Fc region of the antibody represented by the following general formula IX: 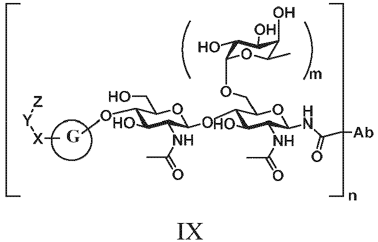
Wherein, in the above general formula IX, ring G and X, Y and Z are as defined as those in any one of claims 1 to 3, respectively, m is selected from 0 or 1, n is selected from 1 or 2; Ab is a monoclonal antibody, a bifunctional antibody or a polyclonal antibody, which is a therapeutic antibody or a functional antibody originated from different species,
Preferably, Ab is selected from the group consisting of: trastuzumab, pertuzumab, rituximab, cetuximab, muromonab, gemtuzumab ozogamicin, abciximab, daclizumab, adalimumab, palivizumab, basiliximab, bevacizumab, panitumumab, nimotuzumab, denosumab, dixituzumab, Ramucirumab, necituzumab, ipilimumab, daratumumab, Brentuximab, alemtuzumab, elotuzumab, blinatumomab, nivolumab, pembrolizumab, atezolizumab, avelumab, durvalumab, toripalimab, catumaxomab, belintumumab, emicizumab, amivantamab (Rybrevant).

10. The glycoengineered antibody according to claim 9, which is represented by the following general formula X: 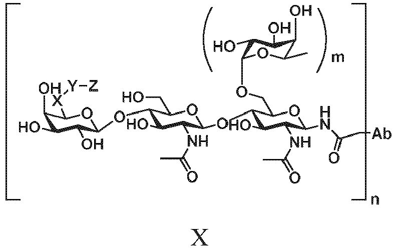 In the above general formula X, X, Y and Z are as defined as those in any one of claims 1 to 3, respectively, m is selected from 0 or 1, n is selected from 1 or 2; Ab is an antibody.

11. A method for preparing the above-mentioned glycoengineered antibody according toclaim 9, wherein the method is carried out by the following method I or the following method II:
Method I: 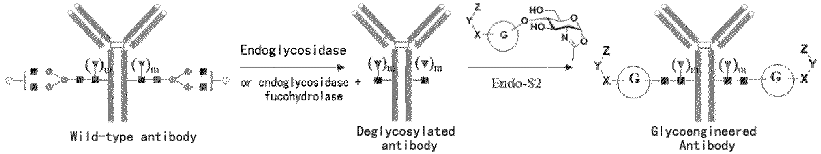
Method II: 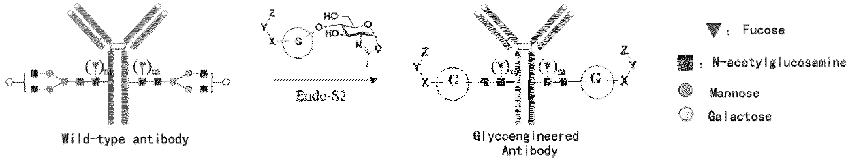
Wherein, in the above reaction scheme, m is selected from 0 or 1, and G ring, X, Y, and Z are as defined as those in any one of claims 1 to 3, respectively,
Method I:
The wild-type antibody is hydrolyzed by endoglycosidase or endoglycosidase combined with fucosidase to remove the heterogeneous sugar chain at the conservative glycosylation site of the natural antibody to obtain a deglycosylated antibody, the disaccharide linker of any one of claims 1 to 3 is then co-incubated with the wild-type antibody, and the disaccharide linker is connected to the conserved glycosylation site of the Fc domain of the antibody under the catalysis of the wild-type endoglycosidase, and the antibody of general formula X modified by a α1,6-acetylglucosamine disaccharide containing or not containing fucose is prepared, and the antibody is modified by the disaccharide linker of general formula I containing the orthogonal reactive group;
Method II:
The disaccharide linker of any one of claims 1 to 3 is co-incubated with the wild-type antibody, the N-oligosaccharide structure of the Fc domain of the wild-type antibody is hydrolyzed under the catalysis of the wild-type endoglycosidase, and at the same time the disaccharide linker is connected to the conservative glycosylation site of the Fc domain of the antibody, thus the antibody of general formula X modified by a α1,6-acetylglucosamine disaccharide containing or not containing fucose is prepared, and the antibody is modified by the disaccharide linker of general formula I containing the orthogonal reactive group,
Preferably, the wild-type endoglycosidase is N-acetylglucosaminidase, more preferably, the N-acetylglucosaminidase is Endo-S2 (Endoglycosidase-S2), for example, Endoglycosidase Endo-S2 derived from Streptococcus pyogenes; when preparing core-free fucosylated compounds, an endoglycosidase should be used with fucohydrolase together.

12. A method for preparing the glycoengineered antibody according to claim 10, the method is performed by the following method I or II:
Method I: 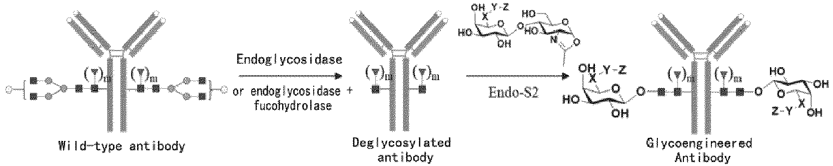
Method II: 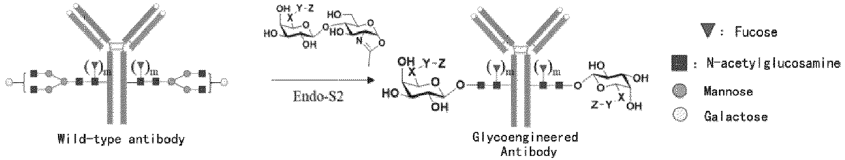 Wherein, in the above reaction scheme, m is selected from 0 or 1, and X, Y, and Z are as defined as those in any one of claims 1 to 3, respectively,
Method I:
The wild-type antibody is hydrolyzed by endoglycosidase or endoglycosidase combined with fucosidase to remove the heterogeneous sugar chain at the conservative glycosylation site of the natural antibody to obtain a deglycosylated antibody, and the disaccharide linker of any one of claims 1 to 3 and the wild-type antibody are then co-incubated, and the disaccharide linker is connected to the conserved glycosylation site of the Fc domain of the antibody under the catalysis of the wild-type endoglycosidase, and the antibody of general formula X modified by a α1,6-acetylglucosamine disaccharide containing or not containing fucose is prepared, and the antibody is modified by the disaccharide linker of general formula I containing an orthogonal reactive group;
Method II:
The disaccharide linker of any one of claims 1 to 3 is co-incubated with the wild-type antibody, the N-oligosaccharide structure of the Fc domain of the wild-type antibody is hydrolyzed under the catalytic action of the wild-type endoglycosidase, and at the same time the disaccharide linker is connected to the conservative glycosylation site of the Fc domain of the antibody, thus the antibody of general formula X modified by a α1,6-acetylglucosamine disaccharide containing or not containing fucose is prepared, and the antibody is modified by the disaccharide linker of general formula I containing an orthogonal reactive group,
Preferably, the wild-type endoglycosidase is N-acetylglucosaminidase, more preferably, the N-acetylglucosaminidase is Endo-S2 (Endoglycosidase-S2), for example, Endoglycosidase Endo-S2 derived from Streptococcus pyogenes; when preparing core-free fucosylated compounds, endoglycosidase should be used with fucohydrolase together.

13. An antibody-drug conjugate represented by the following general formula XI: 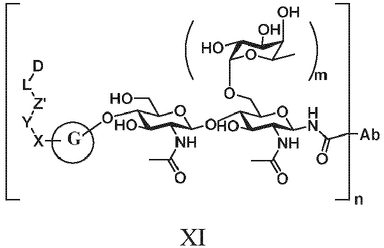 In general formula XI, ring G and X, Y, Z', L and D are as defined as those in claims 6 to 8, respectively, m is selected from 0 or 1, n is selected from 1 or 2; Ab is an antibody, and the connection site of the sugar structure is the conserved N-glycosylation site on antibody Fc.

14. The antibody-drug conjugate according to claim 13, which is represented by the following general formula XII: 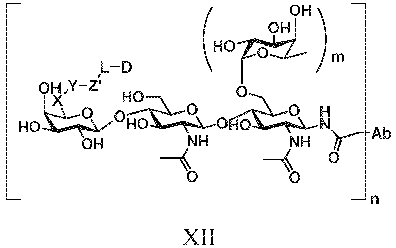 In formula XII, X, Y, Z', L and D are as defined as those in any one of claims 6 to 8, respectively, m is selected from 0 or 1, n is selected from 1 or 2; Ab is an antibody, and the connection site of the sugar structure is the conserved N-glycosylation site on antibody Fc.

15. The antibody-drug conjugate according to claim 13 or 14, wherein in the structures of general formula XI and general formula XII, -Z'-L-D is replaced by: 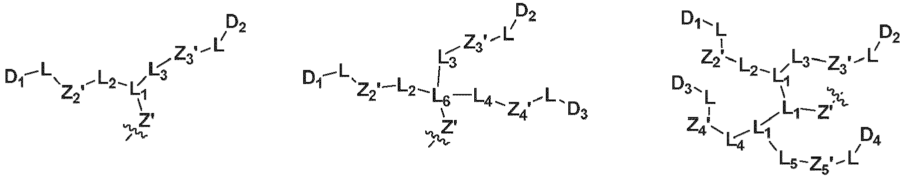 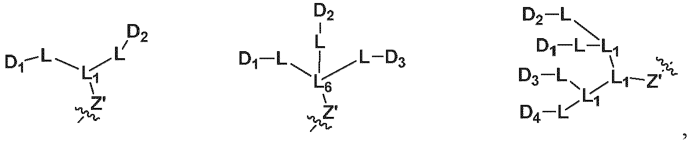 wherein, Z', L, L₁-L₆ and D₁ and D₂ are as defined as those in any one of claims 1 to 3 and claims 6-8, respectively, and Z₂', Z₃', Z₄', Z₅' are linking fragments generated by the reaction between the bioorthogonal groups of functional molecules and Z₂, Z₃, Z₄, Z₅ respectively, they can be absent simultaneously or independently; the definitions of D₃ and D₄ are the same as those of D₁ and D₂; when the structures of D₁-D₄ are the same, the antibody-drug conjugate of general formula XI or XII represents a high drug loading (drug-antibody ratio, drug to antibody ratio, DAR value) antibody-drug conjugate loading the same drug structure, when D₁-D₄ are different, the antibody-drug conjugate of general formula XI or XII represents an antibody-drug conjugate loading different drug structures in a multidrug-form.

16. A method for preparing the antibody-drug conjugate according to claim 13, and the method includes the following two methods I and II:
Method I:
a) The disaccharide linker of claim 1 is co-incubated with a wild-type antibody, the Asn297 N-oligosaccharide structure of the Fc domain of the wild-type antibody is hydrolyzed under the catalytic action of wild-type endoglycosidase, meanwhile the disaccharide linker is linked to the Asn297 site of the Fc domain of the antibody, or the disaccharide linker of claim 1 is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus preparing the antibody of general formula X modified by a α1,6-acetylglucosamine disaccharide containing or not containing fucose, which is modified by the disaccharide linker of general formula I containing the orthogonal reactive group,
b) The antibody of general formula X modified by a α1,6-acetylglucosamine disaccharide containing or not containing fucose, which is modified by the disaccharide linker of general formula I containing the orthogonal reactive group, obtained in step a) is coupled with a small molecule drug modified with a corresponding group capable of performing a specific coupling reaction with the orthogonal reactive group to prepare the antibody-drug conjugate of general formula XI;
Method II:
The disaccharide-small molecule drug conjugate of claim 6 is co-incubated with a wild-type antibody, the Asn297 N-oligosaccharide structure of the Fc domain of the wild-type antibody is hydrolyzed under the catalytic action of wild-type endoglycosidase, meanwhile the disaccharide-small molecule drug conjugate is linked to the Asn297 site of the Fc domain of the antibody, or the disaccharide-small molecule drug conjugate of claim 6 is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus the antibody-drug conjugate of general formula XI is prepared.

17. A method for preparing the antibody-drug conjugate according to claim 14 or 15, and the method includes the following two methods I and II:
Method I:
a) The disaccharide linker of claim 2 is co-incubated with a wild-type antibody, the Asn297 N-oligosaccharide structure of the Fc domain of the wild-type antibody is hydrolyzed under the catalytic action of wild-type endoglycosidase, meanwhile the disaccharide linker is linked to the Asn297 site of the Fc domain of the antibody, or the disaccharide linker of claim 2 or 3 is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus preparing the antibody of general formula X modified by a α1,6-acetylglucosamine disaccharide containing or not containing fucose, which is modified by the disaccharide linker of general formula II containing the orthogonal reactive group,
b) The antibody of general formula X modified by a α1,6-acetylglucosamine disaccharide containing or not containing fucose, which is modified by the disaccharide linker of general formula II containing the orthogonal reactive group, obtained in step a) is coupled with a small molecule drug modified with a corresponding group capable of performing a specific coupling reaction with the orthogonal reactive group to prepare the antibody-drug conjugate of general formula XII;
Method II:
The disaccharide-small molecule drug conjugate of claim 7 or 8 is co-incubated with a wild-type antibody, the Asn297 N-oligosaccharide structure of the Fc domain of the wild-type antibody is hydrolyzed under the catalytic action of wild-type endoglycosidase, meanwhile the disaccharide-small molecule drug conjugate is linked to the Asn297 site of the Fc domain of the antibody, or the disaccharide-small molecule drug conjugate of claim 7 or 8 is co-incubated with a deglycosylated antibody and an endoglycosidase, wherein the deglycosylated antibody is obtained by treating the wild-type antibody with an endoglycosidase in advance, it can be also obtained by removing fucose using a fucohydrolase at the same time, and thus the antibody-drug conjugate of general formula XII is prepared.

18. The method according to claim 16 or 17, wherein the wild-type endoglycosidase is N-acetylglucosaminidase, more preferably, the N-acetylglucosaminidase is Endo-S2 (Endoglycosidase-S2, derived from Streptococcus pyogenes endoglycosidase Endo-S2); when preparing non-core fucosylated compounds, endoglycosidase should be used with fucohydrolase together,
Preferably, in Method I, the orthogonal reactive group and the corresponding group capable of performing a specific coupling reaction with the orthogonal reactive group are selected from any combination of the following: azido group and alkynyl, mercapto and maleimide group, mercapto and mercapto or activated forms of mercapto, aldehyde group and amino, aldehyde group and aminooxy group or hydrazine group,
Preferably, in the step b) of Method I, the drug linker has the following groups, so as to be coupled with the small molecule drug modified by the corresponding group: 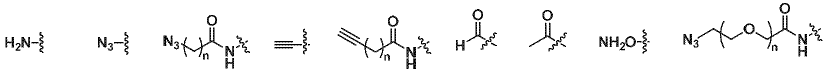 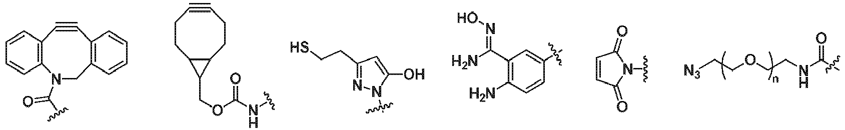 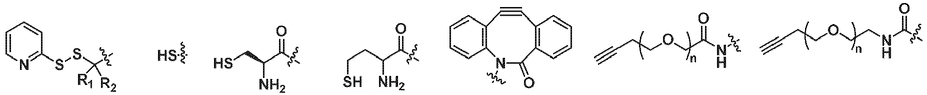 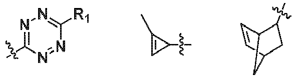
Preferably, the small molecule drug modified by the corresponding group is selected from the following compounds:

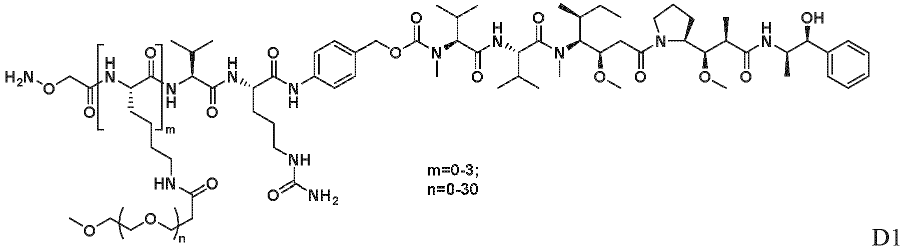

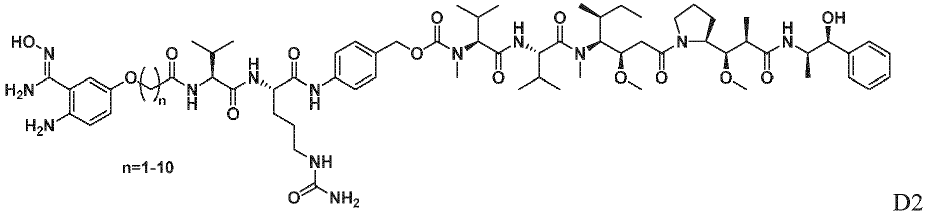

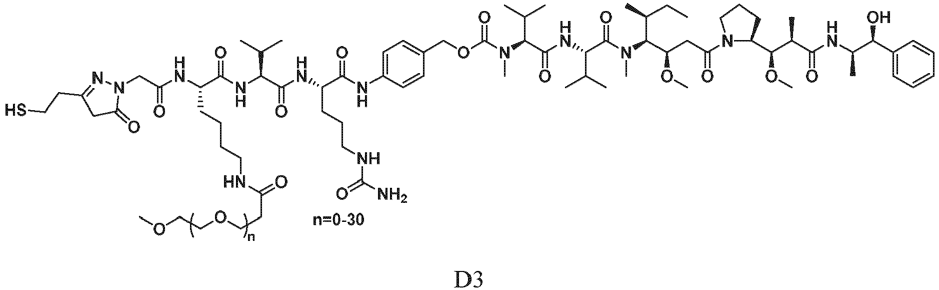

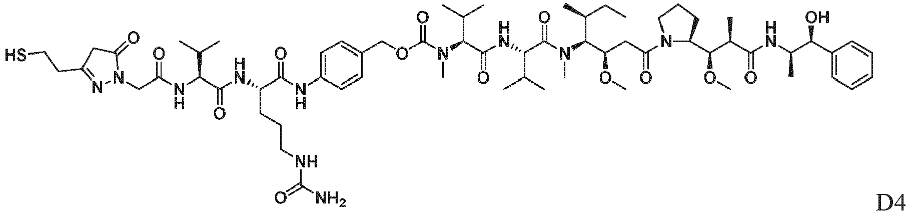

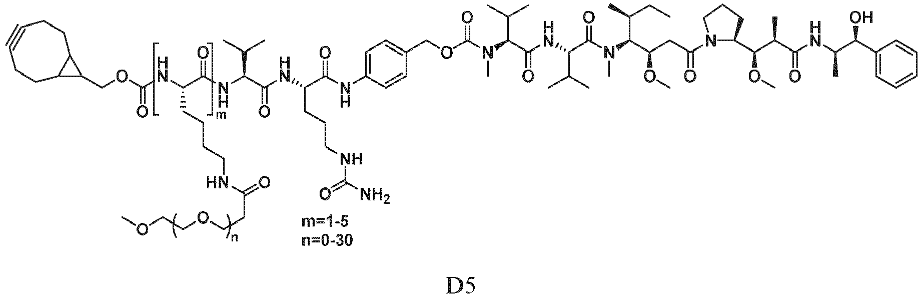

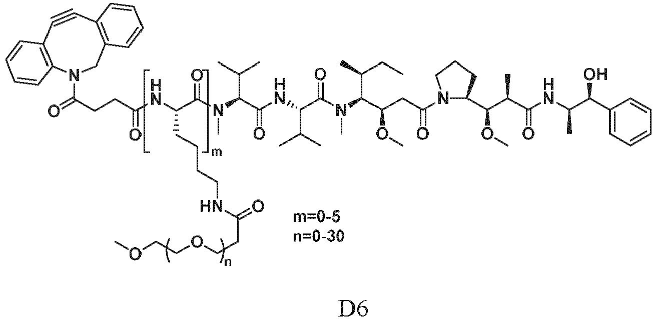

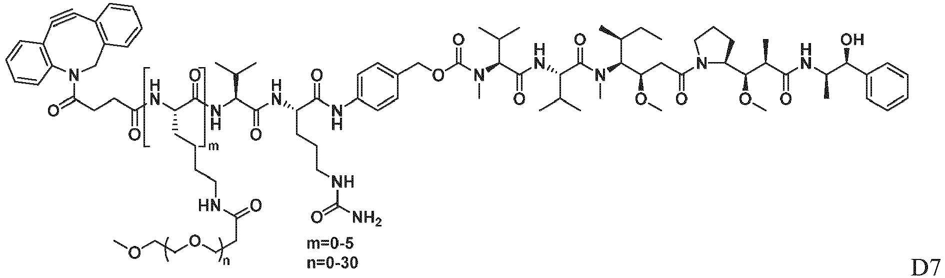

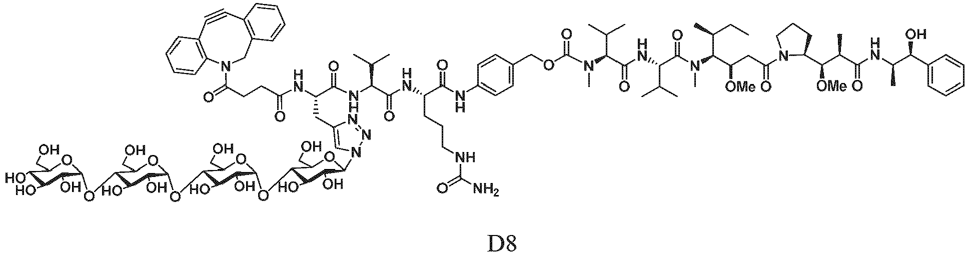

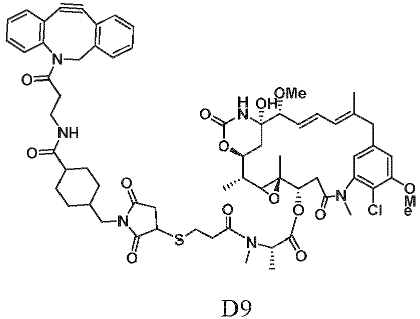

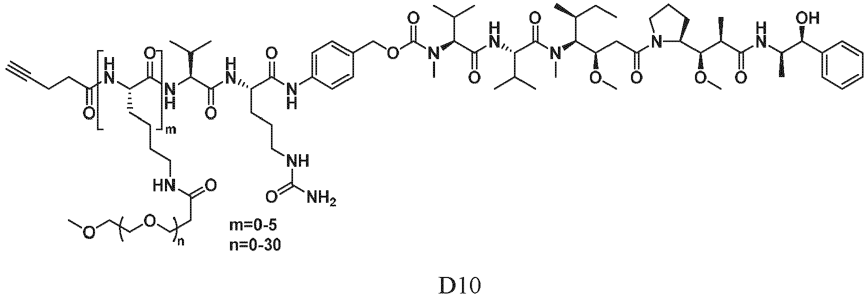

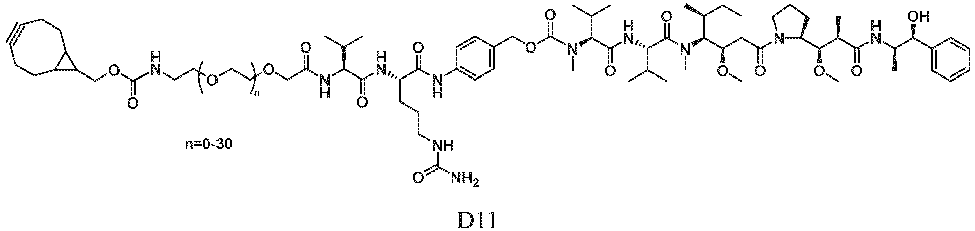

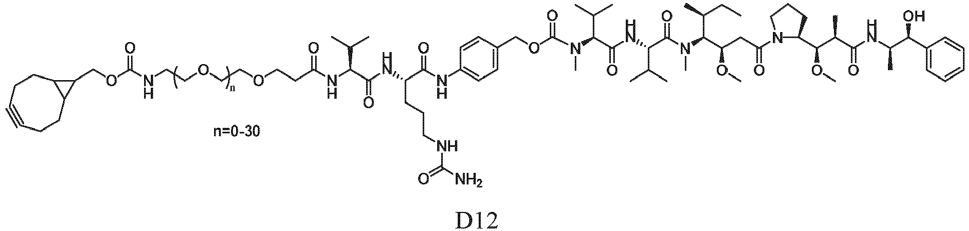

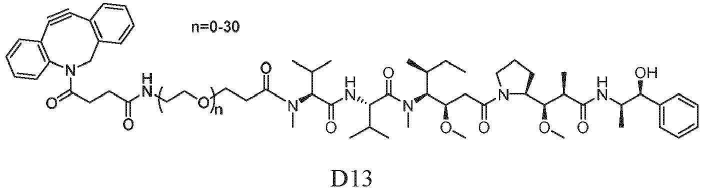

19. The method according to claim 16, wherein method I is performed as shown in the following reaction scheme: 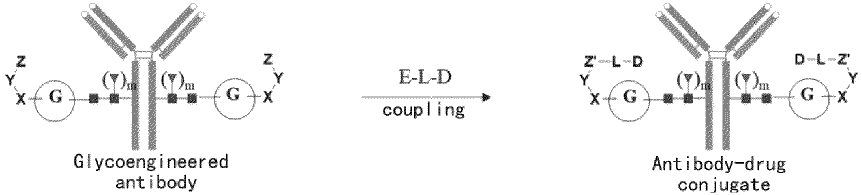 Wherein, in the above reaction scheme, m is selected from 0 or 1, X, Y, Z, Z', L, and D are as defined as those in any one of claims 1 to 3 or claims 6 to 8, respectively; E is an orthogonal reactive group that can react with Z, wherein, the glycoengineered antibody in the reaction scheme is obtained according to the method of claim 11.

20. The method according to claim 17, wherein Method I is performed as shown in the following reaction scheme: 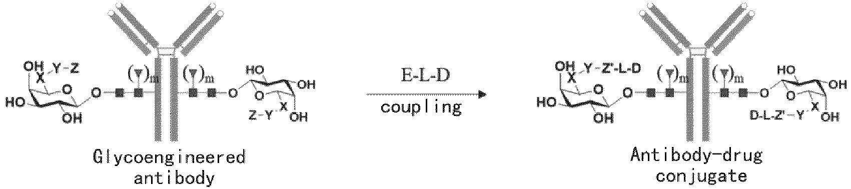
Wherein, in the above reaction scheme, m is selected from 0 or 1, X, Y, Z, Z', L, and D are as defined as those in any one of claims 1 to 3 or claims 6 to 8, respectively; E is an orthogonal reactive group that can react with Z, wherein, the glycoengineered antibody in the reaction scheme is obtained according to the method of claim 12,
preferably, the preparation method is as shown in the following reaction scheme: 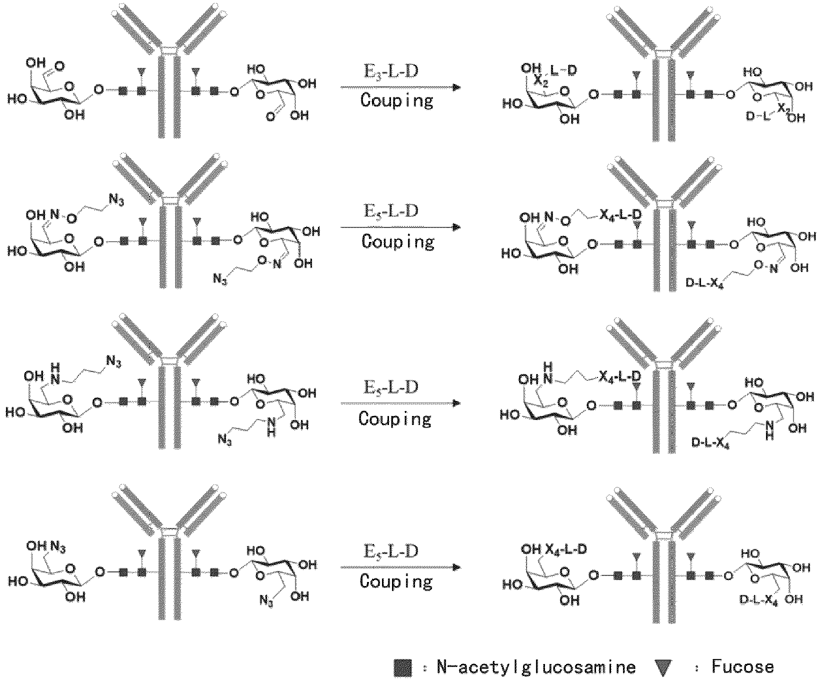
Wherein, L and D are as defined as those in claims 6 to 8, respectively, and E₃ is a corresponding group that reacts orthogonally with an aldehyde group, and is selected from thiopyrazolone, o-aminobenzamidoxime, and hydroxylamine, such as: 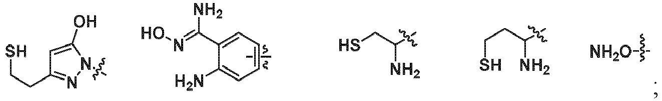
X₂ is the structure formed by the reaction between an aldehyde group and E₃;
E₅ is a corresponding group that undergoes an orthogonal reaction with an azido group, which is selected from a straight-chain alkynyl group, a DBCO structure, and a BCN structure, and X₄ is a structure formed by the reaction between an azido group and E₅.

21. The method according to claim 16, wherein Method II includes:
As shown in the following reaction scheme, the endoglycosidase is co-incubated with an antibody and the disaccharide-small molecule drug conjugate of claim 6, when the N-oligosaccharide at the conserved glycosylation site Asn297 of the Fc domain of the antibody is hydrolyzed, the disaccharide-small molecule drug conjugate is transferred to Asn297 site (Method 1), or the disaccharide-small molecule drug conjugate of claim 6 is co-incubated with a deglycosylated antibody and endoglycosidase (method II), in which the above-mentioned deglycosylated antibody is obtained by treating the wild-type antibody with endoglycosidase in advance, and it can also be obtained by removing fucose using fucohydrolase at the same time, to realize the site-specific and quantitative introduction of small molecule drugs into the sugar chain, and obtain the corresponding antibody- drug conjugates.
Method I: 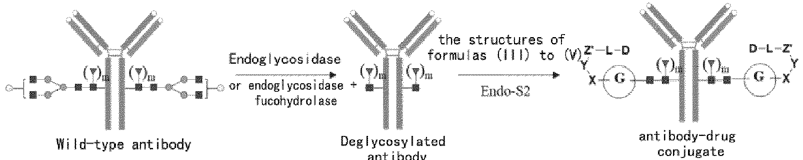
Method II:

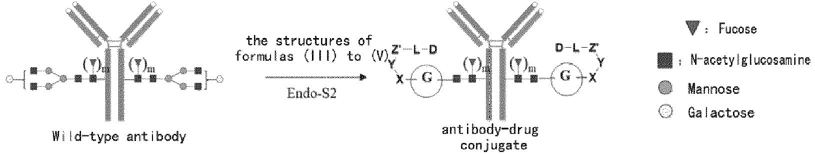

22. The method according to claim 17, wherein Method II includes:
As shown in the following reaction scheme, the endoglycosidase is co-incubated with an antibody and the disaccharide-small molecule drug conjugate of claims 6 to 8, when the N-oligosaccharide at the conserved glycosylation site Asn297 of the Fc domain of the antibody is hydrolyzed, the disaccharide-small molecule drug conjugate is transferred to Asn297 site (Method 1), or the disaccharide-small molecule drug conjugate of claims 6 to 8 is co-incubated with a deglycosylated antibody and endoglycosidase (method II), in which the above-mentioned deglycosylated antibody is obtained by treating the wild-type antibody with endoglycosidase in advance, and it can also be obtained by removing fucose using fucohydrolase at the same time, to realize the site-specific and quantitative introduction of small molecule drugs into the sugar chain, and obtain the corresponding antibody- drug conjugates.
Method I: 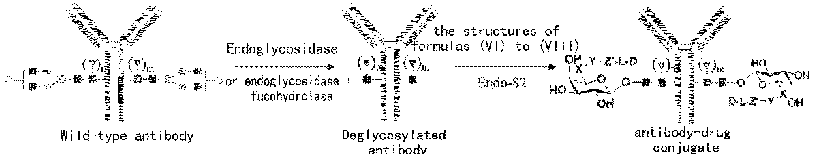
Method II:

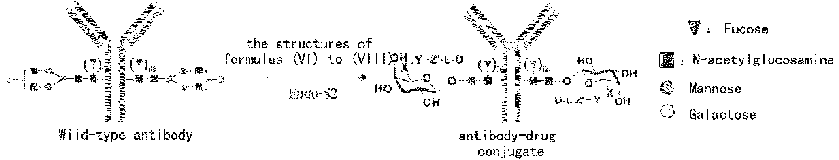

23. Use of the disaccharide linker according to any one of claims 1 to 3 or the disaccharide-small molecule drug conjugate of any one of claims 6 to 8 in antibody glycoengineered modification or in the preparation of an antibody drug conjugate.

24. Use of the antibody-drug conjugate according to any one of claims 13 to 15 in the preparation of drugs, pharmaceutical compositions or diagnostic reagents, wherein the drugs in the conjugate are selected from anti-tumor drugs, anti-inflammatory drugs, antiviral drugs, anti-infectious diseases drugs or other immunotherapeutic drugs.
